(19) Europäisches Patentamt
European Patent Office
Office européen des brevets

(11) EP 1 629 091 B1

(12) **FASCICULE DE BREVET EUROPEEN**

(45) Date de publication et mention de la délivrance du brevet:
**04.05.2011 Bulletin 2011/18**

(21) Numéro de dépôt: **04742880.0**

(22) Date de dépôt: **04.06.2004**

(51) Int Cl.:
***C12N 5/10*** *(2006.01)*

(86) Numéro de dépôt international:
**PCT/FR2004/050214**

(87) Numéro de publication internationale:
**WO 2004/111082 (23.12.2004 Gazette 2004/52)**

(54) **COMPOSITION COMPRENANT LA POLYPROTEINE NS3/NS4 ET LE POLYPEPTIDE NS5B DU VHC, VECTEURS D'EXPRESSION INCLUANT LES SEQUENCES NUCLEIQUES CORRESPONDANTES ET LEUR UTILISATION EN THERAPEUTIQUE**

HCV-POLYPROTEIN NS3/NS4 UND HCV-POLYPEPTID NS5B ENTHALTENDE KOMPOSITION, ENTSPRECHENDE NUKLEINSEQUENZEN UMFASSENDE EXPRESSIONSVEKTOREN UND THERAPEUTISCHE ANWENDUNG DAVON

COMPOSITION CONTAINING THE POLYPROTEIN NS3/NS4 AND THE POLYPEPTIDE NS5B OF HCV, EXPRESSION VECTORS COMPRISING CORRESPONDING NUCLEIC SEQUENCES AND THE THERAPEUTIC APPLICATION THEREOF

(84) Etats contractants désignés:
**AT BE BG CH CY CZ DE DK EE ES FI FR GB GR HU IE IT LI LU MC NL PL PT RO SE SI SK TR**

(30) Priorité: **05.06.2003 FR 0306772**

(43) Date de publication de la demande:
**01.03.2006 Bulletin 2006/09**

(73) Titulaires:
- **Transgene SA**
  **67400 Illkirch Graffenstaden (FR)**
- **INSTITUT NATIONAL DE LA SANTE ET DE LA RECHERCHE MEDICALE (INSERM)**
  **75654 Paris Cédex 13 (FR)**

(72) Inventeurs:
- **FOURNILLIER, Anne**
  **69003 LYON (FR)**
- **INCHAUSPE, Geneviève**
  **F-69003 LYON (FR)**
- **ABRAHAM, Jean-Daniel**
  **F-67200 STRASBOURG (FR)**
- **DIMITROVA-TCHOMAKOV, Maria**
  **F-67000 STRASBOURG (FR)**
- **PARNOT, Marie**
  **F-67200 STRASBOURG (FR)**

(74) Mandataire: **Texier, Christian**
**Cabinet Régimbeau**
**20, rue de Chazelles**
**75847 Paris Cedex 17 (FR)**

(56) Documents cités:
**WO-A-01/30812 US-B1- 6 312 889**

- **PANCHOLI PREETI ET AL: "DNA immunization with hepatitis C virus (HCV) polycistronic genes or immunization by HCV DNA priming-recombinant canarypox virus boosting induces immune responses and protection from recombinant HCV-vaccinia virus infection in HLA-A2.1-transgenic mice." JOURNAL OF VIROLOGY, vol. 77, no. 1, janvier 2003 (2003-01), pages 382-390, XP002308334 ISSN: 0022-538X cité dans la demande**
- **CHO J H ET AL: "Enhanced cellular immunity to hepatitis C virus nonstructural proteins by codelivery of granulocyte macrophage-colony stimulating factor gene in intramuscular DNA immunization" VACCINE, BUTTERWORTH SCIENTIFIC. GUILDFORD, GB, vol. 17, no. 9-10, 5 mars 1999 (1999-03-05), pages 1136-1144, XP004158236 ISSN: 0264-410X**
- **CLARKE B: "Molecular virology of hepatitis C virus" JOURNAL OF GENERAL VIROLOGY, SOCIETY FOR GENERAL MICROBIOLOGY, READING, GB, vol. 78, no. 10, 1997, pages 2397-2410, XP002172331 ISSN: 0022-1317**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- INCHAUSPÉ GENEVIEVE ET AL: "Development of a hepatitis C virus vaccine." CLINICS IN LIVER DISEASE. FEB 2003, vol. 7, no. 1, février 2003 (2003-02), pages 243-259 , xi, XP008039709 ISSN: 1089-3261

## Description

La présente invention concerne le domaine de la vaccination prophylactique et thérapeutique dirigée contre le virus de l'hépatite C (VHC). Elle a notamment pour objet une nouvelle composition contenant une polyprotéine correspondant aux deux protéines colinéaires NS3 et NS4 (appelée ci-après polyprotéine NS3/NS4) et un polypeptide constitué de NS5b, les vecteurs, tels qu'adénovirus ou poxvirus, capables d'exprimer cette composition et leur utilisation en tant que vaccin.

L'hépatite C est la cause principale des hépatites acquises par transfusion. L'hépatite C peut également être transmise par d'autres voies percutanées, par exemple par injection de drogues par voie intraveineuse. Le risque de contamination des professionnels de la santé n'est par ailleurs pas négligeable. La transmission sexuelle a été décrite.

L'hépatite C se distingue des autres formes de maladies du foie associées à des virus, telles que les hépatites A, B ou D. Les infections par le virus de l'hépatite C (VHC ou HCV) sont majoritairement chroniques avec pour résultante des maladies du foie, telles que hépatite, cirrhose et carcinome dans un grand nombre de cas (5 à 20%) et représentent dans les pays développés 30% des transplantations hépatiques.

Bien que le risque de transmission du virus par transfusion ait diminué du fait de la mise en place de tests de criblage dans les années 1990, la fréquence de nouvelles infections par le VHC reste élevée. A titre d'exemple, une étude récente indique qu'il y aurait encore aujourd'hui 10 000 à 15 000 nouveaux cas d'infection par an en France (S. Deuffic et al., Hepatology 1999 ; 29 : 1596-1601). Actuellement, environ 170 millions de personnes à travers le monde sont infectées de manière chronique par le VHC (Hepatitis C: Global prevalence (update) », 2000, Weekly Epidemiological Record, Vol 75(3)). Les populations à risque élevé sont principalement le personnel hospitalier et les utilisateurs de drogues intraveineuses, mais il existe des donneurs de sang asymptomatiques qui n'appartiennent pas à ces groupes à risque élevé et chez lesquels des anticorps anti-VHC circulants ont été retrouvés. Pour ces derniers, la voie de l'infection n'a encore pas été identifiée. Il existe donc des infections à VHC (estimation entre 5 et 10%), dites infections sporadiques dont l'étiologie est inconnue et qui ne peuvent être contrôlées.

Le VHC a été le premier virus hépatotrope isolé au moyen des techniques de biologie moléculaire. Les séquences du génome viral ont été clonées avant que la particule virale n'ait été visualisée.

Le VHC appartient à un nouveau genre de la famille des *Flaviviridae,* les hepacivirus. C'est un virus à ARN simple brin positif, de 9,5 kb, qui se réplique par une copie d'ARN complémentaire et dont le produit de traduction est un précurseur polyprotéique d'environ 3 000 acides aminés. L'extrémité 5' du génome du VHC correspond à une région non traduite adjacente aux gènes qui codent pour les protéines structurales, la protéine core de la nucléocapside, les deux glycoprotéines d'enveloppe, E1 et E2, et une petite protéine appelée p7. La région non traduite 5' et le gène core sont relativement bien conservés dans les différents génotypes. Les protéines d'enveloppe E1 et E2 sont codées par des régions plus variables d'un isolat à un autre. La protéine p7 est une protéine extrêmement hydrophobe qui constituerait un canal ionique. L'extrémité 3' du génome du VHC contient les gènes qui codent pour les protéines non structurales (NS2, NS3, NS4, NS5) et pour une région 3' non codante possédant un domaine bien conservé (Major ME, Feinstone SM, Hepatology, juin 1997, 25(6) : 1527-1538).

A l'heure actuelle, la thérapie la plus efficace pour le traitement de l'hépatite C associe l'interféron pégylé et la ribavirine (Manns MP et al., The Lancet, 22 septembre 2001, Vol. 358, 958-965). Alors que cette thérapie est particulièrement efficace dans le cas des patients infectés par des souches virales appartenant aux génotypes 2 et 3, elle n'a encore qu'un effet limité sur les génotypes 1a, 1b et 4 (Manns MP, *supra*). Moins de 50% des patients traités deviennent des «répondeurs au long terme ». Par ailleurs, cette thérapie est une intervention coûteuse (10 000 à 15 000 euro/patient/an) et est associée à des effets toxiques. En effet, 5 à 10% des patients sont obligés d'interrompre le traitement avant la fin.

Il est donc nécessaire de mettre au point une composition vaccinale ciblant tous les génotypes.

Plusieurs études montrent aujourd'hui que le contrôle d'une infection due au VHC, soit naturellement («résolution spontanée »), soit après traitement («révolution thérapeutique ») est associé à l'induction ou la potentialisation de réponses immunes à médiation cellulaire faisant intervenir les lymphocytes T-$CD4^+$ et T-$CD8^+$ (comme décrit par exemple dans LECHNER, F. et al., Eur. J. Immunol., 30: 2479-2487 (2000) et dans Thimme R et al., 2001, J. Exp. Med., 194(10): 1395-1406).

Les molécules du complexe majeur d'histocompatibilité (CMH ou autrement appelé HLA chez l'homme) sont dites de classe I ou de classe II. Les molécules de classe I sont exprimées sur la quasi-totalité des cellules nucléées et sont capables de présenter des épitopes ou peptides aux de lymphocytes T cytotoxiques (CTL) $CD8^+$. Les molécules de classe II sont capables de présenter des épitopes aux cellules T $CD4^+$, mais leur expression est restreinte aux cellules présentatrices d'antigène.

Les vaccins contre le virus de l'hépatite C actuellement envisagés sont basés sur l'utilisation de protéines recombinantes adjuvantées, de peptides, de vecteurs d'expression parmi lesquels on peut citer les vecteurs d'origine virale ou bactérienne ou d'ADN nu. Dans ce cas, une ou plusieurs protéines virales ou un ou plusieurs gènes codant pour ces protéines virales sont utilisés.

Lorsque plusieurs protéines virales ou un ou plusieurs gènes codant pour ces protéines virales sont sélectionnés, ceux-ci sont souvent constitués soit par une partie ou l'ensemble des protéines structurales (Makimura et al., 1996, Vaccine, 14: 28-34; Fournillier A., et al, 1999, J. Virology, 73: 7497-7504), soit par les protéines non structurales individuelles ou comprenant au moins deux protéines contiguës (Brinster et al., 2001, Hepatology, 34 : 1206-1217), soit par un mélange de protéines structurales et non structurales (Pancholi et al., 2003, J. Virology, 77 :382-390).

La demande de brevet WO99/38880 décrit l'utilisation de trois gènes codant séparément pour les trois protéines NS3, NS4 et NS5 (a et b) dans une composition vaccinale comprenant trois vaccins ADN exprimant chacun séparément ces trois protéines. Les auteurs montrent chez la souris l'induction de lymphocytes T spécifiques des trois antigènes. Seul le vaccin exprimant NS5a et b a été testé *in vivo* dans un test de protection.

La demande de brevet WO01/30812 décrit quant à elle l'utilisation d'une protéine de fusion constituée des protéines non structurales NS3, NS4 et NS5a, le cas échéant en association avec la protéine non structurale NS5b. Les auteurs ont indiqué que cette association permettait d'activer les cellules T spécifiques de VHC. Cette demande de brevet décrit simplement la capacité de formulations vaccinales (type ADN nu, adénovirus recombinant ou virus de la vaccine recombinant) exprimant la protéine de fusion NS3 NS4 NS5a ou la protéine NS5a à induire des réponses immunitaires spécifiques et médiées par des lymphocytes T spécifiques.

Cho et al (Vaccine. 1999 Mar 5;17(9-10):1136-44) décrivent une méthode de vaccination utilisant un vecteur d'expression contenant la moitié 3' du génome du VHC (nucléotides 3395 à 9391, comme indiqué dans le chapitre « 2.1 plasmid construction », page 1137), et codant pour une polyprotéine constituées des 4 polypeptides NS3, NS4, NS5a et NS5b, le polypeptide NS5a étant nécessairement présent.

La Demanderesse a maintenant mis en évidence, contre toute attente, que l'association particulière des protéines non structurales NS3, NS4 et NS5b, NS3 et NS4 étant exprimées de façon colinéaire, présentait un meilleur pouvoir immunogène et protecteur supérieur à celui obtenu avec un vaccin incluant, outre ces protéines non structurales, également la protéine NS5a et/ou d'autres protéines structurales du VHC telles que core, E1 ou E2, et avait un effet sur la capacité des cellules provenant de patients infectés par des souches virales à induire des réponses immunitaires spécifiques.

Ainsi, la présente invention a pour objet une composition peptidique constituée d'une polyprotéine NS3/NS4 du virus de l'hépatite C, ainsi que d'un polypeptide NS5b du virus de l'hépatite C.

Elle a également pour objet, les vecteurs incluant les séquences nucléotidiques codant pour cette composition peptidique, tels que les adénovirus et les poxvirus, ainsi que les microorganismes ou cellules hôtes transformés par ces vecteurs.

Elle a enfin pour objet l'utilisation de la composition peptidique et des vecteurs pour la préparation d'un médicament destiné à l'inhibition ou le contrôle d'une infection provoquée par le virus de l'hépatite C, et dans une composition vaccinale.

La présente invention telle que définie dans les revendications propose donc une nouvelle composition peptidique constituée d'une polyprotéine NS3/NS4 et d'un polypeptide NS5b du VHC, laquelle composition a la capacité de stimuler une réponse immunitaire à médiation cellulaire spécifique du VHC, de sorte qu'elle est utile dans le domaine de la vaccination prophylactique et thérapeutique dirigée contre le virus de l'hépatite C.

La polyprotéine NS3/NS4 de la composition peptidique de l'invention est constituée de la protéine NS3 et de la protéine NS4a et b, sans interruption dans la séquence peptidique, comme dans la polyprotéine native. En effet, comme indiqué précédemment, le génome du VHC contient un seul cadre de lecture ouvert qui est transcrit en une polyprotéine. Cette polyprotéine du VHC peut être clivée pour produire au moins dix parties distinctes, dans l'ordre $NH_2$-Core-E1-E2-p7-NS2-*NS3-NS4a-NS4b*-NS5a-NS5b-COOH.

La protéine NS3 est une protéine de 630 acides aminés qui apparaît approximativement de l'acide aminé 1027 à l'acide aminé 1657 de la polyprotéine. La protéine NS4, protéine de 314 acides aminés, quant à elle apparaît approximativement de l'acide aminé 1658 à l'acide aminé 1972 (numérotation par rapport au VHC-1) (Choo et al., 1991, Proc. Natl. Acad. Sci., vol 88:2451-2455). La polyprotéine NS3/NS4 apparaît donc approximativement de l'acide aminé 1027 à l'acide aminé 1972.

S'agissant du polypeptide NS5b également contenu dans la composition de l'invention, il est constitué de 590 acides aminés et apparaît approximativement de l'acide aminé 2421 à l'acide aminé 3011 de la polyprotéine (Choo et al., 1991, *supra*).

La protéine NS3 comprend deux domaines structuraux distincts, à savoir un domaine N-terminal doté d'une activité protéasique à sérine active intervenant dans la maturation de la polyprotéine virale et un domaine C-terminal comprenant une activité hélicase associée à une activité NTPasique qui joue un rôle dans la réplication du génome viral.

Par « polyprotéine NS3/NS4 » et « polypeptide NS5b », on entend bien entendu les polyprotéines et polypeptides ayant les séquences en acides aminés natives, provenant de toute souche et isolat du VHC, ainsi que leurs analogues, mutéines et homologues.

Par « analogues » ou » mutéines » de la polyprotéine et du polypeptide, on entend les dérivés biologiquement actifs des molécules de référence qui présentent l'activité souhaitée, à savoir la capacité à stimuler une réponse immunitaire à médiation cellulaire comme défini ci-dessus.

De façon générale, le terme « analogue » se réfère à des composés ayant une séquence et une structure polypeptidique native présentant une ou plusieurs additions, substitutions (généralement conservatrice en termes de nature) et/ou délétions d'acide aminé, par rapport à la molécule native, dans la mesure où les modifications ne détruisent pas l'activité immunogène. Par le terme «mutéine », on entend les peptides présentant un ou plusieurs éléments imitant le peptide (« peptoïdes »), tels que ceux décrits dans la demande de brevet PCT WO91/04282. De préférence, l'analogue ou la mutéine ont au moins la même immunoactivité que la molécule native. Des procédés de préparation d'analogues et mutéines polypeptidiques sont connus de l'homme du métier et sont décrits ci-dessous.

Les analogues particulièrement préférés incluent les substitutions conservatrices en nature, c'est-à-dire les substitutions qui prennent place dans une famille d'acides aminés. Spécifiquement, les acides aminés sont généralement divisés en 4 familles, à savoir (1) les acides aminés acides tels que l'aspartate et le glutamate, (2) les acides aminés basiques tels que la lysine, l'arginine et l'histidine, (3) les acides aminés non polaires tels que l'alanine, la leucine, l'isoleucine, la proline, la phénylalanine, la méthionine et le tryptophane et (4) les acides aminés non chargés polaires tels que la glycine, l'asparagine, la glutamine, la cystéine, la sérine, la thréonine et la tyrosine. La phénylalanine, le tryptophane et la tyrosine sont parfois classés en acides aminés aromatiques. Par exemple, on peut prédire de façon raisonnable qu'un remplacement isolé de leucine par de l'isoleucine ou de la valine, d'un aspartate par un glutamate, d'une thréonine par une sérine, ou un remplacement conservateur similaire d'un acide aminé par un autre acide aminé ayant un rapport structurel, n'aura pas d'effet majeur sur l'activité biologique. L'homme du métier déterminera facilement les régions de la molécule peptidique d'intérêt qui peuvent tolérer un changement par référence à aux plots Hopp/Woods et Kyte-Doolite, biens connus dans la technique.

Par « homologie », on entend le pourcentage d'identité entre deux molécules peptidiques, telles que polyprotéines et polypeptides. Deux séquences d'acides aminés sont « sensiblement homologues » l'une par rapport à l'autre lorsque les séquences présentent au moins 60%, de préférence au moins 75%, de préférence encore au moins 80-85%, de préférence encore au moins 90% et d'avantage préféré au moins 95-98% ou plus d'identité de séquence sur une longueur définie des molécules peptidiques.

De manière générale, le terme « identité » se réfère à une correspondance exacte acide aminé par acide aminé de deux séquences peptidiques. Le pourcentage d'identité peut être déterminé par une comparaison directe de l'information de séquence entre deux molécules en alignant les séquences, en comptant le nombre exact de mésappariements entre les deux séquences alignées, en divisant par la longueur de la séquence la plus courte et en multipliant le résultat par 100. Le pourcentage d'identité peut également être déterminé à l'aide de programmes d'ordinateurs tels que ALIGN, Dayhoff, M.O. dans Atlas of Protein Sequence and Structure M.O. Dayhoff ed., 1981, 5 Suppl., 3: 482-489.

Les séquences d'acide nucléique et en acides aminés d'un certains nombre de souches et isolats du VHC, et en particulier de la protéine NS3, de la protéine NS4 et du polypeptide NS5b, ont déjà été déterminées.

Par exemple, l'isolat HCV-J1 est décrit dans Okamoto H. et al., 1992, Nucleic Acids Res., 20: 6410-6410. Les séquences codantes complètes de deux isolats indépendants du VHC, à savoir les isolats HCV-J et -BK, ont été décrits respectivement dans Kato et aL, 1990, Proc. Natl. Acda., Sci., 87 : 9524-9528 et dans Takamizawa et al., 1991, J. Virol., 65 : 1105-1113. S'agissant de l'isolat HCV-1, il est décrit dans Choo et al., 1990, Brit. Med. Bull., 46 : 423-441 et dans Choo et al., 1991, *supra.* L'isolat HVC-H a été décrit dans Inchauspé G. et al;, 1991, Proc. Natl. Acad. Sci., 88: 10292-10296. L'isolat HCV-G9 a été décrit dans Okamoto H., et al., 1994, J. Gen. Virol., 45 : 629-635. Les isolats HCV-J6 et -J8 ont été décrits respectivement dans Okamoto H., et al., 1991, J. Gen. Virol., 72 : 2697-2704 et Okamoto H., et al., 1992, Virology, 188 : 331-341. L'isolat HVC-BEBE1 a été décrit dans Nako H., et al., 1996, J. Gen. Virol., 141 : 701-704 et l'isolat HCV-NZL1 a été décrit dans Sakamoto M., et al., 1994, J. Gen. Virol., 75 : 1761-1768. S'agissant de l'isolat HCV-Tr, il a été décrit dans Chayama K., et al., 1994, J. Gen. Virol., 75 : 3623-3628. Les isolats HCV-ED43 et -EUH1480 ont été décrits respectivement dans Chamberlain R.W., et al., 1997, J. Gen. Virol., 78 : 1341-1347 et Chamberlain RW., et al., 1997, Biochem. Biophys. Res. Commun., 236 : 44-49. L'isolat HCV-EUHK2 a été décrit dans Adams A., et al., 1997, Biochem. Biophys. Res. Commun., 234 : 393-396. Les isolats HCV-VN235, -VN405 et -VN004 ont été décrits dans Tokita H., et aL, 1998, J. Gen. Virol., 79 : 1847. Enfin, s'agissant des isolats HCV-JK049 et -JKD46, ils ont été décrits dans Tokita H. et al., 1996, J. Gen. Virol., 77 : 293-301.

Les souches et isolats du VHC, tel qu'illustrés ci-dessus, peuvent présenter des génotypes différents, à savoir des génotypes la (isolats HCV-1, -J1 et -H), 1b (isolats HCV-J et BK), 1c (isolat HCV-G9), 2a (isolat HCV-J6), 2b (isolat HCV-J8), 2c (isolat HCV-BEBE1), 3a (isolat HCV-NZL1), 3b (isolat HCV-Tr), 4a (isolat HCV-ED43), 5a (isolat HCV-EUH1480), 6a (isolat HCV-EUHK2), 7b (isolat HCV-VN235), 8b (isolat HCV-VN405), 9a (isolat HCV-VN004), 10a (isolat HCV-JK049) et 11a (isolat HCV-JK046).

Selon un mode de réalisation de l'invention, NS3 et/ou NS4 et/ou NS5b proviennent de virus de génotypes différents.

Selon un autre mode de réalisation, NS3 et/ou NS4 et/ou NS5b proviennent de virus de même génotype, de préférence de génotype 1b.

La polyprotéine NS3/NS4 et le polypeptide NS5b contenus dans la composition peptidique de l'invention peuvent être soit d'origine native, soit d'origine recombinante.

La polyprotéine NS3/NS4 et le polypeptide NS5b d'origine native sont obtenus à partir des souches ou isolats du VHC, par le biais de l'utilisation d'amorces oligonucléotidiques synthétiques qui vont servir à amplifier les séquences virales natives, soit à partir de sera de patients infectés par le ou les génotypes viraux ciblés, soit à partir d'ARN viral déjà purifié, provenant par exemple de sang ou de foie de patients, soit à partir d'ADN complémentaire libre ou cloné au préalable dans un vecteur d'expression, soit encore à partir de particules virales purifiées à partir de prélèvements biologiques ou de système de propagation *in vitro.*

La polyprotéine NS3/NS4 et le polypeptide NS5b de l'invention d'origine recombinante peuvent également être obtenus par la technique du génie génétique qui comprend les étapes de :

- culture d'un microorganisme ou de cellules eucaryotes transformé(es) à l'aide d'une séquence nucléotidique codant pour ladite polyprotéine NS3/NS4 ou pour ledit polypeptide NS5b et
- récupération du peptide produit par ledit microorganisme ou lesdites cellules eucaryotes.

Cette technique est bien connue de l'homme du métier. Pour plus de détails la concernant, on pourra se référer à l'ouvrage ci-après : Recombinant DNA Technology I, Editors Ales Prokop, Raskesh K Bajpai; Annals of the New-York Academy of Sciences, Volume 646, 1991.

Les séquences nucléotidiques codant pour la polyprotéine NS3/NS4 et le polypeptide NS5b peuvent être préparées par synthèse chimique couplée à une approche de génie génétique ou par génie génétique seul, en utilisant les techniques bien connues de l'homme du métier et décrites par exemple dans Sambrook J. et al., Molecular Cloning : A Laboratory Manual, 1989.

Les séquences nucléotidiques codant pour la polyprotéine NS3/NS4 et le polypeptide NS5b peuvent être insérées dans des vecteurs d'expression dans un système d'expression adapté, afin d'obtenir la composition peptidique de l'invention.

Bien entendu, les séquences nucléotidiques peuvent être insérées dans un seul vecteur d'expression ou bien dans deux vecteurs d'expression différents. Dans ce dernier cas, la séquence codant pour la polyprotéine NS3/NS4 est insérée dans l'un des deux vecteurs et la séquence codant pour le polypeptide NS5b est insérée dans l'autre vecteur, ces deux vecteurs pouvant être de nature identique ou différente.

Ainsi, un autre objet de l'invention consiste en les vecteurs d'expression pour l'expression de séquences nucléotidiques du virus de l'hépatite C caractérisé en ce que lesdites séquences nucléotidiques du virus de l'hépatite C sont constituées par une séquence nucléotidique codant pour la polyprotéine NS3/NS4 et une séquence nucléotidique codant pour le polypeptide NS5b, ainsi que les moyens nécessaires à leur expression , ladite séquence nucléotidique codant pour la polyprotéine NS3/NS4 et ladite séquence nucléotidique codant pour le polypeptide NS5b étant dans un seul vecteur d'expression.

On entend par moyen nécessaire à l'expression d'un peptide, le terme peptide étant utilisé pour toute molécule peptidique, telle que protéine, polyprotéine, polypeptide, etc., tout moyen qui permet d'obtenir le peptide, tel que notamment un promoteur, un terminateur de transcription, une origine de réplication et de préférence un marqueur de sélection.

Les moyens nécessaires à l'expression d'un peptide sont liés de façon opérationnelle à la séquence d'acide nucléique codant pour le peptide d'intérêt. Par « liés de façon opérationnelle », on entend une juxtaposition desdits éléments nécessaires à l'expression et du gène codant pour le peptide d'intérêt, lesquels sont en une relation telle que cela leur permet de fonctionner de façon attendue. Par exemple, ils peut exister des bases supplémentaires entre le promoteur et le gène d'intérêt tant que leur relation fonctionnelle est préservée.

Les moyens nécessaires à l'expression d'un peptide peuvent être des moyens homologues, c'est-à-dire inclus dans le génome du vecteur utilisé, ou bien être hétérologues. Dans ce dernier cas, lesdits moyens sont clonés avec le peptide d'intérêt à exprimer.

Des exemples de promoteurs hétérologues comprennent (i) les promoteurs viraux tels que le promoteur SV40 (Virus simien 40), le promoteur du gène de la thimidine-kinase du virus simplex de l'Herpès (TK-HSV-1), le LTR du virus du sarcome de Rous (RSV), le promoteur premier immédiat du cytomégolovirus (CMV) et le promoteur dernier majeur adénoviral (MLP), ainsi que (ii) tout promoteur cellulaire qui contrôle la transcription des gènes codant pour des peptides chez des eucaryotes supérieurs, tel que le promoteur du gène de phosphoglycérate-kinase (PGK) constitutif (Adra et al., 1987, Gene, 60: 65-74), le promoteur des gènes spécifiques du foie alphal-antitrypsine et FIX et le promoteur SM22 spécifique des cellules du muscle lisse (Moessler et aL, 1996, Development, 122: 2415-2425)

Selon un mode de réalisation de l'invention, les séquences nucléotidiques codant pour ladite polyprotéine NS3/NS4 et ledit polypeptide NS5b sont issus de génotypes différents.

Selon un autre mode de réalisation, les séquences nucléotidiques codant pour ladite polyprotéine et ledit polypeptide sont issus d'un virus de même génotype, de préférence le génotype 1b.

Là encore, on entend par «séquence nucléotidique », toutes les séquences codant pour la polyprotéine NS3/NS4 et le polypeptide NS5b natifs, ainsi que pour leurs analogues, mutéines et homologues, tels que définis précédemment.

Lesdites séquences contenues dans le vecteur d'expression peuvent être liées directement entre elles sous le contrôle d'un seul promoteur et/ou d'un seul élément régulateur de l'expression, ou bien elles peuvent être séparées en étant sous la dépendance chacune de promoteurs et/ou régulateurs de l'expression indépendants, identiques ou différents.

A titre de vecteur d'expression qui conviennent aux fins de l'invention, on peut citer par exemple les plasmides, les vecteurs viraux type adenovirus, poxvirus, virus de la vaccine, baculovirus, les vecteurs bactériens du type salmonelle, BCG.

Les adénovirus ont été détectés dans de nombreuses espèces animales, ne s'intègrent pas et sont peu pathogènes. Ils sont capables d'infecter une variété de types cellulaires, les cellules en division et les cellules en repos. Ils possèdent un tropisme naturel pour les épithéliums bronchiques. De plus, ils ont été utilisés en tant que vaccins entériques vivants pendant de nombreuses années avec un excellent profile de sécurité. Enfin, on peut les faire pousser facilement et les purifier en grande quantité. Ces caractéristiques ont fait que les adénovirus sont particulièrement appropriés pour une utilisation en tant que vecteurs d'expression et notamment en tant vecteurs de thérapie génique à des fins thérapeutiques et vaccinales.

Selon un mode de réalisation préféré, le vecteur de l'invention est un adénovirus.

Des exemples d'adénovirus à utiliser dans la présente invention peuvent être dérivés de toute source d'origine humaine ou animale, en particulier d'origine canine (par exemple CAV-1 ou CAV-2 ; référence Genbank CAV1GENOM et CAV77082, respectivement), d'origina avienne (référence Genbank AAVEDSDNA), d'origine bovine (telle que BAV3, Seshidhar Reddy et al., 1998, J. Virol., 72: 1394-1402), d'origine ovine, féline, porcine, d'origine simienne, ou bien d'un de leurs hybrides. Tout sérotype peut être utilisé. Toutefois, les adénovirus d'origine humaine sont préférés et en particulier l'adénovirus 5 (AdIV).

De façon générale, les virus cités sont disponibles dans les collections ATCC et ont fait l'objet de nombreuses publications décrivant leur séquence, leur organisation et leur biologie, ce qui permet à l'homme du métier de les appliquer facilement. Par exemple, la séquence de l'adénovirus type 5 est décrite dans la base de donnée Genbank (M73260 et M29978).

Le génome des adénovirus est constitué d'une molécule d'ADN linéaire double brin d'environ 36 kb portant plus d'environ 30 gènes nécessaires pour terminer le cycle viral. Les premiers gènes sont divisés en 4 régions dispersées dans le génome de l'adénovirus (E1 à E4). Les régions E1, E2 et E4 sont essentielles pour la réplication virale. La région E3 est considérée comme une région non essentielle sur la base de l'observation que les virus mutants apparaissant naturellement ou les virus hybrides ayant perdu cette région E3 continuent à se répliquer comme les virus de type sauvage dans les cellules cultivées (Kelly et Lewis, 1973, J. Virol., 12 :643-652). Les derniers gènes (L1 à L5) codent en majorité pour les protéines structurales constituant la capside virale. Ils chevauchent au moins en partie les premiers motifs de transcription et sont transcrits à partir d'un promoteur unique (MLP pour « Major Late Promoter »). De plus, le génome adénoviral porte aux deux extrémités des régions à action en cis essentielles pour la réplication d'ADN, respectivement les motifs de répétition inversés 5' et 3' (ITRs pour « Inverted Terminal Repeats ») et une séquence d'empaquetage.

Les adénovirus actuellement utilisés dans les protocoles de thérapie génique sont dénués de la majorité de la région E1, ce qui rend les virus déficients au niveau de leur réplication pour éviter leur dissémination dans l'environnement et dans l'organisme hôte. En outre, la plupart des adénovirus sont également dénués de la région E3 afin d'accroître leur capacité de clonage. La faisabilité du transfert de gène en utilisant ces vecteurs a été démontrée dans une variété de tissus *in vivo* (voir par exemple Yei et al., 1994, Hum. Gene Ther., 5 : 731-744 ; Dai et al., 1995, Proc. Natl. Acad Sci. USA, 92: 1401-1405 ; US6,099,831 ; et US6,013,638).

De préférence, les promoteurs utilisés dans les adénovirus comme vecteur d'expression, sont des promoteurs hétérologues tels que les promoteurs le CMV et le SV40.

De préférence encore, le promoteur CMV est le promoteur de la polyprotéine NS3/NS4 et le vecteur d'expression comprend comme séquence nucléotidique codant pour ladite polyprotéine la cassette d'expression CMV-NS3-NS4.

Par « cassette d'expression », on entend une séquence d'ADN contenant un promoteur et un cadre de lecture ouvert pour l'expression du peptide d'intérêt, à insérer dans un vecteur.

De préférence également, le promoteur SV40 est le promoteur du polypeptide NS5b et le vecteur d'expression comprend comme séquence nucléotidique codant pour ledit polypeptide la cassette d'expression SV40-NS5b.

Selon un mode de réalisation de l'invention, le génome de l'adénovirus est modifié de façon à remplacer la région E1 par la cassette d'expression CMV-NS3-NS4 et à remplacer la région E3 par la cassette d'expression SV40-NS5b.

Les méthodes de suppression et d'insertion de séquences d'ADN dans des vecteurs d'expression sont largement connues de l'homme du métier et consistent notamment en des étapes de digestion enzymatique et ligature.

Un autre vecteur d'expression particulièrement approprié aux fins de l'invention est un poxvirus, lequel constitue un autre mode de réalisation de l'invention.

Les poxvirus constituent un groupe de virus complexe enveloppés, se distinguant principalement par leur morphologie inhabituelle, leur grand génome d'ADN et leur site cytoplasmique de réplication. Le génome de plusieurs éléments des *poxviridae,* comprenant la souche virale de la vaccine de Copenhagen (VV) (Goebel et aL, 1990, Virol. 179 : 247-266 et 517-563) et la souche du virus de la vaccine modifié d'Ankara (MVA) (Antoine et aL, 1998, Virol., 244: 635-396), a été cartographié et séquencé. La souche VV possède un génome d'ADN double brin d'environ 192 kb codant pour environ 200 protéines dont approximativement 100 sont impliquées dans l'assemblage du virus. La souche MVA est une souche du virus de la vaccine hautement atténuée, générée par plus de 500 passages en série de la souche d'Ankara du virus de la vaccine (CVA) sur des fibroblastes d'embryons de poulet (Mayr et aL, 1975, Infection, 3: 6-16). Le virus MVA a été déposé devant la Collection Nationale de Cultures de Microorganismes (CNCM) sous le numéro I-721. La détermination de la séquence complète du génome du MVA et la comparaison avec celui du VV permet l'identification précise des altérations qui sont apparues dans le génome viral et la définition de sept délétions (I à VII) et de nombreuses mutations conduisant à des cadres de lecture ouverts fragmentés (Antoine et al., 1998, Virology, 244 : 365-396).

D'autres exemples de poxvirus appropriés aux fins de l'invention comprennent le pox du canari, le pox de volaille, le pox de vache, l'entomopox, le pox de singe, le pox de porc et le pox de pingouin.

Le poxvirus se trouve sous deux formes morphologiquement distinctes, appelées virus mature intracellulaire (IMV) et virus extracellulaire enveloppé (EEV).

Le poxvirus utilisé comme vecteur d'expression de l'invention présente au moins l'une des caractéristiques suivantes, prises seules ou en association :

(i) le poxvirus est un virus MVA,
(ii) le poxvirus est sous forme morphologique IMV, et
(iii) le génome du poxvirus est modifié de façon à insérer la cassette d'expression NS3/NS4 et à insérer la cassette d'expression NS5b.

Lorsque le génome du poxvirus est modifié de façon à insérer les deux cassettes d'intérêt, les moyens nécessaires à leur expression sont homologues. Ainsi, dans le cas où on utilise le virus MVA, l'expression de NS3/NS4 peut être par exemple sous le contrôle du promoteur ph5r de sorte que la cassette d'expression correspondant est ph5r-NS3-NS4, et l'expression de NS5b peut être par exemple sous le contrôle du promoteur p7.5 de sorte que la cassette d'expression correspondante est p7.5-NS5b, et vice et versa.

Selon un mode de réalisation particulier, lorsque le génome du poxivirus est modifié de façon à insérer les deux cassettes d'intérêt, les deux dites cassettes d'expression sont orientées dans le même sens.

Selon un autre mode de réalisation particulier, elles sont orientées en sens opposé.

Là encore, les cassettes d'expression sont insérées dans le génome du poxvirus de façon connue par l'homme du métier, comme indiqué précédemment.

Les vecteurs de l'invention peuvent également comprendre des séquences nécessaires au ciblage des peptides vers des compartiments cellulaires particuliers. Un exemple de ciblage peut être le ciblage vers le réticulum endoplasmique obtenu en utilisant des séquences d'adressage du type de la séquence leader issue de la protéine E3 de l'adénovirus (Ciernik I.F., et aL, The Journal of Immunology, 1999, 162, 3915-3925).

Ils peuvent également comprendre des séquences nécessaires au ciblage vers les cellules dendritiques et au ciblage à la membrane des cellules.

L'invention a également pour objet les microorganismes et les cellules eucaryotes transformés par un vecteur d'expression de l'invention.

A titre d'exemples de microorganisme qui conviennent aux fins de l'invention, on peut citer les levures, telles que celles des familles suivantes: *Saccharomyces, Schizosaccharomyces, Kluveromyces, Pichia, Hanseluna, Yarowia, Schwaniomyces, Zygosaccharomyces, Saccharomyces cerevisiae, Saccharomyces carlsbergensis* et *Kluveromyces lactis* étant préférées ; et les bactéries, telles que *E. coli* et celles des familles suivantes: *Lactobacillus, Lactococcus, Salmonella, Strptococcus, Bacillus et Streptomyces.*

A titre d'exemples de cellules eucaryotes, on peut citer les cellules provenant d'animaux tels que les mammifères, les reptiles, les insectes et équivalent. Les cellules eucaryotes préférées sont les cellules provenant du hamster chinois (cellules CHO), du singe (cellules COS et Vero), du rein de hamster nain (cellules BHK), du rein de cochon (cellules PK 15) et du rein de lapin (cellules RK13, les lignées cellulaires humaines de l'ostéosacorme (cellules 143 B), les lignées cellulaires humaines HeLa et les lignées cellulaires humaines de l'hépatome (du type cellules Hep G2), ainsi que les lignées cellulaires d'insecte (par exemple de Spodoptera frugiperda).

Les cellules hôtes peuvent être fournies dans des cultures en suspension ou en flacon, dans des cultures tissulaires, des cultures d'organe et équivalent. Les cellules hôtes peuvent également être des animaux transgéniques.

La présente description concerne également des anticorps dirigés contre l'une des compositions peptidiques de l'invention telles que définies précédemment ou bien contre l'un des vecteurs d'expression de l'invention tels que définis précédemment.

Les anticorps sont soit des anticorps polyclonaux, soit monoclonaux.

Les anticorps polyclonaux susmentionnés peuvent être obtenus par immunisation d'un animal avec la composition peptidique de l'invention ou bien avec le vecteur de l'invention à titre «d'antigène d'intérêt », suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixée un antigène spécifiquement reconnu par les anticorps, notamment un antigène viral d'intérêt

Les anticorps monoclonaux peuvent être obtenus par la technique des hybridomes dont le principe général est rappelé ci-après.

Dans un premier temps, on immunise un animal, généralement une souris, (ou des cellules en culture dans le cadre d'immunisations *in vitro*) avec la composition peptidique de l'invention ou bien avec le vecteur de l'invention à titre «d'antigène d'intérêt », dont les lymphocytes B sont alors capables de produire des anticorps contre ledit antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de l'antigène d'intérêt pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les compositions peptidiques, les vecteurs d'expression, les séquences nucléotidiques codant pour ladite polyprotéine NS3/NS4 et ledit polypeptide NS5b, ainsi que les anticorps sont particulièrement efficaces pour l'inhibition, la prévention et le contrôle de l'infection des patients porteurs du virus du VHC, de sorte que leur utilisation pour la préparation d'un médicament constitue un autre objet de l'invention.

La présente invention concerne également une composition pharmaceutique, notamment vaccin, contenant à titre de substance active la composition peptidique de l'invention, ou bien un vecteur d'expression de l'invention, ou bien un vecteur d'expression comprenant une séquence nucléotidique codant pour la polyprotéine NS3/NS4 avec un vecteur d'expression comprenant une séquence nucléotidique codant pour le polypeptide NS5b, ou bien les séquences nucléotidiques codant pour ladite polyprotéine NS3/NS4 et ledit polypeptide NS5b, lesdites séquences nucléotidiques correspondant aux séquences contenues dans les vecteurs d'expression de l'invention, placées sous le contrôle d'éléments nécessaires à une expression constitutive et/ou inductible desdits peptides, ou bien l'un au moins des anticorps susmentionnés.

Par éléments nécessaires à une expression constitutive des peptides, on entend un promoteur ubiquitaire ou spécifique des cellules eucaryotes.

A titre d'éléments nécessaires à une expression inductible des peptides, on peut citer les éléments de régulation de l'opéron de *E. coli* pour la résistance à la tétracycline (Gossen M. et al, Proc Natl Acad Sci USA, 89 : 5547-5551 (1992).

Selon un mode de réalisation particulier de l'invention, la composition pharmaceutique contient également un véhicule pharmaceutiquement approprié. Bien entendu, l'homme du métier déterminera facilement la nature du véhicule pharmaceutiquement approprié et la quantité de polypeptides à utiliser en fonction des constituants de la composition pharmaceutique.

La quantité et la nature du véhicule pharmaceutiquement approprié peuvent être facilement déterminées par l'homme du métier. Elles sont choisies selon la forme pharmaceutique et le mode d'administration souhaités.

Les compositions pharmaceutiques de l'invention sont appropriées pour l'administration orale, sublinguale, sous-cutanée, intramusculaire, intraveineuse, topique, locale, intratrachéale, intranasale, transdermique, rectale, intraoculaire, intra-auriculaire, ledit principe actif pouvant être administré sous forme unitaire d'administration.

Les formes unitaires d'administration peuvent être par exemple des comprimés, des gélules, des granules, des poudres, des solutions ou suspensions orales injectables, des timbres transdermiques (« patch »), des formes d'administration sublinguale, buccale, intratrachéale, intraoculaire, intranasale, intra-auriculaire, par inhalation, des formes d'administration topique, transdermique, sous-cutanée, intramusculaire ou intraveineuse, des formes d'administration rectale ou des implants. Pour l'administration topique, on peut envisager des crèmes, gels, pommades, lotions ou collyres.

Ces formes galéniques sont préparées selon les méthodes usuelles des domaines considérés.

Lesdites formes unitaires sont dosées pour permettre une administration journalière de 0,001 à 10 mg de substance active par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse du patient.

Les compositions pharmaceutiques de l'invention contiennent de préférence à titre de substance active un des vecteurs de l'invention ou bien un vecteur d'expression comprenant une séquence nucléotidique codant pour la polyprotéine NS3/NS4 avec un vecteur d'expression comprenant une séquence nucléotidique codant pour le polypeptide NS5b, de sorte qu'elles sont utiles en vaccination prophylactique et thérapeutique.

La vaccination prophylactique et thérapeutique peut être mise en oeuvre par injection d'un vaccin à base d'un ou plusieurs vecteurs d'expression de l'invention, dans la mesure où le ou les vecteurs d'expression codent au final pour la polyprotéine NS3/NS4 et pour le polypeptide NS5b à titre de substance active, injection suivie de rappels ou non. Elle peut également être mise en oeuvre en injectant deux types de vecteurs d'expression de l'invention différents, tout d'abord un adénovirus, puis un poxvirus, de façon simultanée ou différée dans le temps, et vice et versa.

Ces vecteurs peuvent être contenus dans un kit pharmaceutique.

Aussi, un autre objet de l'invention consiste en des kits pharmaceutiques, notamment vaccinaux, comprenant au moins un vecteur d'expression comprenant une séquence nucléotidique codant pour la polyprotéine NS3/NS4 et au moins un vecteur d'expression comprenant une séquence nucléotidique codant pour le polypeptide NS5b.

Un autre objet de l'invention consiste en des kits pharmaceutiques, notamment vaccinaux, comprenant au moins un vecteur d'expression de type adénovirus tel que défini précédemment et/ou au moins un vecteur d'expression de type poxvirus tel que défini précédemment.

La vaccination prophylactique et thérapeutique peut également être mise en oeuvre par injection d'un vaccin à base d'au moins un vecteur d'expression de l'invention, ou bien un vecteur d'expression comprenant une séquence nucléotidique codant pour la polyprotéine NS3/NS4 avec un vecteur d'expression comprenant une séquence nucléotidique codant pour le polypeptide NS5b, et d'au moins une composition pharmaceutique de l'invention constituée de la composition peptidique de l'invention ou des anticorps de l'invention. Elle peut également être mise en oeuvre par injection d'un vaccin à base d'au moins un vecteur d'expression de l'invention, ou bien un vecteur d'expression comprenant une séquence nucléotidique codant pour la polyprotéine NS3/NS4 avec un vecteur d'expression comprenant une séquence nucléotidique codant pour le polypeptide NS5b, et d'au moins une séquence nucléotidique codant pour la polyprotéine NS3/NS4 et pour le polypeptide NS5b.

Aussi, un autre objet de l'invention consiste en des kits pharmaceutiques, notamment vaccinaux, comprenant au moins un vecteur d'expression de l'invention, ou bien un vecteur d'expression comprenant une séquence nucléotidique codant pour la polyprotéine NS3/NS4 avec un vecteur d'expression comprenant une séquence nucléotidique codant pour le polypeptide NS5b, et au moins une composition pharmaceutique de l'invention ou au moins une séquence nucléotidique codant pour la polyprotéine NS3/NS4 et pour le polypeptide NS5b.

La présente invention sera mieux comprise à l'aide des exemples suivants donnés uniquement à titre illustratif et non limitatif, ainsi qu'à l'aide des figures 1 à 7 annexées, sur lesquelles :

- la figure 1A à 1K représente les cartes des différents plasmides utilisés pour l'obtention d'un adénovirus AdNS3NS4NS5b selon l'invention, sur lesquelles sont indiqués les sites des différentes enzymes de restriction et l'emplacement des fragments de séquence codant pour NS3/NS4 et pour NS5b,
- la figure 2A à 2H représente les cartes des différents plasmides utilisés pour l'obtention d'un poxvirus MVA NS3NS4NS5b selon l'invention, sur lesquelles sont indiqués les sites des différentes enzymes de restriction et l'emplacement des fragments de séquence codant pour NS3/NS4 et pour NS5b,
- la figure 3 donne la réponse cellulaire induite par l'adénovirus AdNS3NS4, soit selon le test CTL (figure 3A) où on a utilisé l'épitope GLL pour stimuler les splénocytes en culture et pour charger les cibles du CTL et dont le résultat est exprimé en pourcentage de lyse spécifique en fonction du rapport effecteur/cible, soit selon le test ELISPOT (figure 3B), spécifique pour l'épitope GLL, où le résultat est donné en nombre de spots/$10^6$ cellules,
- la figure 4 donne la réponse cellulaire induite par l'adénovirus AdNS5b selon le test ELISPOT, spécifique des épitopes ALY et KLP,
- la figure 5 donne la réponse cellulaire induite par l'adénovirus AdCE1E2 selon le test CTL où on a utilisé l'épitope DLM pour stimuler les splénocytes en culture et pour charger les cibles du CTL et dont le résultat est exprimé en pourcentage de lyse spécifique en fonction du rapport effecteur/cible,
- la figure 6 donne le titre de virus recombinant de la vaccine, résultant du test d'épreuve, en pfu/ml/mg ovaire, pour les 4 groupes de 8 souris immunisées par les différentes combinaisons d'adénovirus : AdNS3NS4 + AdNS5b (1$^{er}$ groupe), les adénovirus AdNS3NS4 + AdNS5b + AdNS5a (2$^{ème}$ groupe), les adénovirus AdNS3NS4 + AdNS5b +AdCE1E2 (3$^{ème}$ groupe) et l'adénovirus AdβGa1 (4$^{ème}$ groupe) et
- la figure 7 donne le titre de virus recombinant de la vaccine, résultant du test d'épreuve, en pfu/ml/mg ovaire, pour les 3 groupes de 8 souris immunisées par les différentes combinaisons d'adénovirus suivantes : AdNS3NS4NS5b (1$^{er}$ groupe), AdNS3NS4 + AdNS5b (2$^{ème}$ groupe) et AdβGa1 (3$^{ème}$ groupe).

**Exemple 1 : Préparation d'un adénovirus selon l'invention permettant l'expression des protéines NS3/NS4 et NS5b**

**1 Adénovirus**

Les adénovirus recombinants sont générés par transfection ($CaPO_3$) de la lignée de complémentation 293 (Graham, Smiley, et al. 1977) après linéarisation des génomes par PacI. Les virus recombinants se propagent et sont amplifiés sur cette même lignée, et leur purification est réalisée à partir des cellules infectées. Les cellules sont récupérées par centrifugation (1500 tpm, (tours par min), 10 min) et lysées par 3 cycles de congélation/décongélation. Le lysat cellulaire est clarifié par deux centrifugations (2000 tpm, 10 min; 8000 tpm, 15 min), puis purifié par deux ultracentrifugations successives. La première est réalisée sur un gradient de Chlorure de Césium (densités 1,4 et 1,25) à 30000 tpm pendant 1 heure. La seconde est réalisée sur un coussin de Chlorure de Césium (densité 1,34) à 35000 tpm pendant 18 heures. Les phases contenant les virions sont prélevées et diluées de moitié dans un tampon saccharose 60%. Les suspensions virales sont alors dialysées contre du tampon de formulation (pour 10 litres: 3423g de saccharose; 12,11g de Tris; 2,033g de $MgCl_2$;87,7g de NaCl), puis aliquotées. Leur titrage est réalisé par immunofluorescence indirecte sur cellules 293 infectées par différentes dilutions virales et marquées par un anticorps spécifique de la DNA-Binding Protein adénovirale (α72K B6-8) (Reich, Sarnow, et al. 1983).

**2 Préparation de l'adénovirus AdNS3NS4**

Cet adénovirus permet l'expression du gène codant pour la polyprotéine NS3/NS4 (SEQ ID N°1 et 2) sous le contrôle du promoteur CMV.

2.1 Amplification par PCR de la séquence nucléotidique codant pour la polyprotéine NS3/NS4

Pour ce faire, on a utilisé les oligonucléotides suivants :

oIV166: 5'-GGG GGG GCT ATG GCG CCT ATC ACG GCC TA-3' (SEQ ID N°9)
oIV171: 5'-GGG GGG ACG CGT TTA GCA TGG CGT GGA GCA GT-3' (SEQ ID N°10)

ainsi que les réactifs suivants :

*Taq* DNA Polymérase, tampon PCR, MgCb 1,5mM et dNTP 10mM (Invitrogen).

Les conditions de PCR ont été les suivantes :

5 min à 94°C, puis
30 cycles de la série : 45 s à 94°C, 45 s à 62°C et 1 min à 72°C, puis
10 min à 72°C

2.2 Insertion du fragment de PCR NS3/NS4 dans le plasmide de transfert pTG13387

On a effectué les étapes suivantes :

- Digestion enzymatique du plasmide pTG13387 (figure 1A, Transgène) par *Nhe*I/*Mlu*I (NheI, Invitrogen dans React 4 Buffer et MluI, Invitrogen dans React 3 Buffer)
- Digestion enzymatique du fragment NS3/NS4 par *Nhe*I/*Mlu*I
- Ligature(T4 DNA Ligase (Invitrogen) dans Reaction Buffer (Invitrogen)),
- Transformation bactérienne (souche 5K, Transgène)
- Sélection des clones bactériens sur milieu LB (Difco) + ampicilline (100 μg/ml, Duchefa)
- Maxi-préparation plasmidique (Qiagen, selon le protocole du fournisseur) d'un clone positif après analyse de restriction
- Analyse de restriction : digestion par *Sma*I (Invitrogen dans React 4 Buffer) et obtention de fragments de : 5450, 2164, 909, 214 et 180 pb
- Obtention du plasmide pIV315 délété de sa région E1 et contenant la séquence NS3/NS4 sous le contrôle du promoteur CMV (figure 1B).

2.3 Recombinaison homologue avec le génome adénoviral complet délété de sa région E3 contenu dans le plasmide pTG6624

On a effectué les étapes suivantes :

- Digestion enzymatique du plasmide obtenu ci-dessus pIV315 par *Pac*I/*Pvu*I (*Pac*I dans tampon NEB1, Biolabs et *Pvu*I dans React 7 Buffer, Invitrogen); isolement sur gel d'agarose du fragment contenant la cassette pCMV-NS3-NS4
- Digestion enzymatique du plasmide pTG6624 (figure 1C) par *Cla*I (dans React 1 Buffer, Invitrogen)
- Transformation bactérienne (souche BJ, Transgène) pour effectuer la recombinaison homologue entre les deux fragments plasmidiques
- Sélection des clones bactériens sur milieu LB + ampicilline (100 μg/ml)
- Maxi-préparation plasmidique (Qiagen) d'un clone positif après analyse de restriction
- Analyse de restriction : digestion par *Sma*I et obtention de fragments de : 2263, 621, 3814, 214, 2164, 909, 180, 2463, 6480, 1398, 4456, 1455, 3540, 3386, 230 et 3685 pb
- Obtention du génome adénoviral complet Adénovirus AdNS3NS4, délété de ses régions E3 et E1, cette dernière ayant été remplacée par la cassette d'expression pCMV-NS3-NS4 (pIV317, figure 1D).

**3 Préparation de l'adénovirus AdNS3NS4NS5b**

Cet adénovirus permet l'expression du gène codant pour la polyprotéine NS3/NS4 sous le contrôle du promoteur CMV et l'expression du gène codant pour le polypeptide NS5b sous le contrôle du promoteur SV40

3.1 Construction du plasmide de transfert permettant le clonage dans la région E3 de adénovirus d'une séquence codante sous le contrôle du promoteur CMV

On a mis en oeuvre les étapes suivantes :

- Digestion enzymatique du plasmide pTG4664 (figure 1E, Transgène) par *Bgl*II (dans React 3 Buffer, Invitrogen)
- Digestion enzymatique du plasmide pTG13074 (figure 1F, Transgène) par *BamH*I/*Bgl*II (dans React 3 Buffer, Invitrogen)
- Ligature (T4 DNA ligase), transformation bactérienne (souche 5K)
- Sélection des clones bactériens sur milieu LB + ampicilline (100 μg/ml)
- Maxi-préparation plasmidique (Qiagen) d'un clone positif après analyse de restriction
- Analyse de restriction: digestion par *Sma*I et obtention de fragments de : 4940, 1305 et 230 pb
- Obtention du plasmide pIV267 (figure 1G)
- Digestion du plasmide ainsi obtenu pIV267 par *Cla*I/*Mun*I (dans React 1 Buffer, Invitrogen)
- Traitement par la DNA Polymerase I, Large (Klenow) Fragment (dans React 2 Buffer, Invitrogen)
- Ligature (T4 DNA Ligase)
- Transformation bactérienne (souche 5K)
- Sélection des clones bactériens sur milieu LB + ampicilline (100 μg/ml)
- Maxi-préparation plasmidique (Qiagen)
- Analyse de restriction : digestion par *Sma*I et obtention de fragments de : 4692, 1305 et 230 pb
- Obtention du plasmide pIV270, plasmide de transfert permettant le clonage dans la région E3 de l'adénovirus d'une séquence codante sous le contrôle du promoteur CMV (figure 1H).

3.2 Remplacement du promoteur CMV par le promoteur SV40 dans pIV270

On a effectué les étapes suivantes :

- Amplification par PCR du fragment nucléotidique correspondant au promoteur SV40, à partir du plasmide commercial pcDNAHygro (Clonetech) grâce aux oligonucléotides suivants:
    - oIV232: 5'-GGG GGG AGA TCT CCA GCA GGC AGA AGT ATG-3' (SEQ ID N°11)
    - oIV233: 5'-GGG GGG GTC GAC CGA AAA GGG ATA TAC AAG CTC-3' (SEQ ID N°12)

et selon le mode opératoire décrit dans le point 2.1 ci-dessus, à ceci près qu'on a utilisé une température de 58°C à la place de 62°C

- Digestion enzymatique de pIV270 par *Bgl*II/*Sal*I (dans React 10 Buffer, Invitrogen)
- Digestion enzymatique du fragment de PCR par *Bgl*II/*Sal*I
- Ligature (T4 DNA ligase), transformation bactérienne (souche 5K)
- Sélection des clones bactériens sur milieu LB + ampicilline (100 μg/ml)
- Maxi-préparation plasmidique (Qiagen) d'un clone positif après analyse de restriction
- Analyse de restriction : digestion par *Sma*I et obtention de fragments de : 4692, 719, 80 et 230 pb
- Obtention du plasmide pIV330, plasmide de transfert permettant le clonage dans la région E3 de l'adénovirus d'une séquence codante sous le contrôle du promoteur SV40 (figure 1I).

3.3 Insertion du fragment de PCR NS5b dans le plasmide de transfert pIV330

On a effectué les étapes suivantes :

- Amplification par PCR de la séquence nucléotidique codant pour la protéine NS5b (SEQ ID N°3 et 4) grâce aux oligonucléotides suivants:
- oIV212: 5'-GGG GGG TCT AGA ATG TCA ATG TCC TAC ACA TGG AC-3' (SEQ ID N° 13)
- oIV218: 5'-GGG GGG TCT AGA TTA CCG GTT GGG GAG CAG GT-3' (SEQ ID N°14)
  et selon le mode opératoire décrit dans le point 2.1 ci-dessus, à ceci près qu'on a utilisé une température de 60°C à la place de 62°C
- Digestion enzymatique du plasmide oIV330 obtenu ci-dessus par *Xba*I (dans React 2 Buffer, Invitrogen)
- Digestion enzymatique du fragment de PCR par *Xba*I
- Ligature (T4 DNA Ligase), transformation bactérienne (souche 5K)
- Sélection des clones bactériens sur milieu LB + ampicilline (100 μg/ml)
- Maxi-préparation plasmidique (Qiagen) d'un clone positif après analyse de restriction
- Analyse de restriction : digestion par *Sma*I et obtention de fragments de : 4692, 1505, 760, 719 et 230 pb
- Obtention du plasmide pIV336, plasmide de transfert dans la délétion E3 contenant la séquence NS5b sous le contrôle du promoteur SV40 (figure 1J)

3.4 Recombinaison homologue avec le génome adénoviral recombinant pIV317 pour obtenir l'adénovirus du titre

On a mis en oeuvre les étapes suivantes :

- Digestion du plasmide pIV317 obtenu dans le point 2.3 ci-dessus par *Srf*I (dans Universal Buffer, Stratagene)
- Digestion du plasmide pIV336 obtenu dans le point 3.3 par *Nhe*I/*Sac*II (dans Buffer T, Amersham Pharmacia Biotech) et isolement sur gel d'agarose du fragment contenant la cassette pSV40-NS5b
- Transformation bactérienne (souche BJ) pour effectuer la recombinaison homologue entre les deux fragments plasmidiques
- Sélection des clones bactériens sur milieu LB + ampicilline (100 μg/ml)
- Maxi-préparation plasmidique (Qiagen) d'un clone positif après analyse de restriction
- Analyse de restriction : digestion par *Sma*I et obtention de fragments de : 6480, 4456, 3814, 3540, 3386, 2739, 2463, 2263, 2164, 1455, 1398, 1105, 909, 760, 719, 621, 230, 214 et 180 pb
- Obtention du génome adénoviral complet souhaité, délété de la région E1, celle-ci ayant été remplacée par la cassette d'expression pCMV-NS3-NS4, et délété de la région E3, celle-ci ayant été remplacée par la cassette d'expression pSV40-NS5B (plasmide pIV342, figure 1K).

**4 Confirmation de l'expression des antigènes insérés dans les différents adénovirus**

L'expression des antigènes du VHC codés par les adénovirus AdNS3NS4, AdNS5b et AdNS3NS4NS5b a été vérifiée par Western blot après infection de cellules Huh7. Comme attendu, tous les antigènes ont été exprimés.

**Exemple 2: Préparation d'un poxvirus selon l'invention permettant l'expression des protéines NS3/NS4 et NS5b**

**1 Poxvirus MVA**

La souche Modified Virus Ankara MVATG N33 a été fourni par TRANSGENE S.A. (Strasbourg, France).

**2 Préparation du plasmide de transfert permettant l'expression du gène NS3/NS4 sous le contrôle du promoteur ph5r**

2.1 Construction du vecteur pIV250 contenant les bras de recombinaison BRG2 et BRD2 du MVA, ainsi que le gène de sélection GPT sous le contrôle du promoteur ph5r (MVA) suivi d'un deuxième promoteur ph5r pour permettre l'expression du gène d'intérêt

Dans ce point, on souhaite l'insertion du fragment ph5r-GPT-BRG3-ph5r (provenant du plasmide pTG9997, Transgène) dans le plasmide pTG6018 (Transgène) contenant les bras de recombinaison BRG2 et BRD2.

Pour ce faire, on a effectué les étapes suivantes :

- Digestion enzymatique par *BamHI*/*Sac*I (dans React 2 Buffer, Invitrogen) du vecteur pTG6018 (figure 2A)
- Digestion enzymatique par *BamHI,* puis digestion partielle par *Sac*I du plasmide pTG9997 (figure 2B)
- Purification selon le protocole de QIAGEN du fragment de restriction de 1047 pb qui contient la séquence codant pour ph5r-GPT-BRG3-ph5r
- Ligature (T4 DNA Ligase), transformation bactérienne (souche TG1, Statagene)
- Sélection des clones bactériens sur ampicilline (100 $\mu$g/ml)
- Maxi-préparation plasmidique (Qiagen) d'un clone positif après analyse de restriction *(EcoRV* + *Hind*III (dans React 2 Buffer, Invitrogen) : fragments de 246, 439, 476, 826 et 2789 pb ; *Sac*I : fragments de 915 et 3861 pb)
- Obtention du plasmide visé (pIV250, figure 2C).

2.2 Amplification par PCR de la séquence nucléotidique codant pour la polyprotéine NS3/NS4

On a utilisé les oligonucléotides suivants :

oIV225: 5'- GGG GGG CTG CAG ATG GCG CCT ATC ACG GCC TA -3' (SEQ ID N°15)
oIV226: 5'- GGG GGG TCT AGA TTA GCA TGG CGT GGA GCA GT -3' (SEQ ID N°16)

et selon le mode opératoire décrit dans l'exemple 1, point 2.1 ci-dessus, à ceci près qu'on a utilisé une température de 52°C à la place de 62°C

2.3 Insertion du fragment de PCR NS3-NS4 dans le plasmide pIV250

Pour ce faire, on a effectué les étapes suivantes :

- Digestion enzymatique du plasmide pIV250 obtenu dans le point 2.1 ci-dessus par *Pst*I (dans React 2 Buffer, Invitrigen)/*Xba*I
- Digestion enzymatique du fragment PCR NS3/NS4 par *Pst*I/*Xba*I
- Ligature (T4 DNA Ligase), transformation bactérienne (souche TG1)
- Sélection des clones bactériens sur ampicilline (100 $\mu$g/ml)
- Maxi-préparation plasmidique (Qiagen) d'un clone positif après analyse de restriction -(*Hind*III (dans React 2 Buffer, Invitrogen) : fragments de 4763 et 2789 pb ; *Sph*I (dans React 6 Buffer, Invitrogen) : 1534 et 5991 pb ; *Nco*I (dans React 3 Buffer, Invitrogen) : 2764 et 4761 pb)
- Obtention du plasmide de transfert contenant la séquence codant pour la polyprotéine NS3/NS4 sous le contrôle du promoteur ph5r (pIV327, figure 2D).

**3 Préparation du plasmide pIV328 permettant l'expression de la protéine NS5b sous le contrôle du promoteur p7,5**

3.1 Amplification par PCR de la séquence nucléotidique codant pour la protéine NS5b

On a utilisé les oligonucléotides suivants :

oIV227 : 5'- GGG GGG GTC GAC ATG TCA ATG TCC TAC ACA TGG AC -3' (SEQ ID N°17)
oIV228 : 5'- GGG GGG GCA TGC TTA CCG GTT GGG GAG CAG GT -3' (SEQ ID N°18)

et selon le mode opératoire décrit dans l'exemple 1, point 2.1 ci-dessus, à ceci près qu'on a utilisé une température de 52°C à la place de 62°C.

3.2 Obtention du plasmide

On a effectué les étapes suivantes :

- Digestion enzymatique du fragment PCR codant pour NS5b par *Sal*I/*Sph*I
- Digestion enzymatique de pTG186 (figure 2E, Transgène) par *Sal*I/*Sph*I
- Déphosphorylation du vecteur pTG186 (phosphatase alkaline ROCHE)
- Ligature (T4 DNA Ligase), transformation bactérienne (souche TG1)
- Sélection des clones bactériens sur ampicilline (100 μg/ml)
- Maxi-préparation plasmidique (Qiagen) d'un clone positif après analyse de restriction : (*Hind*III : fragments de 1984, 2627 et 4437 pb ; *Bgl*II : fragments de 321, 557, 1361, 1451, 2237 et 3121 pb ; *Kpn*I (dans React 4 Buffer, Invitrogen) : fragments de : 2787 et 6261 pb)
- Obtention du plasmide de transfert contenant la séquence codant pour le polypeptide NS5b sous le contrôle du promoteur p7.5 (pIV328, figure 2F)

**4 Préparation des plasmides de transfert pIV329 et pIV344 permettant l'expression du gène codant pour la polyprotéine NS3/NS4 sous le contrôle du promoteur ph5r et du gène codant pour la protéine NS5b sous le contrôle du promoteur p7.5**

Pour ce faire, on a mis en oeuvre les étapes suivantes :

- Amplification par PCR de la séquence nucléotidique codant pour la protéine NS5b à partir du plasmide pIV328 obtenu dans le point 3.2 ci-dessus en utilisant les oligonucléotides suivants:

  oIV229 : 5'- GGG GGG TCT AGA CCG GTA GTT CGC ATA TAC ATA -3' (SEQ ID N°19)
  oIV218 : 5'- GGG GGG TCT AGA TTA CCG GTT GGG GAG CAG GT-3' (SEQ ID N°14)

  et selon le mode opératoire décrit dans l'exemple 1, point 2.1 ci-dessus, à ceci près qu'on a utilisé une température de 50°C à la place de 62°C
- Digestion enzymatique du fragment de PCR par *Xba*I
- Digestion enzymatique du plasmide pIV327 obtenu dans le point 2.3 ci-dessus par *Xba*I
- Ligature (T4 DNA Ligase), transformation bactérienne (souche TG1)
- Sélection des clones bactériens sur ampicilline (100 μg/ml)
- Maxi préparation plasmidique (Qiagen) de 2 clones positifs après analyse de restriction : (Pst*I* : pIV329 : fragments de 3033 et 6466 pb, pIV344 : 4641 et 4858 pb ; *Apa*I (dans React 4 Buffer, Invitrigen) : pN329 : 454, 960 et 8085 pb, pIV344 : 454,1418 et 7627 pb ; *Nco*I : pIV329: 4269, 469 et 4761 pb , pIV344 : 3053, 1685 et 4761 pb ; *Sma*I : pIV329 : 214, 2164, 1444 et 5677 pb, pIV344 : 214, 2164, 928 et 6193 pb)
- Obtention soit du plasmide de transfert permettant l'expression de la polyprotéine NS3/NS4 sous le contrôle du promoteur ph5r et de la protéine NS5b sous le contrôle du promoteur p75, les 2 cassettes d'expression étant orientées dans le même sens (pIV329, figure 2G), soit du plasmide de transfert permettant l'expression de la polyprotéine NS3/NS4 sous le contrôle du promoteur ph5r et de la protéine NS5b sous le contrôle du promoteur p7.5, les 2 cassettes d'expression étant orientées en sens opposés (pN344, figure 2H).

**5 Confirmation de l'expression des antigènes insérés dans les différents poxvirus**

On a vérifié par Western blot, après infection de cellules Huh7 avec les poxvirus concernés, que les poxvirus pIV329 et pIV344, contenant les séquences codant pour la polyprotéine NS3NS4 et le polypeptide NS5b, exprimaient ces dits antigènes du VHC.

**Exemple 3: Mise en évidence de l'immunogénicité de la combinaison NS3/NS4 et NS5b**

**1 Immunisation des souris**

On a immunisé des souris transgéniques HLA-A2.1, une fois, par injection intramusculaire d'au moins un adénovirus choisi parmi les adénovirus suivants :

- AdNS3NS4 préparé dans l'exemple 1 ci-dessus (point 2.3),
- AdNS5b préparé dans l'exemple 1 ci-dessus (point 3.3),

- AdNS5a préparé selon le mode opératoire de l'exemple 1, point 2, à ceci près qu'on a utilisé les amorces nucléotidiques suivantes pour amplifier la séquence nucléotidique codant pour le polypeptide NS5a (SEQ ID N°5 et 6) :

  oIV172: 5'-GGG GGG GGT ACC ATG TCC GGC TCG TGG CTA AGG-3' (SEQ ID N°20),
  oIV173: 5'-GGG GGG TCT AGA TTA GCA GCA GAC GAT GTC GTC-3' (SEQ ID N°21),

  qu'on a remplacé dans la PCR la température de 62°C par 56°C, que la digestion enzymatique de pTG13387 et du fragment NS5a a été mise en oeuvre par *KpnI/XbaI*, l'analyse de restriction par digestion par *SmaI* de pTG13387 donnant les fragments de 180 et 7251 pb et de pTG6624 donnant les fragments de 2263, 621, 5615, 180, 2463, 6480, 1398, 4456, 1455, 3540, 3386, 230 et 3685 pb
- AdCE1E2 selon le mode opératoire de l'exemple 1, point 2, à ceci près qu'on a utilisé les amorces nucléotidiques suivantes pour amplifier la séquence nucléotidique codant pour la polyprotéine Core-E1-E2 (autrement appelée CE1E2) (SEQ ID N°7 et 8) :

  oIV62: 5'-GGG GGG GCT AGC ATG AGC ACA AAT CCT AAA CCT-3' (SEQ ID N°22)
  oIV68: 5'-GGG GGG TCT AGA TCA GGC CTC AGC CTG GGC TAT-3' (SEQ ID N°23),

  qu'on a remplacé dans la PCR la température de 62°C par 56°C, que la digestion enzymatique de pTG13387 et du fragment CE1E2 a été mise en oeuvre par *NheI/XbaI*, l'analyse de restriction par digestion par *SmaI* de pTG13387 donnant les fragments de 163, 435, 2270, 180 et 5254 pb et de pTG6624 donnant les fragments de 2263, 621, 3618, 163, 435, 2270, 180, 2463, 6480, 1398, 4456, 1455, 3540, 3386, 230 et 3685 pb,
- AdNS3NS4NS5b préparé dans l'exemple 1 ci-dessus (point 3) et
- AdβGal (Transgène),

selon le protocole suivant :

- $10^9$ pfu d'AdNS3NS4 ou
- $10^9$ pfu d'AdNS5b ou
- $10^9$ pfu d'AdCE1E2 ou
- $10^9$ pfu d'AdNS3NS4 et $10^9$ pfu d'AdNS5b ou
- $10^9$ pfu d'AdNS3NS4, $10^9$ pfu d'AdNS5b et $10^9$ pfu d'AdNS5a
- $10^9$ pfu d'AdNS3NS4, $10^9$ pfu d'AdNS5b et $10^9$ pfu d'AdCE1E2
- $10^9$ pfu AdNS3NS4NS5b ou
- $10^9$ pfu d'Adβ-Gal à titre de témoin.

Avant immunisation, on a vérifié, par Western blot, l'expression des antigènes du VHC et de β-Gal par les différents adénovirus utilisés pour l'immunisation.

**2 Tests CTL et ELISPOT**

Quinze jours après l'injection, on a analysé la réponse cellulaire en isolant les cellules de la rate (splénocytes) des souris et on a effectué un test CTL et un test ELISPOT comme suit :

Pour le test CTL, on a cultivé ces splénocytes en plaque 24 puits en présence de :

- 5 μM de l'épitope GLL (GLLGCIITSL, SEQ ID N°24) dans le cas des splénocytes provenant de souris ayant reçu AdNS3NS4, 5 μM de l'épitope ALY (ALYDVVSTL, SEQ ID N°25) ou 5 μM de l'épitope KLQ (KLQDCTMLV, SEQ ID N°26) dans le cas des splénocytes provenant de souris ayant reçu AdNS5b ou de 5 μM de l'épitope DLM (DLMGYIPLV, SEQ ID N°27) dans le cas des splénocytes provenant de souris ayant reçu AdCE1E2, lesdits épitopes étant sous la forme de peptide synthétique (Eurogentex), et
- 10 U d'interleukine 2 recombinante murine (Brinster et al., Hepatology 2001) par ml dans du milieu minimum essentiel alpha (αMEM) pendant 5 jours. Au $5^{ème}$ jour, on a effectué l'étape de restimulation qui consiste à rajouter aux splénocytes en culture des splénocytes de souris naïves en présence desdits épitopes pendant 2 jours. Au $7^{ème}$ jour, on a réalisé le test CTL en lui-même qui consiste à mettre en présence les splénocytes des souris immunisées après les 7 jours de culture (cellules effectrices) et des cellules EL4 S3-Rob HDD chargées avec 10μM desdits épitopes et marquées au $Cr^{51}$ (cellules cibles). On a déterminé l'activité cytotoxique spécifique des cellules effectrices par la mesure, après 4 h d'incubation avec les cellules cibles, du $Cr^{51}$ libéré suite à la lyse des cellules cibles en utilisant un appareil de comptage γ-Cobra II (Packard, Rungis, France). On a déterminé la libération spontanée et maximale à partir de puits contenant soit du milieu seul, soit du tampon de lyse (HCl 1N). On a calculé le pourcentage spécifique de cytotoxicité par la formule :

(libération dans l'essai - libération spontanée)/(libération maximale - libération spontanée) $\times$100. On a déterminé la lyse spécifique d'épitope par la différence entre le pourcentage de lyse spécifique obtenu en présence ou en l'absence desdits épitopes.

On a effectué le test ELISPOT en cultivant les splénocytes pendant 48 h dans des plaques 96 puits Multiscreen (Millipore) préalablement «coatées» avec de l'anticorps anti-interféron gamma (IFN$\gamma$) (10$\mu$g/ml final). On a mis en culture les splénocytes en présence de 10$\mu$M des épitopes appropriés, comme indiqué ci-dessus, et de 10 U d'interleukine 2 recombinante murine par ml dans du $\alpha$MEM. Pour le contrôle positif, on a cultivé les splénocytes en présence de concanavaline A (5 $\mu$g/ml). Pour le contrôle négatif, on a cultivé les splénocytes soit en présence d'un peptide non spécifique appartenant à la protéine de capside du VHC, de séquence DLMGYIPLV (également appelé peptide irrelevant), soit en milieu seul sans épitope. On a lavé les puits à trois reprises, respectivement avec du PBS-Tween 0,05% puis du PBS, opération suivie d'une incubation de 2 h avec des anticorps anti-IFN$\gamma$ de souris biotinylés. Après lavage, on a incubé les puits pendant 1 h avec un conjugué streptavidine-peroxydase de raifort et on a révélé l'activité enzymatique par dégradation du substrat AEC (aminoethylcarbazole). Les spots obtenus ont été comptés grâce à un lecteur ELISpot Zeiss (microscope Zeiss couplé au logiciel KS-ELISpot).

Les résultats sont indiqués sur les figures 3 à 5 sur lesquelles S correspond à souris et Souris neg correspond à la souris témoin.

Ces résultats mettent en évidence que

- l'AdNS3NS4 induit bien une réponse à médiation cellulaire spécifique des antigènes exprimés, comme illustré sur la figure 3A et 3B par la détection de lymphocytes T spécifiques de l'épitope GLL contenu dans NS3.
- l'AdNS5b induit bien une réponse à médiation cellulaire spécifique des antigènes exprimés, comme illustré sur la figure 4 par la détection de lymphocytes T spécifiques de l'épitope ALY et KLQ contenus dans NS5b.
- l'AdCE1E2 induit bien une réponse à médiation cellulaire spécifique des antigènes exprimes, comme illustré sur la figure 5 par la détection de lymphocytes T spécifiques de l'épitope DLM contenus dans la protéine Core.

**3 Test d'épreuve in *vivo* à l'aide d'un virus vaccine recombinant**

Afin d'évaluer si les réponses immunes spécifiques induites par les différents adénovirus étaient capables d'induire une protection contre une épreuve infectieuse (« protection *in vivo*»), nous avons soumis les souris vaccinées à une telle épreuve.

La souris n'étant pas infectable directement par le VHC, nous avons utilisé, pour relier l'induction d'une réponse immunitaire spécifique et la résistance à une infection, un virus vaccine recombinant (souche WR) codant pour les protéines non structurales du VHC (NS2 à NS5b) pour réaliser cette épreuve. Ce virus recombinant de la vaccine, après injection intra-péritonéale de $10^7$ pfu à la souris, va se répliquer chez l'animal. La réplication de ce virus induit une réponse immunitaire à la fois spécifique des antigènes de la vaccine et spécifique des antigènes du VHC, comme il exprime aussi les protéines NS du VHC. Cette réponse spécifique des antigènes du VHC sera d'autant plus efficace et vigoureuse que les souris auront déjà reçu un vaccin exprimant les antigènes du VHC. En d'autres termes, plus la vaccination (dans le cas présent réalisée avec les adénovirus recombinants) aura été efficace (c'est-à-dire que le système immun des souris aura été « primé» effacement par le vaccin), plus la réponse anti-VHC générée après l'épreuve par le virus recombinant de la vaccine sera forte et, par voie de conséquence, plus les souris seront « protégées » contre cette épreuve. En pratique, plus le taux résiduel de virus de la vaccine dans les souris sera faible, plus la protection ou la neutralisation due à la vaccination aura été efficace.

La neutralisation du virus vaccine reflète à la fois la réponse cellulaire induite par les protéines du VHC et par les protéines de la vaccine. La neutralisation est évaluée par titration du virus vaccine résiduel à partir des ovaires des animaux comme suit : les ovaires sont prélevés à 4 jours post-épreuve, soniqués, congelés-décongelés 3 fois puis après centrifugation, des dilutions successives de surnageant sont titrées selon la technique des plages de lyse (Murata et al., PNAS, vol. 100, p.6753-6758) sur cellules Hutk-. Les titres viraux sont déterminés en pfu/ml/mg d'ovaire.

**4 Mise en évidence d'une protection supérieure d'une vaccination combinant la polyprotéine NS3/NS4 et le polypeptide NS5b.**

On a déterminé le titre de virus recombinant de la vaccine pour 4 groupes de 8 souris immunisées par les combinaisons d'adénovirus suivantes: AdNS3NS4 + AdNS5b ($1^{er}$ groupe), AdNS3NS4 + AdNS5b + AdNS5a ($2^{ème}$ groupe), AdNS3NS4 + AdNS5b +AdCE1E2 ($3^{ème}$ groupe) et Ad$\beta$Gal ($4^{ème}$ groupe).

Les résultats, donnés sur la figure 6, sont traités de façon statistique en se basant sur le test non paramétrique de Mann Whitney Wilcoxon (méthodes Statistiques à l'usage des médecins et des biologistes, Collection Statistique en Biologie et en Médecine, Flammarion Medecine Sciences, (D. Schwartz), 1977) qui repose sur une comparaison des moyennes, et permet la comparaison des valeurs de deux échantillons x et y indépendants.

Ce test est mis en oeuvre comme suit : l'ensemble des valeurs des deux groupes x et y à comparer est classé de façon croissante. Un rang est ensuite attribué à chaque valeur, et la somme des rangs est effectuée. On obtient alors Wx et Wy. On calcule alors une valeur de référence appelée $(Wx)_t$ (valeur théorique dans l'hypothèse nulle où Wx n'est pas différent de Wy) et liée par le rapport : n(N+1)/2, avec n = nombre de souris testées dans le groupe x et N = nombre de souris testées dans les groupes x et y.

Si Wx est inférieur à $(Wx)_t$ (taux résiduel de virus de la vaccine dans les souris faible), alors on peut conclure que la neutralisation due à la vaccination est significativement efficace.

Si nous prenons l'exemple du groupe AdNS3NS4S5b noté x comparé au groupe AdβGal noté y, nous obtenons les valeurs suivantes :

$$Wx = 1+2+4+6+8+11+13+14 = 59 \text{ (8 souris testées)}$$

$$Wy = 3+5+7+9+10+12+15+16 = 77 \text{ (8 souris testées)}$$

Sous l'hypothèse nulle, Wx n'est pas différent de Wy, la valeur attendue est : $\{Wx)_t$= (1/2)*8*17 = 68

Wx < $(Wx)_t$ ce qui signifie que les valeurs obtenues dans le groupe AdNS3NS4NS5b sont plus petites que celles obtenues dans le groupe AdβGal et que la neutralisation due à la vaccination est significativement efficace.

Les valeurs statistiques pour les autres groupes de souris sont indiquées dans le tableau 1 ci-dessous :

Tableau 1

| Groupe/AdβGal | Wx | $(Wx)_t$ |
|---|---|---|
| AdNS3NS4+NS5b | 52 | 68 |
| AdNS3NS4+NS5b+ NS5a | 68 | 68 |
| AdNS3NS4+NS5b+ CE1E2 | 74 | 68 |

Les valeurs dans le tableau 1 ci-dessus montrent que seule une vaccination des souris par la combinaison des Adénovirus NS3NS4 et adénovirus NS5b est capable d'induire une neutralisation significative de la réplication du virus de la vaccine utilisé dans l'épreuve par rapport au groupe de souris contrôle vacciné par l'AdβGal. Les vaccination réalisées en utilisant les combinaisons comprenant (AdNS3NS4 + AdNS5b + AdNS5a) ou (AdNS3/NS4 + AdNS5b + AdCE1E2), n'aboutissent pas à une différence significative par rapport au groupe de souris contrôle immunisé par AdβGal.

Ces résultats permettent donc de mettre en évidence, de façon inattendue, la protection supérieure d'une vaccination combinant la polyprotéine NS3NS4 et le polypeptide NS5b.

**5 Confirmation de la protection d'une vaccination combinant la polyprotéine NS3/NS4 et le polypeptide NS5b exprimés conjointement par un même vecteur**

On a déterminé le titre de virus recombinant de la vaccine pour 3 groupes de 8 souris immunisées par les combinaisons d'adénovirus suivantes : AdNS3NS4NS5b (1er groupe), AdNS3NS4 + AdNS5b (2ème groupe) et AdβGal (3ème groupe).

Les résultats, donnés sur la figure 7, sont traités de façon statistique en se basant sur le test non paramétrique de Mann Whitney Wilcoxon comme décrit dans l'expérience précédente.

Les valeurs statistiques pour les groupes 1 et 2 comparées au groupe contrôle AdβGal sont indiquées dans le tableau 2ci-dessous :

Tableau 2

| Groupe/AdβGal | Wx | $(Wx)_t$ |
|---|---|---|
| AdNS3NS4NS5b | 49 | 68 |

(suite)

| Groupe/AdβGal | Wx | $(Wx)_t$ |
|---|---|---|
| AdNS3NS4+NS5b | 53 | 68 |

Les valeurs dans le tableau 2 ci-dessus montrent que la vaccination des souris par un adénovirus codant à la fois pour les trois antigènes NS3, NS4 et NS5b, tout comme la combinaison des Adénovirus NS3NS4 et Adénovirus NS5b, est capable d'induire une neutralisation significative de la réplication du virus de la vaccine utilisé dans l'épreuve par rapport au groupe de souris contrôle vacciné par l'AdénoβGal. Ce résultat confirme la protection d'une vaccination combinant la polyprotéine NS3/NS4 et le polypeptide NS5b exprimés conjointement par un même vecteur.

## Revendications

1. Composition peptidique **caractérisée en ce qu'**elle consiste en une polyprotéine NS3/NS4 du virus de l'hépatite C, ainsi qu'un polypeptide NS5b du virus de l'hépatite C.

2. Composition peptidique selon la revendication 1, **caractérisée en ce que** NS3 et/ou NS4 et/ou NS5b proviennent de virus de génotypes différents.

**3.** Composition peptidique selon la revendication 1, **caractérisée en ce que** NS3, NS4 et NS5b proviennent d'un virus de même génotype, de préférence de génotype 1 b.

**4.** Vecteur d'expression pour l'expression de séquences nucléotidiques du virus de l'hépatite C **caractérisé en ce que** lesdites séquences nucléotidiques du virus de l'hépatite C sont constituées par une séquence nucléotidique codant pour la polyprotéine NS3/NS4 et une séquence nucléotidique codant pour le polypeptide NS5b, ainsi que les moyens nécessaires à leur expression, ladite séquence nucléotidique codant pour la polyprotéine NS3/NS4 et ladite séquence nucléotidique codant pour le polypeptide NS5b étant dans un seul vecteur d'expression.

**5.** Vecteur d'expression selon la revendication 4, **caractérisé en ce que** les séquences nucléotidiques codant pour ladite polyprotéine NS3/NS4 et ledit polypeptide NS5b sont issues de virus de génotypes différents.

**6.** Vecteur d'expression selon la revendication 4, **caractérisé en ce que** les séquences nucléotidiques codant pour ladite polyprotéine NS3/NS4 et ledit polypeptide NS5b sont issues d'un virus de même génotype, de préférence le génotype 1b.

**7.** Vecteur d'expression selon l'une quelconque des revendications 4 à 6, **caractérisé en ce que** ce vecteur est un plasmide, un vecteur viral type adénovirus, poxvirus, baculovirus, ou un vecteur bactérien du type salmonelle, BCG.

**8.** Vecteur d'expression selon la revendication 7, **caractérisé en ce que** ce vecteur est un adénovirus.

**9.** Vecteur d'expression selon la revendication 8, **caractérisé en ce que** ledit vecteur est un adénovirus humain, de préférence l'adénovirus 5.

**10.** Vecteur d'expression selon la revendication 8 ou 9, **caractérisé en ce que** le génome de l'adénovirus est modifié de façon à remplacer la région E1 par la cassette d'expression CMV-NS3-NS4 et à remplacer la région E3 par la cassette d'expression SV40-NS5b.

**11.** Vecteur d'expression selon la revendication 7, **caractérisé en ce que** ce vecteur est un poxvirus.

**12.** Vecteur d'expression selon la revendication 11, **caractérisé en ce que** ledit poxvirus est un virus de la vaccine de la souche Copenhagen ou un virus de la vaccine modifié d'Ankara.

**13.** Vecteur d'expression selon la revendication 12, **caractérisé en ce qu'**il présente au moins l'une des caractéristiques suivantes, prises seules ou en association :

i) le poxvirus est un virus MVA
ii) le poxvirus est sous forme morphologique IMV
iii) le génome du poxvirus est modifié de façon à insérer la cassette d'expression NS3/NS4 et à insérer la cassette d'expression NS5b.

**14.** Vecteur d'expression selon l'une des revendications 11 à 13, **caractérisé en ce que** le génome du poxvirus est modifié de façon à insérer la cassette d'expression ph5r-NS3-NS4 et à insérer la cassette d'expression p7.5-NS5b.

**15.** Microorganisme ou cellule hôte transformé par un vecteur d'expression tel que défini dans l'une quelconque des revendications 4 à 14.

**16.** Utilisation

- d'une composition peptidique telle que définie dans l'une quelconque des revendications 1 à 3, ou bien
- d'un vecteur d'expression tel que défini dans l'une quelconque des revendications 4 à 14, ou bien
- d'un vecteur d'expression d'une séquence nucléotidique codant pour la polyprotéine NS3/NS4 du VHC avec un vecteur d'expression d'une séquence nucléotidique codant pour le polypeptide NS5b du VHC, ou bien
- des séquences nucléotidiques codant pour ladite polyprotéine NS3/NS4 et ledit polypeptide NS5b,

lesdites séquences nucléotidiques correspondant aux séquences contenues dans les vecteurs d'expression tels que définis dans l'une quelconque des revendication 4 à 14, placées sous le contrôle d'éléments nécessaires à une expression constitutive et/ou inductible desdits peptides,

pour la préparation d'un médicament destiné à l'inhibition, la prévention ou le contrôle d'une infection provoquée par le virus de l'hépatite C chez un animal.

**17.** Utilisation selon la revendication 16, **caractérisée en ce que** le médicament est destiné à l'inhibition, la prévention ou le contrôle d'une infection provoquée par le virus de l'hépatite C chez l'homme.

**18.** Composition pharmaceutique, comprenant à titre de substance active la composition peptidique telle que définie dans les revendications 1 à 3, ou bien un vecteur d'expression tel que défini dans l'une quelconque des revendications 4 à 14, ou bien un vecteur d'expression d'une séquence nucléotidique codant pour la polyprotéine NS3/NS4 du VHC avec un vecteur d'expression d'une séquence nucléotidique codant pour le polypeptide NS5b du VHC, ainsi que les moyens nécessaires à leur expression.

**19.** Composition pharmaceutique selon la revendication 18, **caractérisée en ce qu'**elle comprend également un véhicule pharmaceutiquement approprié.

**20.** Composition pharmaceutique selon la revendication 18 ou 19 **caractérisée en ce qu'**elle est appropriée pour une administration sous-cutanée, intramusculaire, intraveineuse, topique ou transdermique.

**21.** Kit pharmaceutique, **caractérisé en ce qu'**il comprend au moins un vecteur d'expression d'une séquence nucléotidique codant pour la polyprotéine NS3/NS4 du VHC et au moins un vecteur d'expression d'une séquence nucléotidique codant pour le polypeptide NS5b du VHC, ainsi que les moyens nécessaires à leur expression.

**22.** Kit pharmaceutique, **caractérisé en ce qu'**il comprend un vecteur d'expression tel que défini dans l'une des revendications 7 à 10 et un vecteur d'expression tel que défini dans l'une des revendications 11 à 14.

**23.** Kit pharmaceutique, comprenant un vecteur d'expression tel que défini dans l'une quelconque des revendications 4 à 14, ou bien un vecteur d'expression d'une séquence nucléotidique codant pour la polyprotéine NS3/NS4 du VHC avec un vecteur d'expression d'une séquence nucléotidique codant pour le polypeptide NS5b du VHC, ainsi que les moyens nécessaires à leur expression, et

a. au moins une composition peptidique telle que définie dans les revendications 1 à 3 ou
b. au moins une séquence nucléotidique codant pour la polyprotéine NS3/NS4 du VHC et pour le polypeptide NS5b du VHC.

**24.** Composition pharmaceutique selon l'un quelconque des revendications 18-20 ou kit pharmaceutique selon l'une quelconque des revendications 21-23, **caractérisé en ce qu'**il s'agit d'un vaccin.

**Claims**

**1.** Peptide composition **characterised in that** it consists of a hepatitis C virus NS3/NS4 polyprotein, and a hepatitis C virus NS5b polypeptide.

**2.** Peptide composition according to claim 1, **characterised in that** NS3 and/or NS4 and/or NS5b are obtained from viruses having different genotypes.

**3.** Peptide composition according to claim 1, **characterised in that** NS3, NS4 and NS5b are obtained from a virus having the same genotype, preferably genotype 1 b.

**4.** Expression vector for expressing hepatitis C virus nucleotide sequences **characterised in that** said hepatitis C virus nucleotide sequences consist of a nucleotide sequence coding for NS3/NS4 polyprotein and a nucleotide sequence coding for NS5b polypeptide, and the means required for the expression thereof, said nucleotide sequence coding for NS3/NS4 polyprotein and said nucleotide sequence coding for NS5b polypeptide being in a single expression vector.

**5.** Expression vector according to claim 4, **characterised in that** the nucleotide sequences coding for said NS3/NS4 polyprotein and said NS5b polypeptide are obtained from viruses having different genotypes.

**6.** Expression vector according to claim 4, **characterised in that** the nucleotide sequences coding for said NS3/NS4 polyprotein and said NS5b polypeptide are obtained from a virus having the same genotype, preferably genotype 1b.

**7.** Expression vector according to any of claims 4 to 6, **characterised in that** said vector is a plasmid, an adenovirus, poxvirus, baculovirus type viral vector, or a salmonella, BCG type bacterial vector.

**8.** Expression vector according to claim 7, **characterised in that** this vector is an adenovirus.

**9.** Expression vector according to claim 8, **characterised in that** said vector is a human adenovirus, preferably adenovirus 5.

**10.** Expression vector according to claim 8 or 9, **characterised in that** the adenovirus genome is modified to replace region E1 by the expression cassette CMV-NS3-NS4 and to replace region E3 by the expression cassette SV40-NS5b.

**11.** Expression vector according to claim 7, **characterised in that** this vector is a poxvirus.

**12.** Expression vector according to claim 11, **characterised in that** said poxvirus is a Copenhagen strain vaccinia virus or a modified vaccinia Ankara virus.

**13.** Expression vector according to claim 12, **characterised in that** it has at least one of the following features, alone or in association:

i) the poxvirus is an MVA virus
ii) the poxvirus is in IMV morphological form
iii) the poxvirus genome is modified so as to insert the NS3/NS4 expression cassette and insert the NS5b expression cassette.

**14.** Expression vector according to any of claims 11 to 13, **characterised in that** the poxvirus genome is modified so as to insert the expression cassette ph5r-NS3-NS4 and insert the expression cassette p7.5-NS5b.

**15.** Micro-organism or host cell transformed by an expression vector as defined in any of claims 4 to 14.

**16.** Use

- of a peptide composition as defined in any of claims 1 to 3, or
- of an expression vector as defined in any of claims 4 to 14, or
- of an expression vector of a nucleotide sequence coding for HCV NS3/NS4 polyprotein with an expression vector of a nucleotide sequence coding for HCV NS5b polypeptide, or
- of the nucleotide sequences coding for said NS3/NS4 polyprotein and said NS5b polypeptide,

said nucleotide sequences corresponding to the sequences contained in the expression vectors as defined in any of claims 4 to 14, placed under the control of sequences required for constitutive and/or inducible expression of said peptides,
for the preparation of a medicinal product intended to inhibit, prevent or control an infection caused by hepatitis C virus in an animal.

**17.** Use according to claim 16, **characterised in that** the medicinal product is intended to inhibit, prevent or control an infection caused by hepatitis C virus in humans.

**18.** Pharmaceutical composition, comprising as active substance the peptide composition as defined in claims 1 to 3, or an expression vector as defined in any of claims 4 to 14, or an expression vector of a nucleotide sequence coding for HCV NS3/NS4 polyprotein with an expression vector of a nucleotide sequence coding for HCV NS5b polypeptide, and the means required for the expression thereof.

**19.** Pharmaceutical composition according to claim 18, **characterised in that** it also comprises a suitable pharmaceutical vehicle.

20. Pharmaceutical composition according to claim 18 or 19, **characterised in that** it is suitable for subcutaneous, intramuscular, intravenous, topical or transdermal administration.

21. Pharmaceutical kit, **characterised in that** it comprises at least one expression vector of a nucleotide sequence coding for HCV NS3/NS4 polyprotein and at least one expression vector of a nucleotide sequence coding for HCV NS5b polypeptide, and the means required for the expression thereof.

22. Pharmaceutical kit, **characterised in that** it comprises an expression vector as defined in any of claims 7 to 10 and an expression vector as defined in any of claims 11 to 14.

23. Pharmaceutical kit, comprising an expression vector as defined in any of claims 4 to 14, or an expression vector of a nucleotide sequence coding for HCV NS3/NS4 polyprotein with an expression vector of a nucleotide sequence coding for HCV NS5b polypeptide, and the means required for the expression thereof, and

    (i) at least one peptide composition as defined in claims 1 to 3 or
    (ii) at least one nucleotide sequence coding for HCV NS3/NS4 polyprotein and for HCV NS5b polypeptide.

24. Pharmaceutical composition according to any of claims 18 to 20, or pharmaceutical kit according to any of claims 21-23, **characterised in that** it is a vaccine.

**Patentansprüche**

1. Peptid-Zusammensetzung, **dadurch gekennzeichnet, dass** sie aus einem Polyprotein NS3/NS4 des Hepatitis C-Virus sowie einem Polypeptid NS5b des Hepatitis C-Virus besteht.

2. Peptid-Zusammensetzung nach Anspruch 1, **dadurch gekennzeichnet, dass** NS3 und/oder NS4 und/oder NS5b von Viren verschiedener Genotypen stammen.

3. Peptid-Zusammensetzung nach Anspruch 1, **dadurch gekennzeichnet, dass** NS3 und/oder NS4 und/oder NS5b von einem Virus desselben Genotyps, vorzugsweise des Genotyps 1 b, stammen.

4. Expressionsvektor für die Expression von Nucleotidsequenzen des Hepatitis C-Virus, **dadurch gekennzeichnet, dass** die Nucleotidsequenzen des Hepatitis C-Virus aus einer Nucleotidsequenz, die für das Polyprotein NS3/NS4 codiert, und einer Nucleotidsequenz, die für das Polypeptid NS5b codiert, sowie den erforderlichen Mitteln für deren Expression zusammengestellt sind, wobei die Nucleotidsequenz, die für das Polyprotein NS3/NS4 codiert, und die Nucleotidsequenz, die für das Polypeptid NS5b codiert, sich in einem einzigen Expressionsvektor befinden.

5. Expressionsvektor nach Anspruch 4, **dadurch gekennzeichnet, dass** die Nucleotidsequenzen, die für das Polyprotein NS3/NS4 und für das Polypeptid NS5b codieren, aus Viren verschiedener Genotypen hervorgegangen sind.

6. Expressionsvektor nach Anspruch 4, **dadurch gekennzeichnet, dass** die Nucleotidsequenzen, die für das Polyprotein NS3/NS4 und für das Polypeptid NS5b codieren, aus einem Virus desselben Genotyps, vorzugsweise des Genotyps 1 b, hervorgegangen sind.

7. Expressionsvektor nach irgendeinem der Ansprüche 4 bis 6, **dadurch gekennzeichnet, dass** der Vektor ein Plasmid, ein viraler Vektor vom Typ Adenovirus, Poxvirus, Bakulovirus oder ein bakterieller Vektor vom Typ Salmonella oder BCG ist.

8. Expressionsvektor nach Anspruch 7, **dadurch gekennzeichnet, dass** der Vektor ein Adenovirus ist.

9. Expressionsvektor nach Anspruch 8, **dadurch gekennzeichnet, dass** der Vektor ein humanes Adenovirus, vorzugsweise das Adenovirus 5, ist.

10. Expressionsvektor nach Anspruch 8 oder 9, **dadurch gekennzeichnet, dass** das Genom des Adenovirus so modifiziert ist, dass die Region E1 durch die Expressionskassette CMV-NS3-NS4 ersetzt ist und die Region E3 durch die Expressionskassette SV40-NS5b ersetzt ist.

**11.** Expressionsvektor nach Anspruch 7, **dadurch gekennzeichnet, dass** der Vektor ein Poxvirus ist.

**12.** Expressionsvektor nach Anspruch 11, **dadurch gekennzeichnet, dass** das Poxvirus ein Virus der Vakzine des Stamms Kopenhagen oder ein Virus der modifizierten Vakzine von Ankara ist.

**13.** Expressionsvektor nach Anspruch 12, **dadurch gekennzeichnet, dass** er mindestens eine der folgenden Eigenschaften, allein oder zusammen, aufweist:

i) das Poxvirus ist ein MVA-Virus
ii) das Poxvirus liegt in der morphologischen Form IMV vor
iii) das Genom des Poxvirus ist so modifiziert, dass die Expressionskassette NS3/NS4 inseriert ist und dass die Expressionskassette NS5b inseriert ist.

**14.** Expressionsvektor nach einem der Ansprüche 11 bis 13, **dadurch gekennzeichnet, dass** das Genom des Poxvirus so modifiziert ist, dass die Expressionskassette ph5r-NS3-NS4 inseriert ist und die Expressionskassette p7.5-NS5b inseriert ist.

**15.** Mikroorganismus oder Wirtszelle, transformiert durch einen Expressionsvektor wie in irgendeinem der Ansprüche 4 bis 14 definiert.

**16.** Verwendung

- einer Peptid-Zusammensetzung wie in irgendeinem der Ansprüche 1 bis 3 definiert, oder
- eines Expressionsvektors wie in irgendeinem der Ansprüche 4 bis 14 definiert, oder
- eines Expressionsvektors einer Nucleotidsequenz, die für das Polyprotein NS3/NS4 des HCV codiert, mit einem Expressionsvektor einer Nucleotidsequenz, die für das Polypeptid NS5b des HCV codiert, oder
- von Nucleotidsequenzen, die für das Polyprotein NS3/NS4 und das Polypeptid NS5b codieren,

wobei die Nucleotidsequenzen den Sequenzen entsprechen, die in den Expressionsvektoren enthalten sind, wie sie in irgendeinem der Ansprüche 4 bis 14 definiert, und unter die Kontrolle von Elementen gestellt sind, die für eine konstitutive und/oder induzierbare Expression der Peptide erforderlich sind,
für die Herstellung eines Medikaments, das zur Hemmung, Verhütung oder Bekämpfung einer Infektion bestimmt ist, die durch das Hepatitis C-Virus bei einem Lebewesen hervorgerufen wird.

**17.** Verwendung nach Anspruch 16, **dadurch gekennzeichnet, dass** das Medikament zur Hemmung, Verhütung oder Bekämpfung einer Infektion bestimmt ist, die durch das Hepatitis C-Virus beim Menschen hervorgerufen wird.

**18.** Pharmazeutische Zusammensetzung, umfassend als Wirkstoff die Peptid-Zusammensetzung, wie in irgendeinem der Ansprüche 1 bis 3 definiert, oder einen Expressionsvektor, wie in irgendeinem der Ansprüche 4 bis 14 definiert, oder einen Expressionsvektor einer Nucleotidsequenz, die für das Polyprotein NS3/NS4 des HCV codiert, mit einem Expressionsvektor einer Nucleotidsequenz, die für das Polypeptid NS5b des HCV codiert, sowie die für deren Expression erforderlichen Mittel.

**19.** Pharmazeutische Zusammensetzung nach Anspruch 18, **dadurch gekennzeichnet, dass** sie auch ein geeignetes pharmazeutisches Vehikel umfasst.

**20.** Pharmazeutische Zusammensetzung nach Anspruch 18 oder 19, **dadurch gekennzeichnet, dass** sie für eine subkutane, intramuskuläre, intravenöse, topische oder transdermale Verabreichung geeignet ist.

**21.** Pharmazeutisches Kit, **dadurch gekennzeichnet, dass** es mindestens einen Expressionsvektor einer Nucleotidsequenz, die für das Polyprotein NS3/NS4 des HCV codiert, und mindestens einen Expressionsvektor einer Nucleotidsequenz, die für das Polypeptid NS5b des HCV codiert, sowie die Mittel, die für deren Expression erforderlich sind, umfasst.

**22.** Pharmazeutisches Kit, **dadurch gekennzeichnet, dass** es einen Expressionsvektor, wie in einem der Ansprüche 7 bis 10 definiert, und einen Expressionsvektor, wie in einem der Ansprüche 11 bis 14 definiert, umfasst.

**23.** Pharmazeutisches Kit, umfassend einen Expressionsvektor, wie in einem der Ansprüche 4 bis 14 definiert, oder einen Expressionsvektor einer Nucleotidsequenz, die für das Polyprotein NS3/NS4 des HCV codiert, mit einem Expressionsvektor einer Nucleotidsequenz, die für das Polypeptid NS5b des HCV codiert, sowie die Mittel, die für deren Expression erforderlich sind, und

a. mindestens eine Peptid-Zusammensetzung, wie in den Ansprüchen 1 bis 3 definiert, oder
b. mindestens eine Nucleotidsequenz, die für das Polyprotein NS3/NS4 des HCV und für das Polypeptid NS5b des HCV codiert.

**24.** Pharmazeutische Zusammensetzung nach irgendeinem der Ansprüche 18 - 20 oder pharmazeutisches Kit nach irgendeinem der Ansprüche 21 - 23, **dadurch gekennzeichnet, dass** es sich um eine Vakzine handelt.

Figure 1

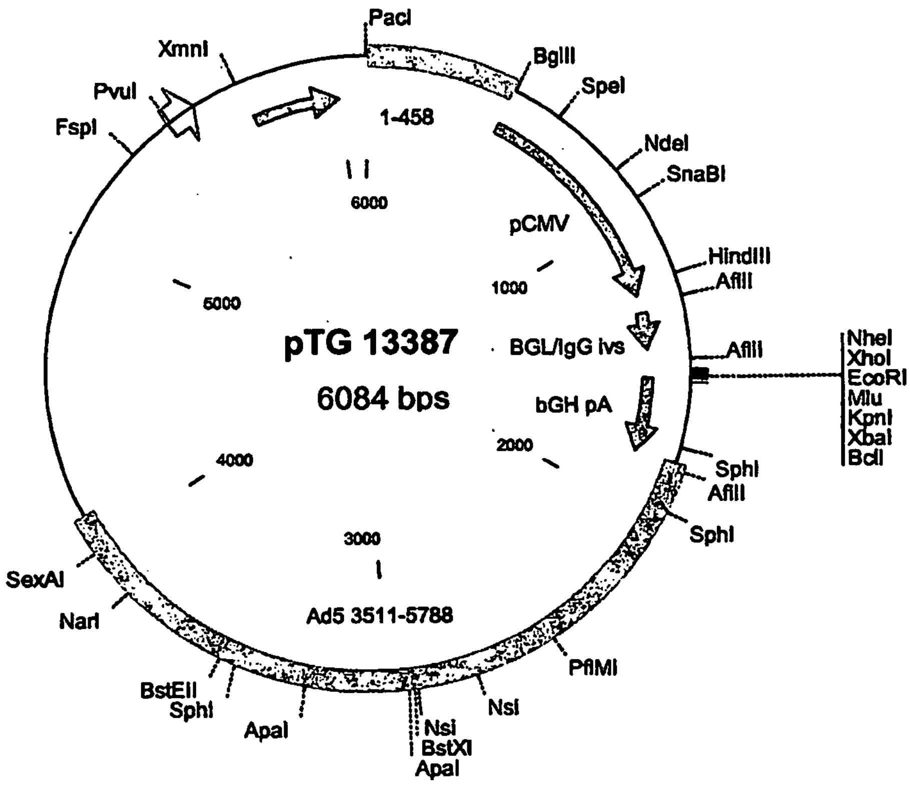


Figure 1A

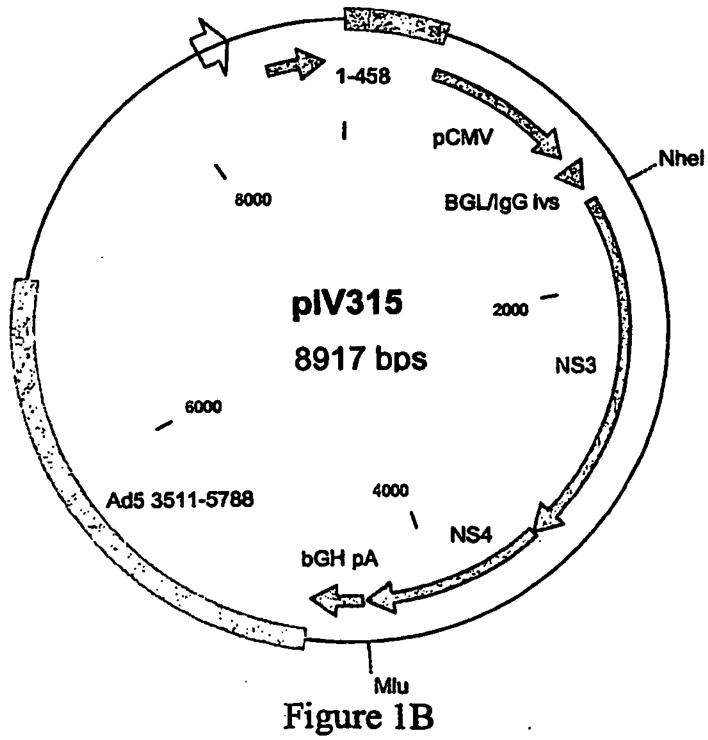


Figure 1B

Figure 1 (suite)

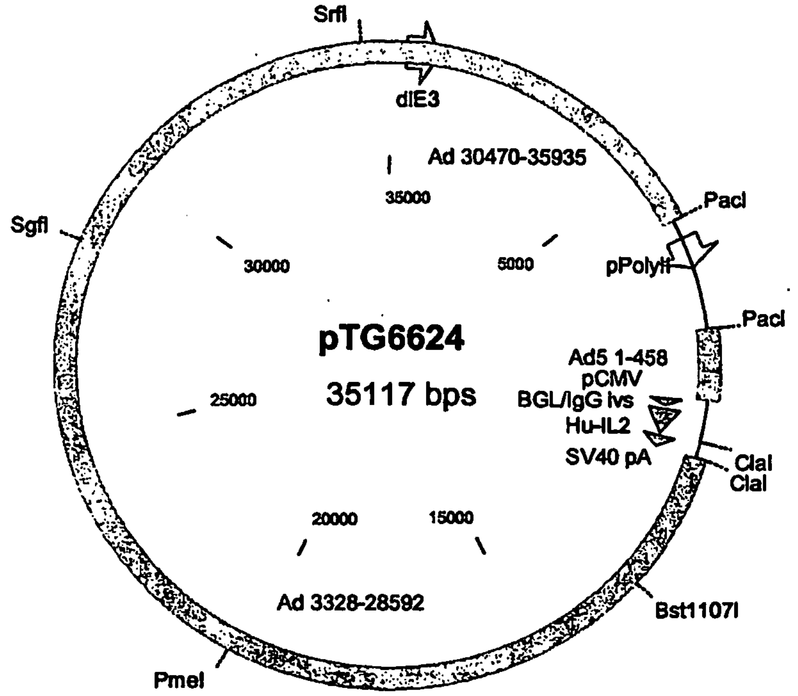


Figure 1C

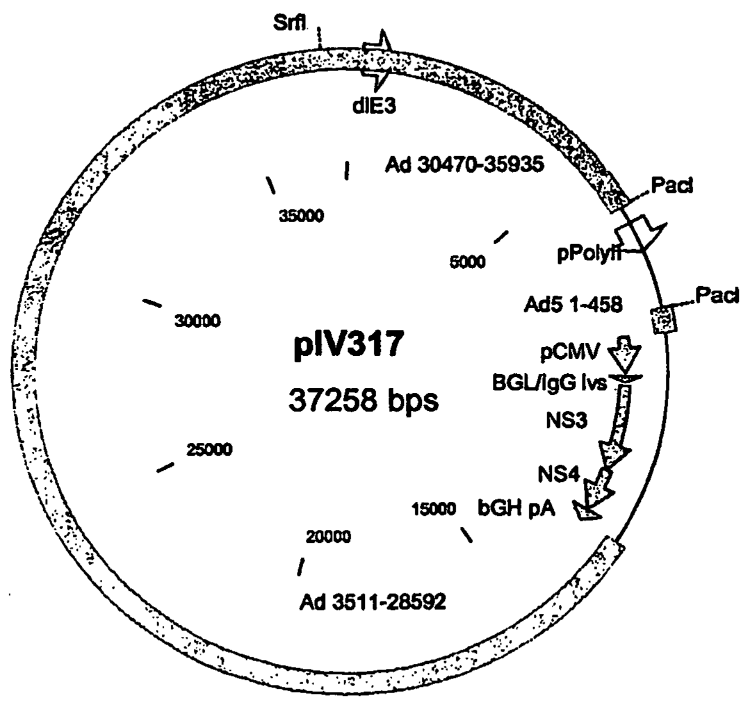


Figure 1D

Figure 1 (suite)

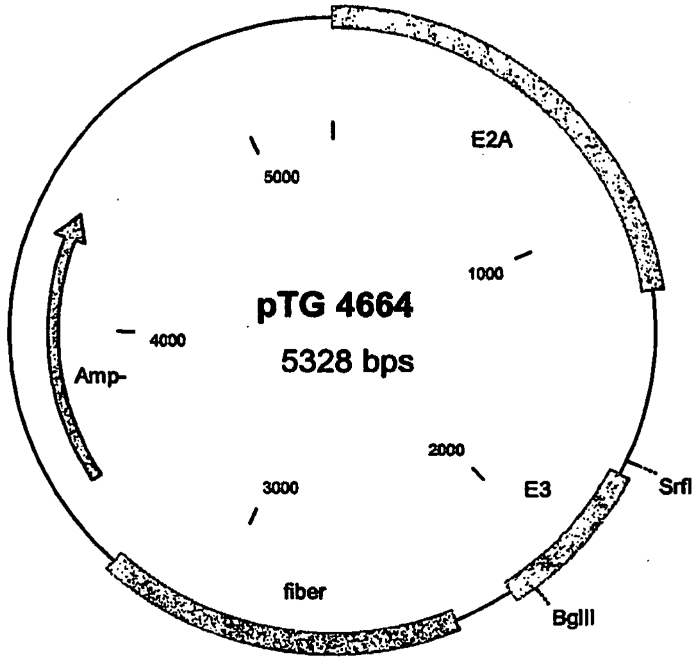


Figure 1E

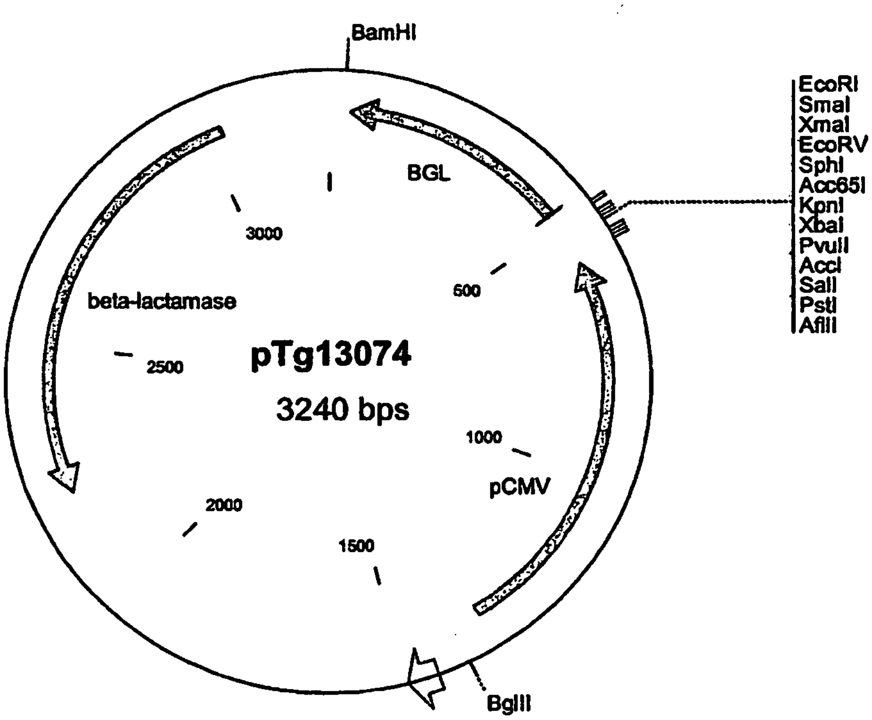


Figure 1F

Figure 1 (suite)

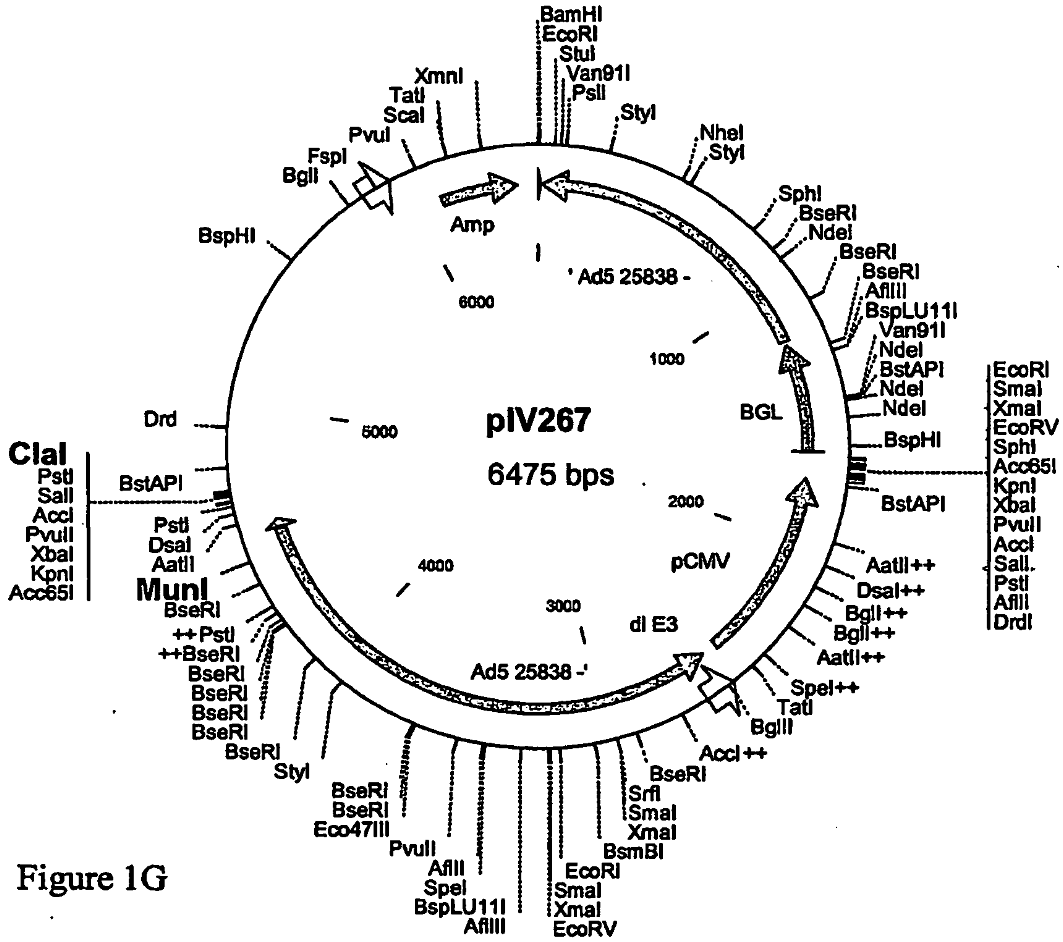


Figure 1G

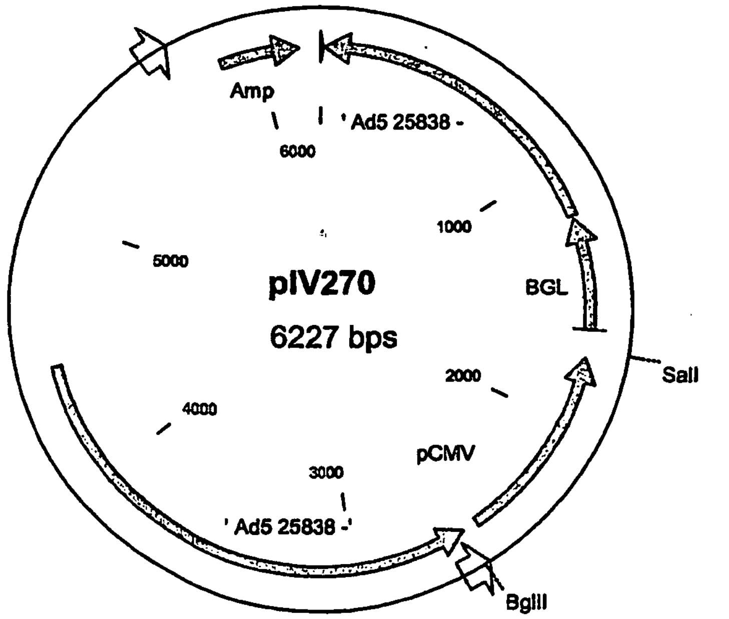


Figure 1H

Figure 1 (suite)

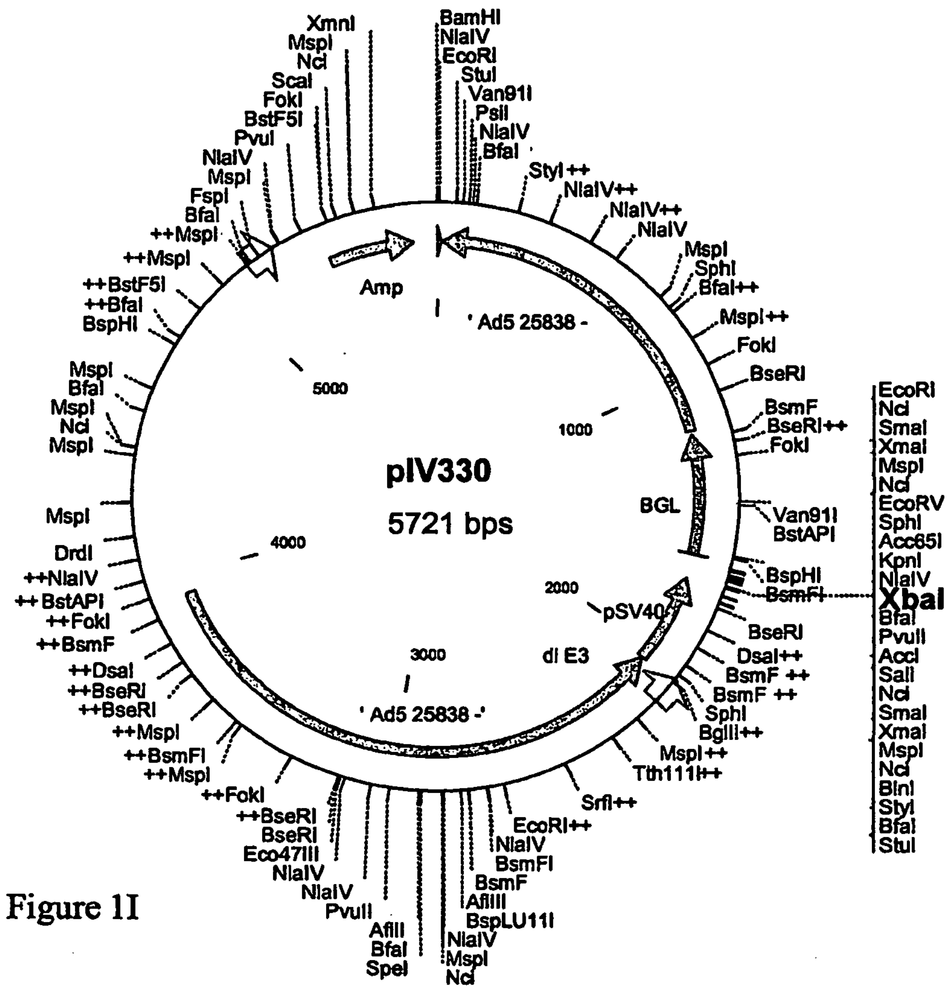


Figure 1I

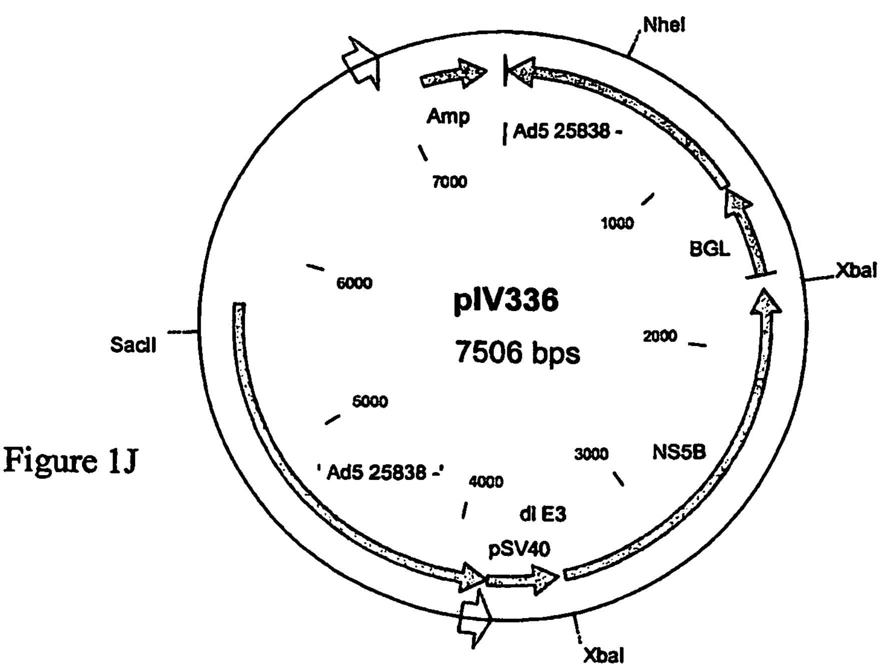


Figure 1J

Figure 1 (suite)

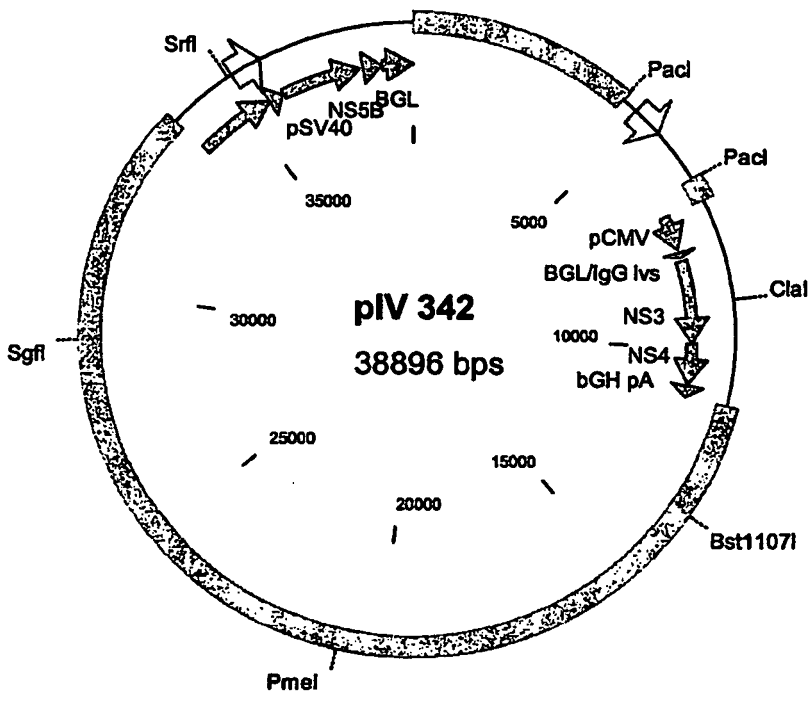


Figure 1K

Figure 2

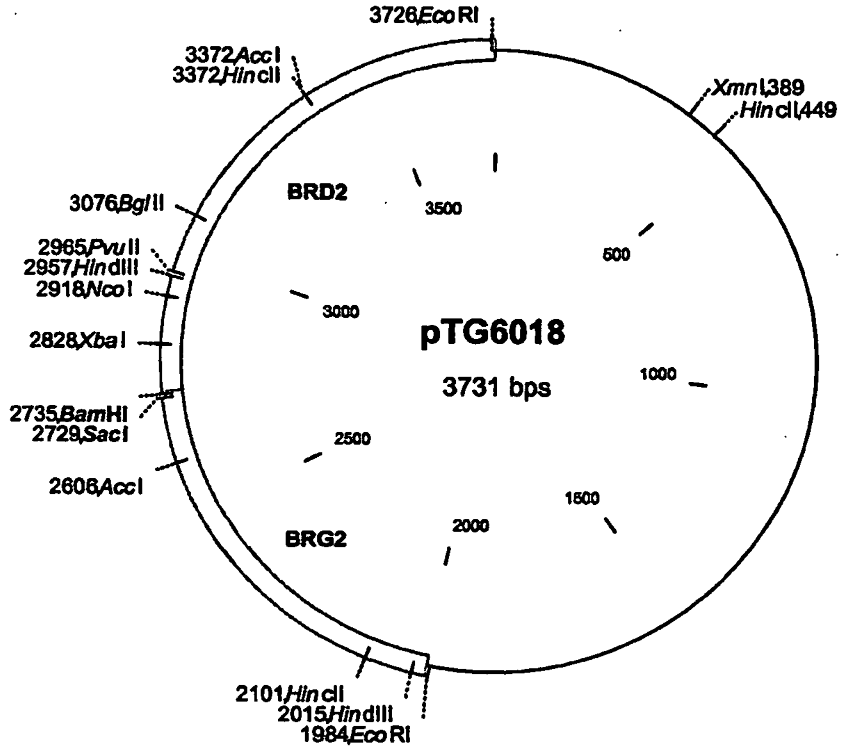


Figure 2A

Figure 2 (suite)

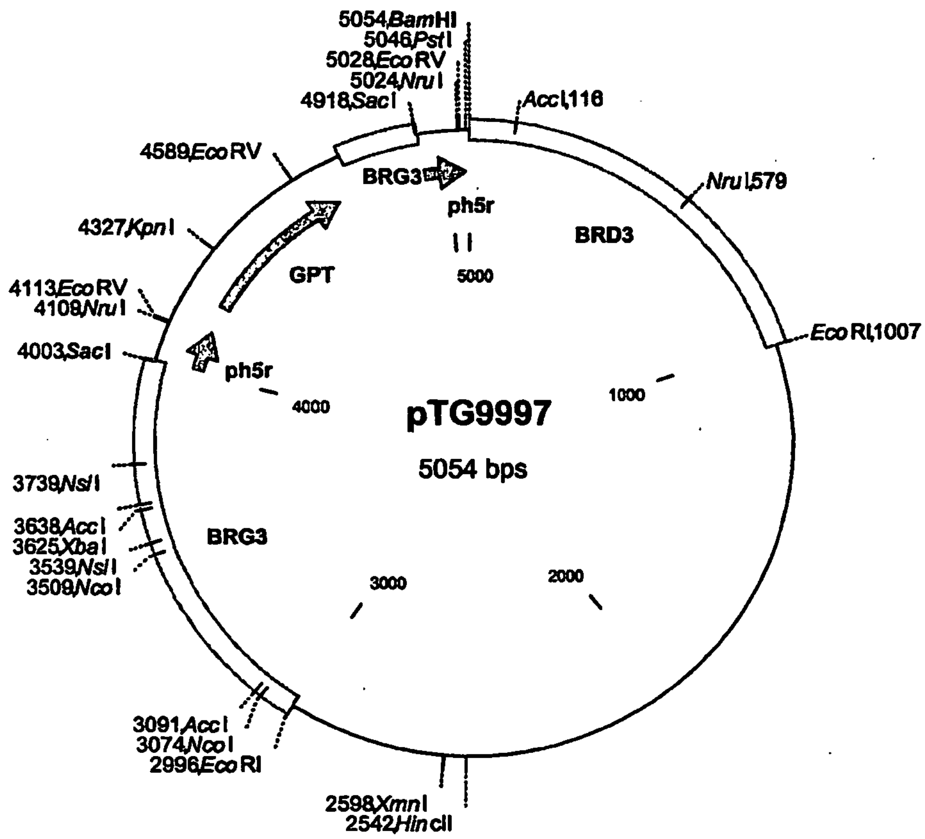


Figure 2B

Figure 2 (suite)

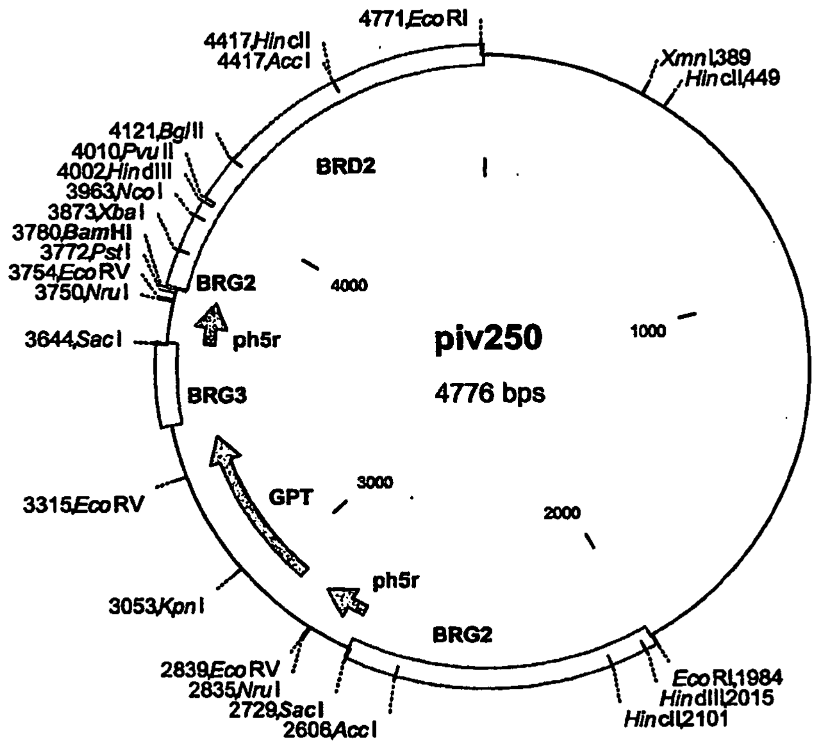


Figure 2C

Figure 2 (suite)

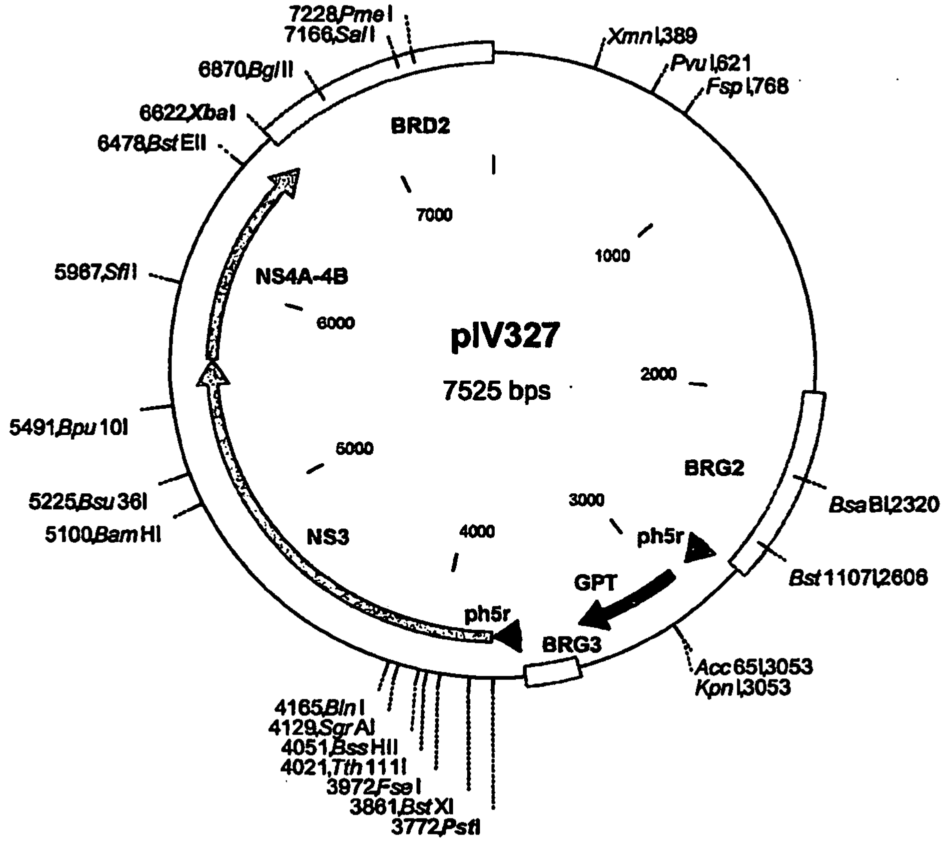


Figure 2D

Figure 2 (suite)

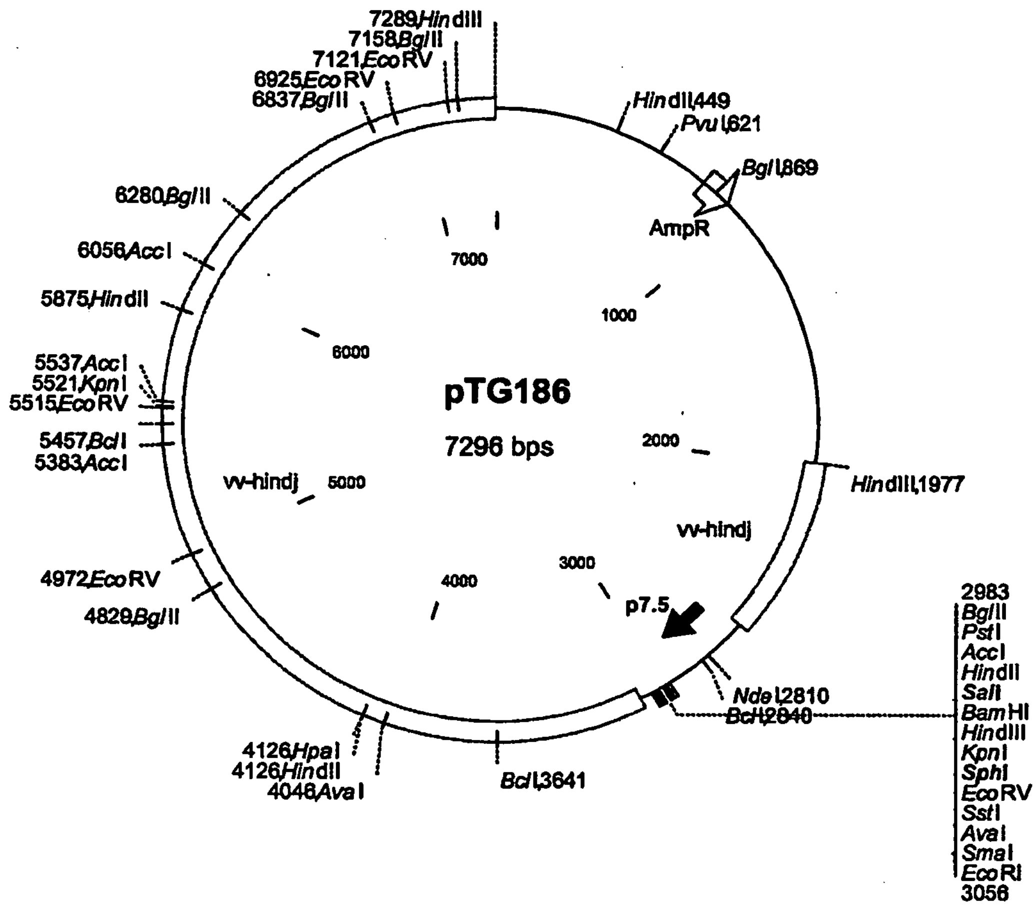


Figure 2E

Figure 2 (suite)

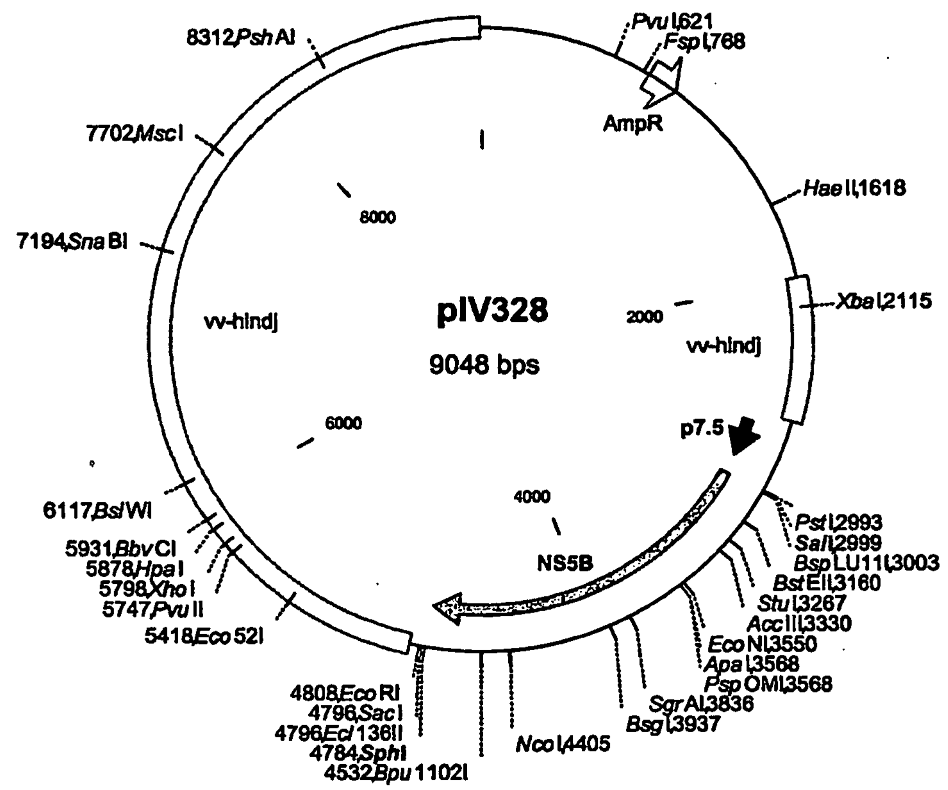


Figure 2F

Figure 2 (suite)

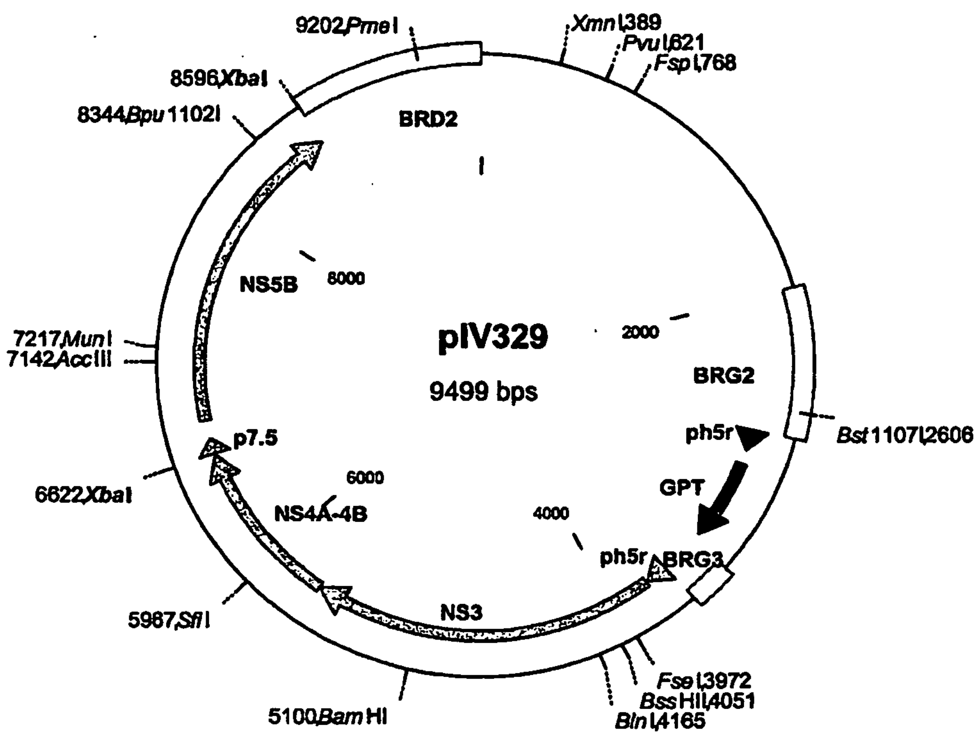


Figure 2G

Figure 2 (suite)

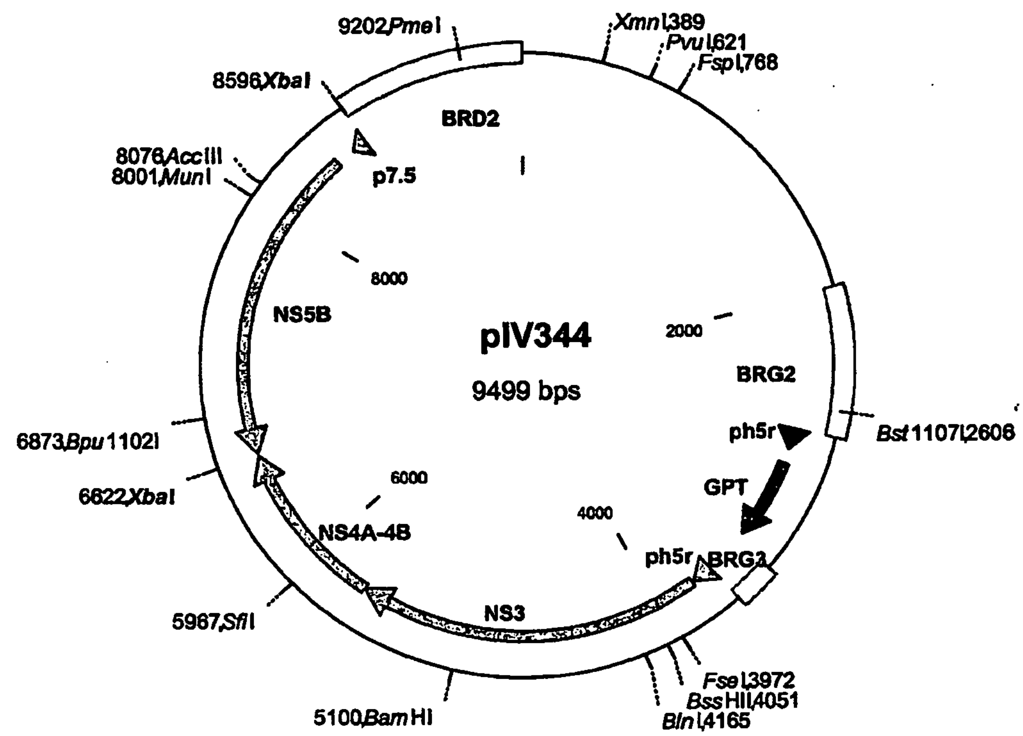


Figure 2H

# Figure 3

## Figure 3A

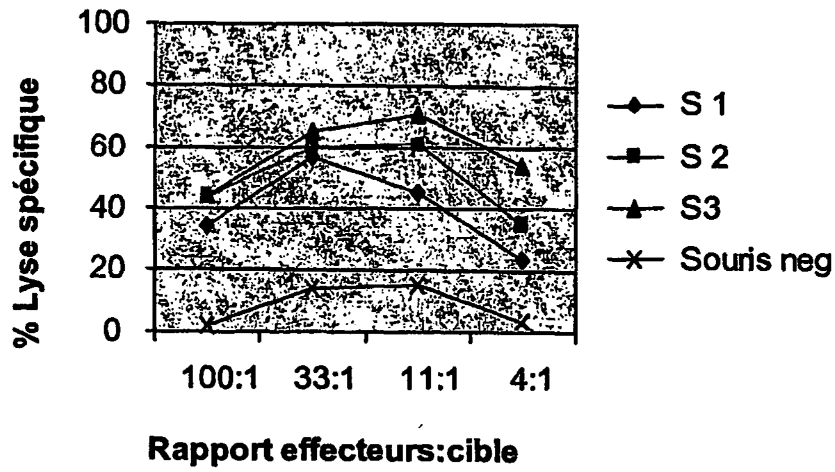


## Figure 3B

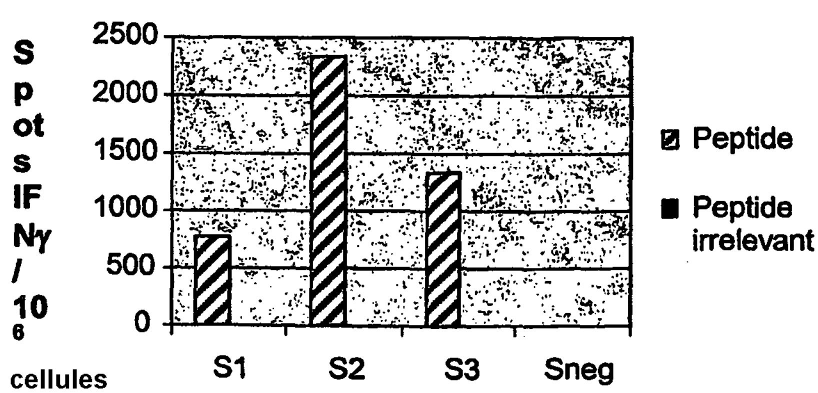

Figure 4

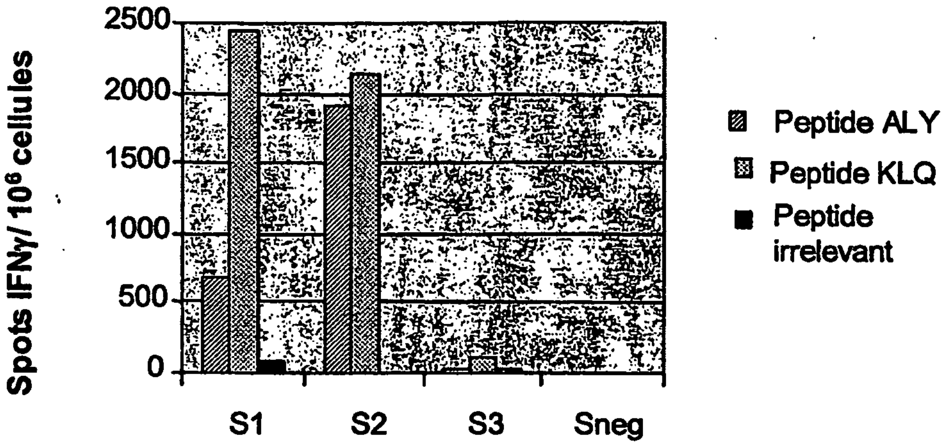


Figure 5

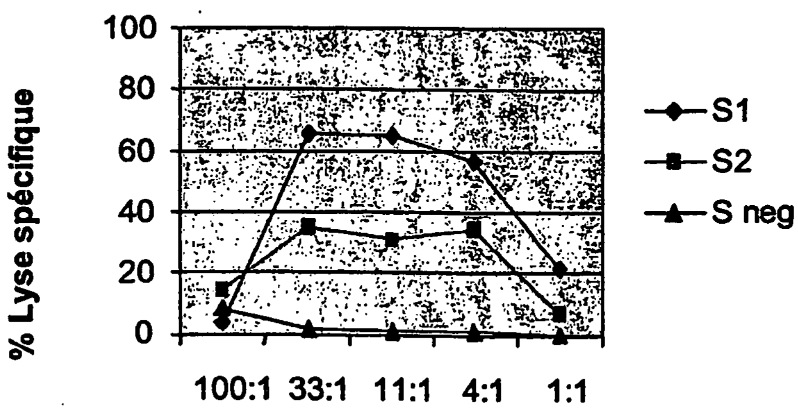

Figure 6

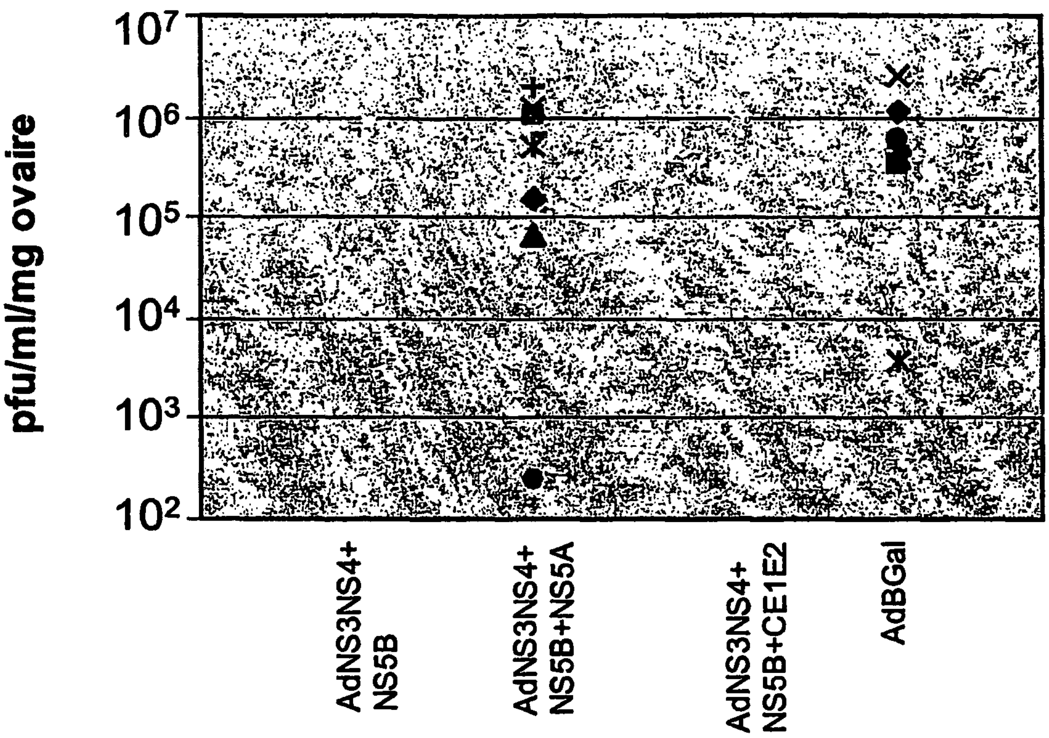


Figure 7

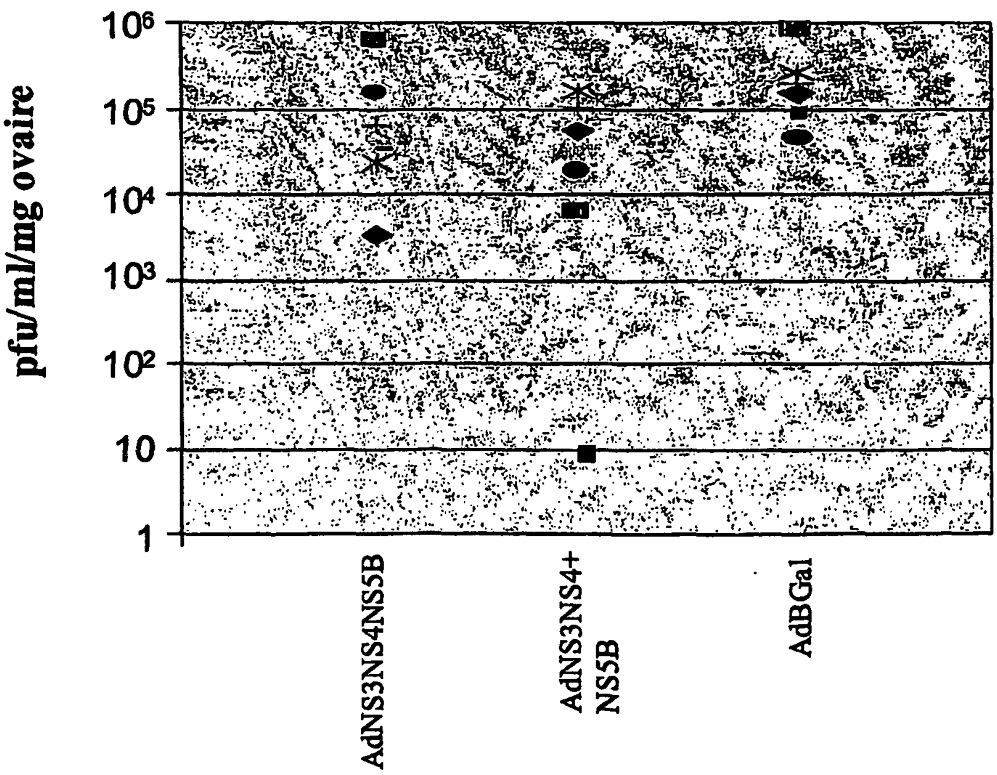

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9938880 A
- WO 0130812 A
- WO 9104282 A
- US 6099831 A
- US 6013638 A


### Littérature non-brevet citée dans la description


- **S. Deuffic et al.** *Hepatology,* 1999, vol. 29, 1596-1601
- **Major ME ; Feinstone SM.** *Hepatology,* Juin 1997, vol. 25 (6), 1527-1538
- **Manns MP et al.** *The Lancet,* 22 Septembre 2001, vol. 358, 958-965
- **LECHNER, F. et al.** *Eur. J. Immunol.,* 2000, vol. 30, 2479-2487
- **Thimme R et al.** *J. Exp. Med.,* 2001, vol. 194 (10), 1395-1406
- **Makimura et al.** *Vaccine,* 1996, vol. 14, 28-34
- **Fournillier A. et al.** *J. Virology,* 1999, vol. 73, 7497-7504
- **Brinster et al.** *Hepatology,* 2001, vol. 34, 1206-1217
- **Pancholi et al.** *J. Virology,* 2003, vol. 77, 382-390
- **Cho et al.** *Vaccine,* 05 Mars 1999, vol. 17 (9-10), 1136-44
- 2.1 plasmid construction. nucléotides. 1137
- **Choo et al.** *Proc. Natl. Acad. Sci.,* 1991, vol. 88, 2451-2455
- **ALIGN, Dayhoff, M.O.** Atlas of Protein Sequence and Structure. 1981, vol. 3, 482-489
- **Okamoto H. et al.** *Nucleic Acids Res.,* 1992, vol. 20, 6410-6410
- **Kato et al.** *Proc. Natl. Acda., Sci.,* 1990, vol. 87, 9524-9528
- **Takamizawa et al.** *J. Virol.,* 1991, vol. 65, 1105-1113
- **Choo et al.** *Brit. Med. Bull.,* 1990, vol. 46, 423-441
- **Inchauspé G. et al.** *Proc. Natl. Acad. Sci.,* 1991, vol. 88, 10292-10296
- **Okamoto H. et al.** *J. Gen. Virol.,* 1994, vol. 45, 629-635
- **Okamoto H. et al.** *J. Gen. Virol.,* 1991, vol. 72, 2697-2704
- **Okamoto H. et al.** *Virology,* 1992, vol. 188, 331-341
- **Nako H. et al.** *J. Gen. Virol.,* 1996, vol. 141, 701-704
- **Sakamoto M. et al.** *J. Gen. Virol.,* 1994, vol. 75, 1761-1768
- **Chayama K. et al.** *J. Gen. Virol.,* 1994, vol. 75, 3623-3628
- **Chamberlain R.W. et al.** *J. Gen. Virol.,* 1997, vol. 78, 1341-1347
- **Chamberlain RW. et al.** *Biochem. Biophys. Res. Commun.,* 1997, vol. 236, 44-49
- **Adams A. et al.** *Biochem. Biophys. Res. Commun.,* 1997, vol. 234, 393-396
- **Tokita H. et al.** *J. Gen. Virol.,* 1998, vol. 79, 1847
- **Tokita H. et al.** *J. Gen. Virol.,* 1996, vol. 77, 293-301
- Recombinant DNA Technology I. Annals of the New-York Academy of Sciences. 1991, vol. 646
- **Sambrook J. et al.** Molecular Cloning : A Laboratory Manual. 1989
- **Adra et al.** *Gene,* 1987, vol. 60, 65-74
- **Moessler et al.** *Development,* 1996, vol. 122, 2415-2425
- **Seshidhar Reddy et al.** *J. Virol.,* 1998, vol. 72, 1394-1402
- **Kelly ; Lewis.** *J. Virol.,* 1973, vol. 12, 643-652
- **Yei et al.** *Hum. Gene Ther.,* 1994, vol. 5, 731-744
- **Dai et al.** *Proc. Natl. Acad Sci. USA,* 1995, vol. 92, 1401-1405
- **Goebel et al.** *Virol.,* 1990, vol. 179, 247-266517-563
- **Antoine et al.** *Virol.,* 1998, vol. 244, 635-396
- **Mayr et al.** *Infection,* 1975, vol. 3, 6-16
- **Antoine et al.** *Virology,* 1998, vol. 244, 365-396
- **Ciernik I.F. et al.** *The Journal of Immunology,* 1999, vol. 162, 3915-3925
- **Gossen M. et al.** *Proc Natl Acad Sci USA,* 1992, vol. 89, 5547-5551
- **Brinster et al.** *Hepatology,* 2001
- **Murata et al.** *PNAS,* vol. 100, 6753-6758

SEQUENCE LISTING

<110> BIOMERIEUX
INSTITUT NATIONAL DE LA SANTE ET DE LA RECHERCHE MEDICALE

<120> Composition comprenant la polyprotéine NS3/NS4 et le polypeptide NS5b du VHC, vecteurs d'expression incluant les séquences nucléiques correspondantes et leur utilisation en thérapeutique

<130> ADENOVIR

<160> 27

<170> PatentIn version 3.1

<210> 1
<211> 2844
<212> DNA
<213> Artificial sequence

<220>
<223> séquence codant pour NS3NS4

<220>
<221> CDS
<222> (1)..(2844)
<223>

<400> 1

```
atg gcg cct atc acg gcc tat tcc caa caa acg cgg ggc ctg ctt ggc      48
Met Ala Pro Ile Thr Ala Tyr Ser Gln Gln Thr Arg Gly Leu Leu Gly
1               5                   10                  15

tgt atc atc act agc ctc aca ggt cgg gac aag aac cag gtc gat ggg      96
Cys Ile Ile Thr Ser Leu Thr Gly Arg Asp Lys Asn Gln Val Asp Gly
            20                  25                  30

gag gtt cag gtg ctc tcc acc gca acg caa tct ttc ctg gcg acc tgc     144
Glu Val Gln Val Leu Ser Thr Ala Thr Gln Ser Phe Leu Ala Thr Cys
        35                  40                  45

gtc aat ggc gtg tgt tgg acc gtc tac cat ggt gcc ggc tcg aag acc     192
Val Asn Gly Val Cys Trp Thr Val Tyr His Gly Ala Gly Ser Lys Thr
    50                  55                  60

ctg gcc ggc ccg aag ggt cca atc acc caa atg tac acc aat gta gac     240
Leu Ala Gly Pro Lys Gly Pro Ile Thr Gln Met Tyr Thr Asn Val Asp
65                  70                  75                  80

cag gac ctc gtc ggc tgg ccg gcg ccc ccc ggg gcg cgc tcc atg aca     288
Gln Asp Leu Val Gly Trp Pro Ala Pro Pro Gly Ala Arg Ser Met Thr
                85                  90                  95

ccg tgc acc tgc ggc agc tcg gac ctt tac ttg gtc acg agg cat gcc     336
Pro Cys Thr Cys Gly Ser Ser Asp Leu Tyr Leu Val Thr Arg His Ala
            100                 105                 110

gat gtc att ccg gtg cgc cgg cga ggc gac agc agg ggg agt cta ctc     384
Asp Val Ile Pro Val Arg Arg Arg Gly Asp Ser Arg Gly Ser Leu Leu
        115                 120                 125
```

```
tcc cct agg ccc gtc tcc tac ctg aag ggc tcc tcg ggt gga cca ctg      432
Ser Pro Arg Pro Val Ser Tyr Leu Lys Gly Ser Ser Gly Gly Pro Leu
    130                 135                 140

ctt tgc cct tcg ggg cac gtt gta ggc atc ttc cgg gct gct gtg tgc      480
Leu Cys Pro Ser Gly His Val Val Gly Ile Phe Arg Ala Ala Val Cys
145                 150                 155                 160

acc cgg ggg gtt gcg aag gcg gtg gac ttc ata ccc gtt gag tct atg      528
Thr Arg Gly Val Ala Lys Ala Val Asp Phe Ile Pro Val Glu Ser Met
                165                 170                 175

gaa act acc atg cgg tct ccg gtc ttc aca gac aac tca tcc cct ccg      576
Glu Thr Thr Met Arg Ser Pro Val Phe Thr Asp Asn Ser Ser Pro Pro
            180                 185                 190

gcc gta ccg caa aca ttc caa gtg gca cat tta cac gct ccc act ggc      624
Ala Val Pro Gln Thr Phe Gln Val Ala His Leu His Ala Pro Thr Gly
        195                 200                 205

agc ggc aag agc acc aaa gtg ccg gct gca tat gca gcc caa ggg tac      672
Ser Gly Lys Ser Thr Lys Val Pro Ala Ala Tyr Ala Ala Gln Gly Tyr
    210                 215                 220

aag gtg ctc gtc cta aac ccg tcc gtt gct gcc aca ttg ggc ttt gga      720
Lys Val Leu Val Leu Asn Pro Ser Val Ala Ala Thr Leu Gly Phe Gly
225                 230                 235                 240

gcg tat atg tcc aag gca cat ggc atc gag cct aac atc aga act ggg      768
Ala Tyr Met Ser Lys Ala His Gly Ile Glu Pro Asn Ile Arg Thr Gly
                245                 250                 255

gta agg acc atc acc acg ggc ggc ccc atc acg tac tcc acc tat ggc      816
Val Arg Thr Ile Thr Thr Gly Gly Pro Ile Thr Tyr Ser Thr Tyr Gly
            260                 265                 270

aag ttc ctt gcc gac ggt gga tgc tcc ggg ggc gcc tat gac atc ata      864
Lys Phe Leu Ala Asp Gly Gly Cys Ser Gly Gly Ala Tyr Asp Ile Ile
        275                 280                 285

ata tgt gac gaa tgc cac tca act gac tgg aca acc atc ttg ggc atc      912
Ile Cys Asp Glu Cys His Ser Thr Asp Trp Thr Thr Ile Leu Gly Ile
    290                 295                 300

ggc aca gtc ctg gat cag gca gag acg gct gga gcg cgg ctc gtc gtg      960
Gly Thr Val Leu Asp Gln Ala Glu Thr Ala Gly Ala Arg Leu Val Val
305                 310                 315                 320

ctc gcc acc gcc acg cct ccg gga tcg atc acc gtg cca cac ccc aac     1008
Leu Ala Thr Ala Thr Pro Pro Gly Ser Ile Thr Val Pro His Pro Asn
                325                 330                 335

atc gag gaa gtg gcc ctg tcc aac act ggg gag att ccc ttc tat ggc     1056
Ile Glu Glu Val Ala Leu Ser Asn Thr Gly Glu Ile Pro Phe Tyr Gly
            340                 345                 350

aaa gcc atc ccc att gag gcc atc aag ggg gga agg cat ctc atc ttc     1104
Lys Ala Ile Pro Ile Glu Ala Ile Lys Gly Gly Arg His Leu Ile Phe
        355                 360                 365
```

```
tgc cat tcc aag aag aag tgt gac gag ctc gcc gca aag ctg aca ggc      1152
Cys His Ser Lys Lys Lys Cys Asp Glu Leu Ala Ala Lys Leu Thr Gly
    370                 375                 380

ctc gga ctc aat gct gta gcg tat tac cgg ggt ctc gat gtg tcc gtc      1200
Leu Gly Leu Asn Ala Val Ala Tyr Tyr Arg Gly Leu Asp Val Ser Val
385                 390                 395                     400

ata ccg act agc gga gac gtc gtt gtc gtg gca aca gac gct cta atg      1248
Ile Pro Thr Ser Gly Asp Val Val Val Val Ala Thr Asp Ala Leu Met
                405                 410                 415

acg ggc ttt acc ggc gac ttt gac tca gtg atc gac tgc aac aca tgt      1296
Thr Gly Phe Thr Gly Asp Phe Asp Ser Val Ile Asp Cys Asn Thr Cys
            420                 425                 430

gtc acc cag aca gtc gat ttc agc ttg gat ccc acc ttc acc att gag      1344
Val Thr Gln Thr Val Asp Phe Ser Leu Asp Pro Thr Phe Thr Ile Glu
        435                 440                 445

acg aca acc gtg ccc caa gac gcg gtg tcg cgc tcg cag cgg cga ggt      1392
Thr Thr Thr Val Pro Gln Asp Ala Val Ser Arg Ser Gln Arg Arg Gly
    450                 455                 460

agg act ggc agg ggc agg agt ggc atc tac agg ttt gtg act cca gga      1440
Arg Thr Gly Arg Gly Arg Ser Gly Ile Tyr Arg Phe Val Thr Pro Gly
465                 470                 475                     480

gaa cgg ccc tca ggc atg ttc gac tcc tcg gtc ctg tgt gag tgc tat      1488
Glu Arg Pro Ser Gly Met Phe Asp Ser Ser Val Leu Cys Glu Cys Tyr
                485                 490                 495

gac gca ggc tgc gct tgg tat gag ctc acg ccc gct gag act aca gtc      1536
Asp Ala Gly Cys Ala Trp Tyr Glu Leu Thr Pro Ala Glu Thr Thr Val
            500                 505                 510

agg ttg cgg gct tac ctg aat aca cca ggg ttg ccc gtc tgc cag gac      1584
Arg Leu Arg Ala Tyr Leu Asn Thr Pro Gly Leu Pro Val Cys Gln Asp
        515                 520                 525

cat ctg gag ttc tgg gaa agc gtc ttc aca ggc ctc acc cac ata gat      1632
His Leu Glu Phe Trp Glu Ser Val Phe Thr Gly Leu Thr His Ile Asp
    530                 535                 540

gcc cac ttc ctg tcc caa acc aag cag gca gga gac aac ttc ccc tac      1680
Ala His Phe Leu Ser Gln Thr Lys Gln Ala Gly Asp Asn Phe Pro Tyr
545                 550                 555                     560

ctg gtg gca tac caa gcc acg gtg tgc gcc agg gct cag gct cca cct      1728
Leu Val Ala Tyr Gln Ala Thr Val Cys Ala Arg Ala Gln Ala Pro Pro
                565                 570                 575

cca tcg tgg gat caa atg tgg aag tgt ctc ata cgg ctt aaa cct acg      1776
Pro Ser Trp Asp Gln Met Trp Lys Cys Leu Ile Arg Leu Lys Pro Thr
            580                 585                 590

ctg cac ggg cca aca ccc ctg ctg tat agg cta gga gcc gtt caa aat      1824
Leu His Gly Pro Thr Pro Leu Leu Tyr Arg Leu Gly Ala Val Gln Asn
        595                 600                 605
```

```
gag atc acc ctc aca cat ccc ata acc aaa ttc gtc atg gca tgc atg      1872
Glu Ile Thr Leu Thr His Pro Ile Thr Lys Phe Val Met Ala Cys Met
    610                 615                 620

tcg gcc gac ctg gag gtc gtc act agc acc tgg gtg ctg gta ggc gga      1920
Ser Ala Asp Leu Glu Val Val Thr Ser Thr Trp Val Leu Val Gly Gly
625                 630                 635                 640

gtc ctt gca gct ctg gcc gca tat tgc ctg aca acc ggt agt gtg gtc      1968
Val Leu Ala Ala Leu Ala Ala Tyr Cys Leu Thr Thr Gly Ser Val Val
                645                 650                 655

att gtg ggt agg atc att ttg tcc ggg agg ccg gct gtt gtt ccc gac      2016
Ile Val Gly Arg Ile Ile Leu Ser Gly Arg Pro Ala Val Val Pro Asp
            660                 665                 670

agg gaa gtc ctc tac cgg gag ttc gat gaa atg gaa gag tgc gcc tca      2064
Arg Glu Val Leu Tyr Arg Glu Phe Asp Glu Met Glu Glu Cys Ala Ser
        675                 680                 685

cac ctc cct tac atc gag caa gga atg cag ctc gcc gag cag ttc aag      2112
His Leu Pro Tyr Ile Glu Gln Gly Met Gln Leu Ala Glu Gln Phe Lys
    690                 695                 700

cag cag gca ctc ggg ttg ctg caa aca gcc acc aag caa gcg gag gcc      2160
Gln Gln Ala Leu Gly Leu Leu Gln Thr Ala Thr Lys Gln Ala Glu Ala
705                 710                 715                 720

gct gct ccc gtg gtg gag tcc agg tgg cgg gcc ctt gag gcc ttc tgg      2208
Ala Ala Pro Val Val Glu Ser Arg Trp Arg Ala Leu Glu Ala Phe Trp
                725                 730                 735

gca aag cac atg tgg aac ttc atc agc ggg ata cag tac tta gca ggc      2256
Ala Lys His Met Trp Asn Phe Ile Ser Gly Ile Gln Tyr Leu Ala Gly
            740                 745                 750

tta tcc act ctg cct ggg aac ccc gcg ata gca tca ctg atg gca ttc      2304
Leu Ser Thr Leu Pro Gly Asn Pro Ala Ile Ala Ser Leu Met Ala Phe
        755                 760                 765

aca gcc tct atc acc agt ccg ctc acc acc cag aat acc ctc cta ttc      2352
Thr Ala Ser Ile Thr Ser Pro Leu Thr Thr Gln Asn Thr Leu Leu Phe
    770                 775                 780

aac atc tta ggg gga tgg gtg gct gct caa ctc gct cct ccc agt gct      2400
Asn Ile Leu Gly Gly Trp Val Ala Ala Gln Leu Ala Pro Pro Ser Ala
785                 790                 795                 800

gct tcg gcc ttc gtg ggt gcc ggc att gcc ggt gcg gcc att ggc agc      2448
Ala Ser Ala Phe Val Gly Ala Gly Ile Ala Gly Ala Ala Ile Gly Ser
                805                 810                 815

ata ggc ctt ggg aag gtg ctt gtg gac att ctg gcg ggc tat gga gcg      2496
Ile Gly Leu Gly Lys Val Leu Val Asp Ile Leu Ala Gly Tyr Gly Ala
            820                 825                 830

ggg gtg gcc ggt gca ctc gtg gct ttt aag gtc atg agc ggc gag gcg      2544
Gly Val Ala Gly Ala Leu Val Ala Phe Lys Val Met Ser Gly Glu Ala
        835                 840                 845
```

```
ccc tcc gcc gag gac ctg gtt aac ttg ctc cct gcc atc ctc tcc ccc      2592
Pro Ser Ala Glu Asp Leu Val Asn Leu Leu Pro Ala Ile Leu Ser Pro
    850                 855                 860

ggc gcc ttg gtc gtc ggg atc gtg tgt gca gca atc ctg cgt cgg cac      2640
Gly Ala Leu Val Val Gly Ile Val Cys Ala Ala Ile Leu Arg Arg His
865                 870                 875                 880

gtg ggc ccg gga gag ggg gct gtg cag tgg atg aac cgg ctg ata gcg      2688
Val Gly Pro Gly Glu Gly Ala Val Gln Trp Met Asn Arg Leu Ile Ala
                885                 890                 895

ttc gct tcg cgg ggt aac cac gtt tcc ccc acg cac tac gtg cct gag      2736
Phe Ala Ser Arg Gly Asn His Val Ser Pro Thr His Tyr Val Pro Glu
            900                 905                 910

agc gac gcc gca gca cgt gta act cag atc ctc tcc agc ctc acc atc      2784
Ser Asp Ala Ala Ala Arg Val Thr Gln Ile Leu Ser Ser Leu Thr Ile
        915                 920                 925

act cag ctg ctg aag agg ctt cac cag tgg att aat gag gac tgc tcc      2832
Thr Gln Leu Leu Lys Arg Leu His Gln Trp Ile Asn Glu Asp Cys Ser
    930                 935                 940

acg cca tgc taa                                                      2844
Thr Pro Cys
945
```

<210> 2
<211> 947
<212> PRT
<213> Artificial sequence

<220>
<223> séquence codant pour NS3NS4

<400> 2

```
Met Ala Pro Ile Thr Ala Tyr Ser Gln Gln Thr Arg Gly Leu Leu Gly
1               5                   10                  15

Cys Ile Ile Thr Ser Leu Thr Gly Arg Asp Lys Asn Gln Val Asp Gly
            20                  25                  30

Glu Val Gln Val Leu Ser Thr Ala Thr Gln Ser Phe Leu Ala Thr Cys
        35                  40                  45

Val Asn Gly Val Cys Trp Thr Val Tyr His Gly Ala Gly Ser Lys Thr
    50                  55                  60

Leu Ala Gly Pro Lys Gly Pro Ile Thr Gln Met Tyr Thr Asn Val Asp
65                  70                  75                  80

Gln Asp Leu Val Gly Trp Pro Ala Pro Pro Gly Ala Arg Ser Met Thr
                85                  90                  95

Pro Cys Thr Cys Gly Ser Ser Asp Leu Tyr Leu Val Thr Arg His Ala
            100                 105                 110
```

```
Asp Val Ile Pro Val Arg Arg Arg Gly Asp Ser Arg Gly Ser Leu Leu
        115                 120                 125

Ser Pro Arg Pro Val Ser Tyr Leu Lys Gly Ser Ser Gly Gly Pro Leu
    130                 135                 140

Leu Cys Pro Ser Gly His Val Val Gly Ile Phe Arg Ala Ala Val Cys
145                 150                 155                 160

Thr Arg Gly Val Ala Lys Ala Val Asp Phe Ile Pro Val Glu Ser Met
                165                 170                 175

Glu Thr Thr Met Arg Ser Pro Val Phe Thr Asp Asn Ser Ser Pro Pro
            180                 185                 190

Ala Val Pro Gln Thr Phe Gln Val Ala His Leu His Ala Pro Thr Gly
        195                 200                 205

Ser Gly Lys Ser Thr Lys Val Pro Ala Ala Tyr Ala Ala Gln Gly Tyr
    210                 215                 220

Lys Val Leu Val Leu Asn Pro Ser Val Ala Ala Thr Leu Gly Phe Gly

225                 230                 235                 240

Ala Tyr Met Ser Lys Ala His Gly Ile Glu Pro Asn Ile Arg Thr Gly
                245                 250                 255

Val Arg Thr Ile Thr Thr Gly Gly Pro Ile Thr Tyr Ser Thr Tyr Gly
            260                 265                 270

Lys Phe Leu Ala Asp Gly Gly Cys Ser Gly Gly Ala Tyr Asp Ile Ile
        275                 280                 285

Ile Cys Asp Glu Cys His Ser Thr Asp Trp Thr Thr Ile Leu Gly Ile
    290                 295                 300

Gly Thr Val Leu Asp Gln Ala Glu Thr Ala Gly Ala Arg Leu Val Val
305                 310                 315                 320

Leu Ala Thr Ala Thr Pro Pro Gly Ser Ile Thr Val Pro His Pro Asn
                325                 330                 335

Ile Glu Glu Val Ala Leu Ser Asn Thr Gly Glu Ile Pro Phe Tyr Gly
            340                 345                 350

Lys Ala Ile Pro Ile Glu Ala Ile Lys Gly Gly Arg His Leu Ile Phe
        355                 360                 365

Cys His Ser Lys Lys Lys Cys Asp Glu Leu Ala Ala Lys Leu Thr Gly
    370                 375                 380

Leu Gly Leu Asn Ala Val Ala Tyr Tyr Arg Gly Leu Asp Val Ser Val
385                 390                 395                 400

Ile Pro Thr Ser Gly Asp Val Val Val Val Ala Thr Asp Ala Leu Met
                405                 410                 415

Thr Gly Phe Thr Gly Asp Phe Asp Ser Val Ile Asp Cys Asn Thr Cys
            420                 425                 430
```

```
Val Thr Gln Thr Val Asp Phe Ser Leu Asp Pro Thr Phe Thr Ile Glu
        435                 440                 445

Thr Thr Thr Val Pro Gln Asp Ala Val Ser Arg Ser Gln Arg Arg Gly
    450                 455                 460

Arg Thr Gly Arg Gly Arg Ser Gly Ile Tyr Arg Phe Val Thr Pro Gly
465                 470                 475                 480

Glu Arg Pro Ser Gly Met Phe Asp Ser Ser Val Leu Cys Glu Cys Tyr
                485                 490                 495

Asp Ala Gly Cys Ala Trp Tyr Glu Leu Thr Pro Ala Glu Thr Thr Val
            500                 505                 510

Arg Leu Arg Ala Tyr Leu Asn Thr Pro Gly Leu Pro Val Cys Gln Asp
        515                 520                 525

His Leu Glu Phe Trp Glu Ser Val Phe Thr Gly Leu Thr His Ile Asp
    530                 535                 540

Ala His Phe Leu Ser Gln Thr Lys Gln Ala Gly Asp Asn Phe Pro Tyr
545                 550                 555                 560

Leu Val Ala Tyr Gln Ala Thr Val Cys Ala Arg Ala Gln Ala Pro Pro
                565                 570                 575

Pro Ser Trp Asp Gln Met Trp Lys Cys Leu Ile Arg Leu Lys Pro Thr
            580                 585                 590

Leu His Gly Pro Thr Pro Leu Leu Tyr Arg Leu Gly Ala Val Gln Asn
        595                 600                 605

Glu Ile Thr Leu Thr His Pro Ile Thr Lys Phe Val Met Ala Cys Met
    610                 615                 620

Ser Ala Asp Leu Glu Val Val Thr Ser Thr Trp Val Leu Val Gly Gly
625                 630                 635                 640

Val Leu Ala Ala Leu Ala Ala Tyr Cys Leu Thr Thr Gly Ser Val Val
                645                 650                 655

Ile Val Gly Arg Ile Ile Leu Ser Gly Arg Pro Ala Val Val Pro Asp
            660                 665                 670

Arg Glu Val Leu Tyr Arg Glu Phe Asp Glu Met Glu Glu Cys Ala Ser
        675                 680                 685

His Leu Pro Tyr Ile Glu Gln Gly Met Gln Leu Ala Glu Gln Phe Lys
    690                 695                 700

Gln Gln Ala Leu Gly Leu Leu Gln Thr Ala Thr Lys Gln Ala Glu Ala
705                 710                 715                 720

Ala Ala Pro Val Val Glu Ser Arg Trp Arg Ala Leu Glu Ala Phe Trp
                725                 730                 735

Ala Lys His Met Trp Asn Phe Ile Ser Gly Ile Gln Tyr Leu Ala Gly
            740                 745                 750
```

```
Leu Ser Thr Leu Pro Gly Asn Pro Ala Ile Ala Ser Leu Met Ala Phe
        755                 760                 765

Thr Ala Ser Ile Thr Ser Pro Leu Thr Thr Gln Asn Thr Leu Leu Phe
    770                 775                 780

Asn Ile Leu Gly Gly Trp Val Ala Ala Gln Leu Ala Pro Pro Ser Ala
785                 790                 795                 800

Ala Ser Ala Phe Val Gly Ala Gly Ile Ala Gly Ala Ala Ile Gly Ser
                805                 810                 815

Ile Gly Leu Gly Lys Val Leu Val Asp Ile Leu Ala Gly Tyr Gly Ala
            820                 825                 830

Gly Val Ala Gly Ala Leu Val Ala Phe Lys Val Met Ser Gly Glu Ala
        835                 840                 845

Pro Ser Ala Glu Asp Leu Val Asn Leu Leu Pro Ala Ile Leu Ser Pro
    850                 855                 860

Gly Ala Leu Val Val Gly Ile Val Cys Ala Ala Ile Leu Arg Arg His
865                 870                 875                 880

Val Gly Pro Gly Glu Gly Ala Val Gln Trp Met Asn Arg Leu Ile Ala
                885                 890                 895

Phe Ala Ser Arg Gly Asn His Val Ser Pro Thr His Tyr Val Pro Glu
            900                 905                 910

Ser Asp Ala Ala Ala Arg Val Thr Gln Ile Leu Ser Ser Leu Thr Ile
        915                 920                 925

Thr Gln Leu Leu Lys Arg Leu His Gln Trp Ile Asn Glu Asp Cys Ser
    930                 935                 940

Thr Pro Cys
945
```

<210> 3
<211> 1779
<212> DNA
<213> Artificial sequence

<220>
<223> séquence codant pour NS5b

<220>
<221> CDS
<222> (1)..(1779)
<223>

<400> 3

```
atg tca atg tcc tac aca tgg aca ggt gcc ttg atc acg cca tgc gct      48
Met Ser Met Ser Tyr Thr Trp Thr Gly Ala Leu Ile Thr Pro Cys Ala
1               5                   10                  15

gcg gag gag agc aag ttg ccc atc aat ccg ttg agc aac tct ttg ctg      96
Ala Glu Glu Ser Lys Leu Pro Ile Asn Pro Leu Ser Asn Ser Leu Leu
            20                  25                  30
```

```
cgt cac cac agt atg gtc tac tcc aca aca tct cgc agc gca agt ctg      144
Arg His His Ser Met Val Tyr Ser Thr Thr Ser Arg Ser Ala Ser Leu
        35                  40                  45

cgg cag aag aag gtc acc ttt gac aga ctg caa gtc ctg gac gac cac      192
Arg Gln Lys Lys Val Thr Phe Asp Arg Leu Gln Val Leu Asp Asp His
    50                  55                  60

tac cgg gac gtg ctc aag gag atg aag gcg aag gcg tcc aca gtt aag      240
Tyr Arg Asp Val Leu Lys Glu Met Lys Ala Lys Ala Ser Thr Val Lys
65                  70                  75                  80

gct agg ctt cta tct ata gag gag gcc tgc aaa ctg acg ccc cca cat      288
Ala Arg Leu Leu Ser Ile Glu Glu Ala Cys Lys Leu Thr Pro Pro His
                85                  90                  95

tcg gcc aaa tcc aaa ttt ggc tac ggg gcg aag gac gtc cgg agc cta      336
Ser Ala Lys Ser Lys Phe Gly Tyr Gly Ala Lys Asp Val Arg Ser Leu
            100                 105                 110

tcc agc agg gcc gtc aac cac atc cgc tcc gtg tgg gag gac ttg ctg      384
Ser Ser Arg Ala Val Asn His Ile Arg Ser Val Trp Glu Asp Leu Leu
        115                 120                 125

gaa gac act gaa aca cca att gat acc acc atc atg gca aaa aat gag      432
Glu Asp Thr Glu Thr Pro Ile Asp Thr Thr Ile Met Ala Lys Asn Glu
    130                 135                 140

gtt ttc tgc gtc caa cca gag aaa gga ggc cgc aag cca gct cgc ctt      480
Val Phe Cys Val Gln Pro Glu Lys Gly Gly Arg Lys Pro Ala Arg Leu
145                 150                 155                 160

atc gta ttc cca gac ctg ggg gta cgt gta tgc gag aag atg gcc ctt      528
Ile Val Phe Pro Asp Leu Gly Val Arg Val Cys Glu Lys Met Ala Leu
                165                 170                 175

tac gac gtg gtc tcc acc ctt cct cag gcc gtg atg ggc ccc tca tac      576
Tyr Asp Val Val Ser Thr Leu Pro Gln Ala Val Met Gly Pro Ser Tyr
            180                 185                 190

gga ttc cag tac tct cct ggg cag cgg gtc gag ttc ctg gtg aat acc      624
Gly Phe Gln Tyr Ser Pro Gly Gln Arg Val Glu Phe Leu Val Asn Thr
        195                 200                 205

tgg aaa tca aag aaa tgc cct atg ggc ttc tca tat gac acc cgc tgc      672
Trp Lys Ser Lys Lys Cys Pro Met Gly Phe Ser Tyr Asp Thr Arg Cys
    210                 215                 220

ttt gac tca acg gtc act gag aat gac atc cgt act gag gag tca atc      720
Phe Asp Ser Thr Val Thr Glu Asn Asp Ile Arg Thr Glu Glu Ser Ile
225                 230                 235                 240

tac caa tgt tgt gac ttg gcc ccc gaa gcc aga cag gcc ata aag tcg      768
Tyr Gln Cys Cys Asp Leu Ala Pro Glu Ala Arg Gln Ala Ile Lys Ser
                245                 250                 255

ctc aca gag cgg ctc tac atc ggg ggt ccc ctg act aat tca aaa ggg      816
Leu Thr Glu Arg Leu Tyr Ile Gly Gly Pro Leu Thr Asn Ser Lys Gly
            260                 265                 270
```

```
cag aac tgc ggt tat cgc cgg tgc cgc gcg agc ggc gtg ctg acg act       864
Gln Asn Cys Gly Tyr Arg Arg Cys Arg Ala Ser Gly Val Leu Thr Thr
        275                 280                 285

agc tgc ggc aat acc ctc aca tgc tac ttg aaa gcc act gcg gcc tgt       912
Ser Cys Gly Asn Thr Leu Thr Cys Tyr Leu Lys Ala Thr Ala Ala Cys
    290                 295                 300

cga gct gca aag ctc cag gac tgc acg atg ctc gtg aac gga gac gac       960
Arg Ala Ala Lys Leu Gln Asp Cys Thr Met Leu Val Asn Gly Asp Asp
305                 310                 315                 320

ctt gtc gtt atc tgc gaa agc gcg gga acc cag gag gat gcg gcg agc      1008
Leu Val Val Ile Cys Glu Ser Ala Gly Thr Gln Glu Asp Ala Ala Ser
                325                 330                 335

cta cga gtc ttc acg gag gct atg act agg tac tct gcc ccc ccc ggg      1056
Leu Arg Val Phe Thr Glu Ala Met Thr Arg Tyr Ser Ala Pro Pro Gly
            340                 345                 350

gac ccg ccc caa cca gaa tac gac ttg gag ctg ata acg tca tgc tcc      1104
Asp Pro Pro Gln Pro Glu Tyr Asp Leu Glu Leu Ile Thr Ser Cys Ser
        355                 360                 365

tcc aat gtg tcg gtc gcg cac gat gca tcc ggc aaa agg gtg tac tac      1152
Ser Asn Val Ser Val Ala His Asp Ala Ser Gly Lys Arg Val Tyr Tyr
    370                 375                 380

ctc acc cgt gac ccc acc acc ccc ctc gca cgg gct gcg tgg gag aca      1200
Leu Thr Arg Asp Pro Thr Thr Pro Leu Ala Arg Ala Ala Trp Glu Thr
385                 390                 395                 400

gtt aga cac act cca gtc aac tcc tgg cta ggc aat atc atc atg tat      1248
Val Arg His Thr Pro Val Asn Ser Trp Leu Gly Asn Ile Ile Met Tyr
                405                 410                 415

gcg ccc acc cta tgg gcg agg atg att ctg atg act cat ttc ttc tct      1296
Ala Pro Thr Leu Trp Ala Arg Met Ile Leu Met Thr His Phe Phe Ser
            420                 425                 430

atc ctt cta gct cag gag caa ctt gaa aaa gcc ctg gat tgt cag atc      1344
Ile Leu Leu Ala Gln Glu Gln Leu Glu Lys Ala Leu Asp Cys Gln Ile
        435                 440                 445

tac ggg gcc tgc tac tcc att gag cca ctt gac cta cct cag atc atc      1392
Tyr Gly Ala Cys Tyr Ser Ile Glu Pro Leu Asp Leu Pro Gln Ile Ile
    450                 455                 460

gaa cga ctc cat ggt ctt agc gca ttt tca ctc cat agt tac tct cca      1440
Glu Arg Leu His Gly Leu Ser Ala Phe Ser Leu His Ser Tyr Ser Pro
465                 470                 475                 480

ggt gag atc aat agg gtg gct tca tgc ctc agg aaa ctt ggg gta cca      1488
Gly Glu Ile Asn Arg Val Ala Ser Cys Leu Arg Lys Leu Gly Val Pro
                485                 490                 495

ccc ttg cga gtc tgg aga cat cgg gcc aga agt gtc cgc gct aag ttg      1536
Pro Leu Arg Val Trp Arg His Arg Ala Arg Ser Val Arg Ala Lys Leu
            500                 505                 510
```

```
ctg tcc cag ggg ggg agg gcc gcc act tgc ggc aaa tac ctc ttc aac      1584
Leu Ser Gln Gly Gly Arg Ala Ala Thr Cys Gly Lys Tyr Leu Phe Asn
        515                 520                 525

tgg gca gta agg acc aag ctt aaa ctc act cca atc ccg gct gcg tcc      1632
Trp Ala Val Arg Thr Lys Leu Lys Leu Thr Pro Ile Pro Ala Ala Ser
    530                 535                 540

cag cta gac ttg tcc ggc tgg ttc gtt gct ggt tac aac ggg gga gac      1680
Gln Leu Asp Leu Ser Gly Trp Phe Val Ala Gly Tyr Asn Gly Gly Asp
545                 550                 555                 560

ata tat cac agc ctg tct cgt gcc cga ccc cgt tgg ttc atg ttg tgc      1728
Ile Tyr His Ser Leu Ser Arg Ala Arg Pro Arg Trp Phe Met Leu Cys
                565                 570                 575

cta ctc cta ctt tct gta ggg gta ggc atc tac ctg ctc ccc aac cgg      1776
Leu Leu Leu Leu Ser Val Gly Val Gly Ile Tyr Leu Leu Pro Asn Arg
            580                 585                 590

taa                                                                  1779
```

<210> 4
<211> 592
<212> PRT
<213> Artificial sequence

<220>
<223> séquence codant pour NS5b

<400> 4

```
Met Ser Met Ser Tyr Thr Trp Thr Gly Ala Leu Ile Thr Pro Cys Ala
1               5                   10                  15

Ala Glu Glu Ser Lys Leu Pro Ile Asn Pro Leu Ser Asn Ser Leu Leu
            20                  25                  30

Arg His His Ser Met Val Tyr Ser Thr Thr Ser Arg Ser Ala Ser Leu
        35                  40                  45

Arg Gln Lys Lys Val Thr Phe Asp Arg Leu Gln Val Leu Asp Asp His
    50                  55                  60

Tyr Arg Asp Val Leu Lys Glu Met Lys Ala Lys Ala Ser Thr Val Lys
65                  70                  75                  80

Ala Arg Leu Leu Ser Ile Glu Glu Ala Cys Lys Leu Thr Pro Pro His
                85                  90                  95

Ser Ala Lys Ser Lys Phe Gly Tyr Gly Ala Lys Asp Val Arg Ser Leu
            100                 105                 110

Ser Ser Arg Ala Val Asn His Ile Arg Ser Val Trp Glu Asp Leu Leu
        115                 120                 125

Glu Asp Thr Glu Thr Pro Ile Asp Thr Thr Ile Met Ala Lys Asn Glu
    130                 135                 140
```

```
Val Phe Cys Val Gln Pro Glu Lys Gly Gly Arg Lys Pro Ala Arg Leu
145                 150                 155                 160

Ile Val Phe Pro Asp Leu Gly Val Arg Val Cys Glu Lys Met Ala Leu
                165                 170                 175

Tyr Asp Val Val Ser Thr Leu Pro Gln Ala Val Met Gly Pro Ser Tyr
            180                 185                 190

Gly Phe Gln Tyr Ser Pro Gly Gln Arg Val Glu Phe Leu Val Asn Thr
        195                 200                 205

Trp Lys Ser Lys Lys Cys Pro Met Gly Phe Ser Tyr Asp Thr Arg Cys
    210                 215                 220

Phe Asp Ser Thr Val Thr Glu Asn Asp Ile Arg Thr Glu Glu Ser Ile
225                 230                 235                 240

Tyr Gln Cys Cys Asp Leu Ala Pro Glu Ala Arg Gln Ala Ile Lys Ser
                245                 250                 255

Leu Thr Glu Arg Leu Tyr Ile Gly Gly Pro Leu Thr Asn Ser Lys Gly
            260                 265                 270

Gln Asn Cys Gly Tyr Arg Arg Cys Arg Ala Ser Gly Val Leu Thr Thr
        275                 280                 285

Ser Cys Gly Asn Thr Leu Thr Cys Tyr Leu Lys Ala Thr Ala Ala Cys
    290                 295                 300

Arg Ala Ala Lys Leu Gln Asp Cys Thr Met Leu Val Asn Gly Asp Asp
305                 310                 315                 320

Leu Val Val Ile Cys Glu Ser Ala Gly Thr Gln Glu Asp Ala Ala Ser
                325                 330                 335

Leu Arg Val Phe Thr Glu Ala Met Thr Arg Tyr Ser Ala Pro Pro Gly
            340                 345                 350

Asp Pro Pro Gln Pro Glu Tyr Asp Leu Glu Leu Ile Thr Ser Cys Ser
        355                 360                 365

Ser Asn Val Ser Val Ala His Asp Ala Ser Gly Lys Arg Val Tyr Tyr
    370                 375                 380

Leu Thr Arg Asp Pro Thr Thr Pro Leu Ala Arg Ala Ala Trp Glu Thr
385                 390                 395                 400

Val Arg His Thr Pro Val Asn Ser Trp Leu Gly Asn Ile Ile Met Tyr
                405                 410                 415

Ala Pro Thr Leu Trp Ala Arg Met Ile Leu Met Thr His Phe Phe Ser
            420                 425                 430

Ile Leu Leu Ala Gln Glu Gln Leu Glu Lys Ala Leu Asp Cys Gln Ile
        435                 440                 445

Tyr Gly Ala Cys Tyr Ser Ile Glu Pro Leu Asp Leu Pro Gln Ile Ile
    450                 455                 460
```

Glu Arg Leu His Gly Leu Ser Ala Phe Ser Leu His Ser Tyr Ser Pro
465 470 475 480

Gly Glu Ile Asn Arg Val Ala Ser Cys Leu Arg Lys Leu Gly Val Pro
485 490 495

Pro Leu Arg Val Trp Arg His Arg Ala Arg Ser Val Arg Ala Lys Leu
500 505 510

Leu Ser Gln Gly Gly Arg Ala Ala Thr Cys Gly Lys Tyr Leu Phe Asn
515 520 525

Trp Ala Val Arg Thr Lys Leu Lys Leu Thr Pro Ile Pro Ala Ala Ser
530 535 540

Gln Leu Asp Leu Ser Gly Trp Phe Val Ala Gly Tyr Asn Gly Gly Asp
545 550 555 560

Ile Tyr His Ser Leu Ser Arg Ala Arg Pro Arg Trp Phe Met Leu Cys
565 570 575

Leu Leu Leu Leu Ser Val Gly Val Gly Ile Tyr Leu Leu Pro Asn Arg
580 585 590

<210> 5
<211> 1344
<212> DNA
<213> Artificial sequence

<220>
<223> séquence codant pour NS5a

<220>
<221> CDS
<222> (1)..(1344)
<223>

<400> 5

```
atg tcc ggc tcg tgg cta agg gat gtt tgg gac tgg ata tgc acg gtg      48
Met Ser Gly Ser Trp Leu Arg Asp Val Trp Asp Trp Ile Cys Thr Val
1               5                   10                  15

ttg act gac ttc aag acc tgg ctc cag tcc aag ctc ctg ccg aaa ttg      96
Leu Thr Asp Phe Lys Thr Trp Leu Gln Ser Lys Leu Leu Pro Lys Leu
            20                  25                  30

ccg gga gtc cct ttc ttc tca tgc caa cgc ggg tac aag gga gtc tgg     144
Pro Gly Val Pro Phe Phe Ser Cys Gln Arg Gly Tyr Lys Gly Val Trp
        35                  40                  45

cgg ggg gac ggc atc atg caa acc acc tgc cca tgt gga gca caa att     192
Arg Gly Asp Gly Ile Met Gln Thr Thr Cys Pro Cys Gly Ala Gln Ile
    50                  55                  60

acc gga cat gtc aaa aac ggt tcc atg agg atc gtt ggg cct aaa acc     240
Thr Gly His Val Lys Asn Gly Ser Met Arg Ile Val Gly Pro Lys Thr
65                  70                  75                  80
```

```
tgc agc aac acg tgg cac gga acg ttc ccc atc aac gcg tac acc aca       288
Cys Ser Asn Thr Trp His Gly Thr Phe Pro Ile Asn Ala Tyr Thr Thr
                85                  90                  95

ggc ccc tgc aca ccc tcc ccg gcg ccg aac tat tcc agg gcg ctg tgg       336
Gly Pro Cys Thr Pro Ser Pro Ala Pro Asn Tyr Ser Arg Ala Leu Trp
            100                 105                 110

cgg gtg gct gct gaa gag tac gtg gag att acg cgg gtg ggg gac ttc       384
Arg Val Ala Ala Glu Glu Tyr Val Glu Ile Thr Arg Val Gly Asp Phe
        115                 120                 125

cac tac gtg acg ggt atg acc acc gac aac gta aaa tgc ccg tgc cag       432
His Tyr Val Thr Gly Met Thr Thr Asp Asn Val Lys Cys Pro Cys Gln
    130                 135                 140

gtc ccg gcc ccc gaa ttc ttc act gaa ttg gac ggg gtg cgg ttg cac       480
Val Pro Ala Pro Glu Phe Phe Thr Glu Leu Asp Gly Val Arg Leu His
145                 150                 155                 160

agg tac gct ccg gcg tgc aga cct ctc cta cgg gtg gat gtc aca ttc       528
Arg Tyr Ala Pro Ala Cys Arg Pro Leu Leu Arg Val Asp Val Thr Phe
                165                 170                 175

cag gtc ggg ctc aac caa tac ctg gtt ggg tca cag ctc cca tgc gag       576
Gln Val Gly Leu Asn Gln Tyr Leu Val Gly Ser Gln Leu Pro Cys Glu
            180                 185                 190

cct gag ccg gat gtg gca gtg ctc act tcc atg ctc acc gac ccc tcc       624
Pro Glu Pro Asp Val Ala Val Leu Thr Ser Met Leu Thr Asp Pro Ser
        195                 200                 205

cac att aca gca gag acg gct aaa cgt agg ccg gcc agg ggg tct ccc       672
His Ile Thr Ala Glu Thr Ala Lys Arg Arg Pro Ala Arg Gly Ser Pro
    210                 215                 220

ccc tcc ttg gcc agc tct tca gct agc caa ttg tct gcg cct tcc ttg       720
Pro Ser Leu Ala Ser Ser Ser Ala Ser Gln Leu Ser Ala Pro Ser Leu
225                 230                 235                 240

aag gca aca tgc act acc cac cat gac tcc ccg gac gct gac ctc atc       768
Lys Ala Thr Cys Thr Thr His His Asp Ser Pro Asp Ala Asp Leu Ile
                245                 250                 255

gag gcc aac ctc ctg tgg cgg cag gag atg ggc gga aac atc acc cgt       816
Glu Ala Asn Leu Leu Trp Arg Gln Glu Met Gly Gly Asn Ile Thr Arg
            260                 265                 270

gtg gag tca gag aat aag gtg gta att ttg gac tct ttc gac ccg ctt       864
Val Glu Ser Glu Asn Lys Val Val Ile Leu Asp Ser Phe Asp Pro Leu
        275                 280                 285

cga gcg gaa gag gat gag agg gaa gta tcc gtt gca gca gag atc ctg       912
Arg Ala Glu Glu Asp Glu Arg Glu Val Ser Val Ala Ala Glu Ile Leu
    290                 295                 300

cga aaa tcc aag aag ttc ccc ccc gcg ttg ccc ata tgg gca cgc ccg       960
Arg Lys Ser Lys Lys Phe Pro Pro Ala Leu Pro Ile Trp Ala Arg Pro
305                 310                 315                 320
```

```
gat tac aac cct cca ctg tta gag tcc tgg aaa agt ccg gac tac gtc      1008
Asp Tyr Asn Pro Pro Leu Leu Glu Ser Trp Lys Ser Pro Asp Tyr Val
                325                 330                 335

cct ccg gcg gtg cat ggg tgc cca ttg ccg cct acc acg ggc cct cca      1056
Pro Pro Ala Val His Gly Cys Pro Leu Pro Pro Thr Thr Gly Pro Pro
            340                 345                 350

ata ccg cct cca cgg aaa aag agg acg gtt gtt ctg aca gag tcc acc      1104
Ile Pro Pro Pro Arg Lys Lys Arg Thr Val Val Leu Thr Glu Ser Thr
        355                 360                 365

gtg tct tct gcc ttg gcg gag ctg gct act aag act ttc ggc agc tcc      1152
Val Ser Ser Ala Leu Ala Glu Leu Ala Thr Lys Thr Phe Gly Ser Ser
    370                 375                 380

gga tcg tcg gcc gtt gac agc ggc acg gcg acc gcc cct ccc gat cag      1200
Gly Ser Ser Ala Val Asp Ser Gly Thr Ala Thr Ala Pro Pro Asp Gln
385                 390                 395                 400

acc tct gac gac ggt gac aaa gaa tct gac att gag tcg tac tcc tcc      1248
Thr Ser Asp Asp Gly Asp Lys Glu Ser Asp Ile Glu Ser Tyr Ser Ser
                405                 410                 415

atg ccc ccc ctt gag ggg gag ccg ggg gac cct gat ctc agc gac ggg      1296
Met Pro Pro Leu Glu Gly Glu Pro Gly Asp Pro Asp Leu Ser Asp Gly
            420                 425                 430

tct tgg tct acc gtg agc ggg gag gcc ggc gac gac atc gtc tgc tgc      1344
Ser Trp Ser Thr Val Ser Gly Glu Ala Gly Asp Asp Ile Val Cys Cys
        435                 440                 445
```

<210> 6
<211> 448
<212> PRT
<213> Artificial sequence

<220>
<223> séquence codant pour NS5a

<400> 6

```
Met Ser Gly Ser Trp Leu Arg Asp Val Trp Asp Trp Ile Cys Thr Val
1               5                   10                  15

Leu Thr Asp Phe Lys Thr Trp Leu Gln Ser Lys Leu Leu Pro Lys Leu
            20                  25                  30

Pro Gly Val Pro Phe Phe Ser Cys Gln Arg Gly Tyr Lys Gly Val Trp
        35                  40                  45

Arg Gly Asp Gly Ile Met Gln Thr Thr Cys Pro Cys Gly Ala Gln Ile
    50                  55                  60

Thr Gly His Val Lys Asn Gly Ser Met Arg Ile Val Gly Pro Lys Thr
65                  70                  75                      80

Cys Ser Asn Thr Trp His Gly Thr Phe Pro Ile Asn Ala Tyr Thr Thr
                85                  90                  95
```

Gly Pro Cys Thr Pro Ser Pro Ala Pro Asn Tyr Ser Arg Ala Leu Trp
100 105 110

Arg Val Ala Ala Glu Glu Tyr Val Glu Ile Thr Arg Val Gly Asp Phe
115 120 125

His Tyr Val Thr Gly Met Thr Thr Asp Asn Val Lys Cys Pro Cys Gln
130 135 140

Val Pro Ala Pro Glu Phe Phe Thr Glu Leu Asp Gly Val Arg Leu His
145 150 155 160

Arg Tyr Ala Pro Ala Cys Arg Pro Leu Leu Arg Val Asp Val Thr Phe
165 170 175

Gln Val Gly Leu Asn Gln Tyr Leu Val Gly Ser Gln Leu Pro Cys Glu
180 185 190

Pro Glu Pro Asp Val Ala Val Leu Thr Ser Met Leu Thr Asp Pro Ser
195 200 205

His Ile Thr Ala Glu Thr Ala Lys Arg Arg Pro Ala Arg Gly Ser Pro
210 215 220

Pro Ser Leu Ala Ser Ser Ser Ala Ser Gln Leu Ser Ala Pro Ser Leu
225 230 235 240

Lys Ala Thr Cys Thr Thr His His Asp Ser Pro Asp Ala Asp Leu Ile
245 250 255

Glu Ala Asn Leu Leu Trp Arg Gln Glu Met Gly Gly Asn Ile Thr Arg
260 265 270

Val Glu Ser Glu Asn Lys Val Val Ile Leu Asp Ser Phe Asp Pro Leu
275 280 285

Arg Ala Glu Glu Asp Glu Arg Glu Val Ser Val Ala Ala Glu Ile Leu
290 295 300

Arg Lys Ser Lys Lys Phe Pro Pro Ala Leu Pro Ile Trp Ala Arg Pro
305 310 315 320

Asp Tyr Asn Pro Pro Leu Leu Glu Ser Trp Lys Ser Pro Asp Tyr Val
325 330 335

Pro Pro Ala Val His Gly Cys Pro Leu Pro Pro Thr Thr Gly Pro Pro
340 345 350

Ile Pro Pro Pro Arg Lys Lys Arg Thr Val Val Leu Thr Glu Ser Thr
355 360 365

Val Ser Ser Ala Leu Ala Glu Leu Ala Thr Lys Thr Phe Gly Ser Ser
370 375 380

Gly Ser Ser Ala Val Asp Ser Gly Thr Ala Thr Ala Pro Pro Asp Gln
385 390 395 400

Thr Ser Asp Asp Gly Asp Lys Glu Ser Asp Ile Glu Ser Tyr Ser Ser
405 410 415

Met Pro Pro Leu Glu Gly Glu Pro Gly Asp Pro Asp Leu Ser Asp Gly
420 425 430

Ser Trp Ser Thr Val Ser Gly Glu Ala Gly Asp Asp Ile Val Cys Cys
435 440 445

<210> 7
<211> 2241
<212> DNA
<213> Artificial sequence

<220>
<223> séquence codant pour CE1E2

<220>
<221> CDS
<222> (1)..(2241)
<223>

<400> 7

```
atg agc aca aat cct aaa cct caa aga aaa acc aaa cgt aac acc aac      48
Met Ser Thr Asn Pro Lys Pro Gln Arg Lys Thr Lys Arg Asn Thr Asn
1               5                   10                  15

cgc cgc cca cag gac gtt aag ttc ccg ggc ggt ggt cag atc gtt ggt      96
Arg Arg Pro Gln Asp Val Lys Phe Pro Gly Gly Gly Gln Ile Val Gly
            20                  25                  30

gga gtt tac ctg ttg ccg cgc agg ggc ccc agg ttg ggt gtg cgc gcg     144
Gly Val Tyr Leu Leu Pro Arg Arg Gly Pro Arg Leu Gly Val Arg Ala
        35                  40                  45

act agg aag act tcc gag cgg tcg caa cct cgt gga agg cga caa cct     192
Thr Arg Lys Thr Ser Glu Arg Ser Gln Pro Arg Gly Arg Arg Gln Pro
    50                  55                  60

atc ccc aag gct cgc cgg ccc gag ggt agg acc tgg gct cag ccc ggg     240
Ile Pro Lys Ala Arg Arg Pro Glu Gly Arg Thr Trp Ala Gln Pro Gly
65                  70                  75                  80

tac cct tgg ccc ctc tat ggc aac gag ggt atg ggg tgg gca gga tgg     288
Tyr Pro Trp Pro Leu Tyr Gly Asn Glu Gly Met Gly Trp Ala Gly Trp
                85                  90                  95

ctc ctg tca ccc cgt ggc tct cgg cct agt tgg ggc ccc aca gac ccc     336
Leu Leu Ser Pro Arg Gly Ser Arg Pro Ser Trp Gly Pro Thr Asp Pro
            100                 105                 110

cgg cgt agg tcg cgt aat ttg ggt aag gtc atc gat acc ctt aca tgc     384
Arg Arg Arg Ser Arg Asn Leu Gly Lys Val Ile Asp Thr Leu Thr Cys
        115                 120                 125

ggc ttc gcc gac ctc atg ggg tac att ccg ctt gtc ggc gcc ccc cta     432
Gly Phe Ala Asp Leu Met Gly Tyr Ile Pro Leu Val Gly Ala Pro Leu
    130                 135                 140

gga ggc gct gcc agg gcc ctg gcg cat ggc gtc cgg gtt ctg gag gac     480
Gly Gly Ala Ala Arg Ala Leu Ala His Gly Val Arg Val Leu Glu Asp
145                 150                 155                 160
```

```
ggc gtg aac tat gca aca ggg aat ctg ccc ggt tgc tct ttc tct atc      528
Gly Val Asn Tyr Ala Thr Gly Asn Leu Pro Gly Cys Ser Phe Ser Ile
                165                 170                 175

ttc ctc tta gct ttg ctg tct tgt ttg acc atc cca gct tcc gct tac      576
Phe Leu Leu Ala Leu Leu Ser Cys Leu Thr Ile Pro Ala Ser Ala Tyr
            180                 185                 190

gag gtg cgc aac gtg tcc ggg ata tac cat gtc acg aac gac tgc tcc      624
Glu Val Arg Asn Val Ser Gly Ile Tyr His Val Thr Asn Asp Cys Ser
        195                 200                 205

aac tca agt att gtg tat gag gca gcg gac atg atc atg cac acc ccc      672
Asn Ser Ser Ile Val Tyr Glu Ala Ala Asp Met Ile Met His Thr Pro
    210                 215                 220

ggg tgc gtg ccc tgc gtc cgg gag agt aat ttc tcc cgt tgc tgg gta      720
Gly Cys Val Pro Cys Val Arg Glu Ser Asn Phe Ser Arg Cys Trp Val
225                 230                 235                 240

gcg ctc act ccc acg ctc gcg gcc agg aac agc agc atc ccc acc acg      768
Ala Leu Thr Pro Thr Leu Ala Ala Arg Asn Ser Ser Ile Pro Thr Thr
                245                 250                 255

aca ata cga cgc cac gtc gat ttg ctc gtt ggg gcg gct gct ctc tgt      816
Thr Ile Arg Arg His Val Asp Leu Leu Val Gly Ala Ala Ala Leu Cys
            260                 265                 270

tcc gct atg tac gtt ggg gat ctc tgc gga tcc gtt ttt ctc gtc tcc      864
Ser Ala Met Tyr Val Gly Asp Leu Cys Gly Ser Val Phe Leu Val Ser
        275                 280                 285

cag ctg ttc acc ttc tca cct cgc cgg tat gag acg gta caa gat tgc      912
Gln Leu Phe Thr Phe Ser Pro Arg Arg Tyr Glu Thr Val Gln Asp Cys
    290                 295                 300

aat tgc tca atc tat ccc ggc cac gta tca ggt cac cgc atg gct tgg      960
Asn Cys Ser Ile Tyr Pro Gly His Val Ser Gly His Arg Met Ala Trp
305                 310                 315                 320

gat atg atg atg aac tgg tca cct aca acg gcc cta gtg gta tcg cag     1008
Asp Met Met Met Asn Trp Ser Pro Thr Thr Ala Leu Val Val Ser Gln
                325                 330                 335

cta ctc cgg atc cca caa gcc gtc gtg gac atg gtg gcg ggg gcc cac     1056
Leu Leu Arg Ile Pro Gln Ala Val Val Asp Met Val Ala Gly Ala His
            340                 345                 350

tgg ggt gtc cta gcg ggc ctt gcc tac tat tcc atg gtg ggg aac tgg     1104
Trp Gly Val Leu Ala Gly Leu Ala Tyr Tyr Ser Met Val Gly Asn Trp
        355                 360                 365

gct aag gtc ttg att gtg atg cta ctc ttt gct ggc gtt gac ggg cac     1152
Ala Lys Val Leu Ile Val Met Leu Leu Phe Ala Gly Val Asp Gly His
    370                 375                 380

acc cac gtg aca ggg gga agg gta gcc tcc agc acc cag agc ctc gtg     1200
Thr His Val Thr Gly Gly Arg Val Ala Ser Ser Thr Gln Ser Leu Val
385                 390                 395                 400
```

```
tcc tgg ctc tca caa ggg cca tct cag aaa atc caa ctc gtg aac acc     1248
Ser Trp Leu Ser Gln Gly Pro Ser Gln Lys Ile Gln Leu Val Asn Thr
            405                 410                 415

aac ggc agc tgg cac atc aac agg acc gct ctg aat tgc aat gac tcc     1296
Asn Gly Ser Trp His Ile Asn Arg Thr Ala Leu Asn Cys Asn Asp Ser
            420                 425                 430

ctc caa act ggg ttc att gct gcg ctg ttc tac gca cac agg ttc aac     1344
Leu Gln Thr Gly Phe Ile Ala Ala Leu Phe Tyr Ala His Arg Phe Asn
        435                 440                 445

gcg tcc gga tgt cca gag cgc atg gcc agc tgc cgc ccc atc gac aag     1392
Ala Ser Gly Cys Pro Glu Arg Met Ala Ser Cys Arg Pro Ile Asp Lys
    450                 455                 460

ttc gct cag ggg tgg ggt ccc atc act cac gtt gtg cct aac atc tcg     1440
Phe Ala Gln Gly Trp Gly Pro Ile Thr His Val Val Pro Asn Ile Ser
465                 470                 475                 480

gac cag agg cct tat tgc tgg cac tat gca ccc caa ccg tgc ggt att     1488
Asp Gln Arg Pro Tyr Cys Trp His Tyr Ala Pro Gln Pro Cys Gly Ile
                485                 490                 495

gta ccc gcg tcg cag gtg tgt ggc cca gtg tat tgc ttc acc ccg agt     1536
Val Pro Ala Ser Gln Val Cys Gly Pro Val Tyr Cys Phe Thr Pro Ser
            500                 505                 510

cct gtt gtg gtg ggg acg acc gac cgt tcc gga gtc ccc acg tat agc     1584
Pro Val Val Val Gly Thr Thr Asp Arg Ser Gly Val Pro Thr Tyr Ser
        515                 520                 525

tgg ggg gag aat gag aca gac gtg ctg cta ctc aac aac acg cgg ccg     1632
Trp Gly Glu Asn Glu Thr Asp Val Leu Leu Leu Asn Asn Thr Arg Pro
    530                 535                 540

ccg caa ggc aac tgg ttc ggc tgt aca tgg atg aat agc acc ggg ttc     1680
Pro Gln Gly Asn Trp Phe Gly Cys Thr Trp Met Asn Ser Thr Gly Phe
545                 550                 555                 560

acc aag acg tgc ggg ggc ccc ccg tgt aac atc ggg ggg gtt ggc aac     1728
Thr Lys Thr Cys Gly Gly Pro Pro Cys Asn Ile Gly Gly Val Gly Asn
                565                 570                 575

aac acc ttg att tgc ccc acg gat tgc ttc cga aag cac ccc gag gcc     1776
Asn Thr Leu Ile Cys Pro Thr Asp Cys Phe Arg Lys His Pro Glu Ala
            580                 585                 590

act tac acc aaa tgc ggc tcg ggt cct tgg ttg aca cct agg tgt cta     1824
Thr Tyr Thr Lys Cys Gly Ser Gly Pro Trp Leu Thr Pro Arg Cys Leu
        595                 600                 605

gtt gac tac cca tac aga ctt tgg cac tac ccc tgc act atc aat ttt     1872
Val Asp Tyr Pro Tyr Arg Leu Trp His Tyr Pro Cys Thr Ile Asn Phe
    610                 615                 620

acc atc ttc aag gtc agg atg tac gtg ggg ggc gtg gag cac agg ctc     1920
Thr Ile Phe Lys Val Arg Met Tyr Val Gly Gly Val Glu His Arg Leu
625                 630                 635                 640
```

```
aac gcc gcg tgc aat tgg acc cga gga gag cgc tgt gac ctg gag gac      1968
Asn Ala Ala Cys Asn Trp Thr Arg Gly Glu Arg Cys Asp Leu Glu Asp
                645                 650                 655

agg gat aga tca gag ctt agc ccg ctg cta ttg tct aca acg gag tgg      2016
Arg Asp Arg Ser Glu Leu Ser Pro Leu Leu Leu Ser Thr Thr Glu Trp
            660                 665                 670

cag gta ctg ccc tgt tcc ttt acc acc cta ccg gct ctg tcc act gga      2064
Gln Val Leu Pro Cys Ser Phe Thr Thr Leu Pro Ala Leu Ser Thr Gly
        675                 680                 685

ttg atc cac ctc cat cag aat atc gtg gac gtg caa tac ctg tac ggt      2112
Leu Ile His Leu His Gln Asn Ile Val Asp Val Gln Tyr Leu Tyr Gly
    690                 695                 700

gta ggg tca gtg gtt gtc tcc gtc gta atc aaa tgg gag tat gtt ctg      2160
Val Gly Ser Val Val Val Ser Val Val Ile Lys Trp Glu Tyr Val Leu
705                 710                 715                 720

ctg ctc ttc ctt ctc ctg gcg gac gcg cgc gtc tgt gcc tgc ttg tgg      2208
Leu Leu Phe Leu Leu Leu Ala Asp Ala Arg Val Cys Ala Cys Leu Trp
                725                 730                 735

atg atg ctg ctg ata gcc cag gct gag gcc tga                          2241
Met Met Leu Leu Ile Ala Gln Ala Glu Ala
            740                 745
```

<210> 8
<211> 746
<212> PRT
<213> Artificial sequence

<220>
<223> séquence codant pour CE1E2

<400> 8

```
Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Ala Arg Arg Pro Glu Gly Arg Thr Trp Ala Gln Pro Gly
65                  70                  75                  80

Tyr Pro Trp Pro Leu Tyr Gly Asn Glu Gly Met Gly Trp Ala Gly Trp
                85                  90                  95

Leu Leu Ser Pro Arg Gly Ser Arg Pro Ser Trp Gly Pro Thr Asp Pro
            100                 105                 110
```

```
Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Ile Pro Ala Ser Ala Tyr
            180                 185                 190

Glu Val Arg Asn Val Ser Gly Ile Tyr His Val Thr Asn Asp Cys Ser
        195                 200                 205

Asn Ser Ser Ile Val Tyr Glu Ala Ala Asp Met Ile Met His Thr Pro
    210                 215                 220

Gly Cys Val Pro Cys Val Arg Glu Ser Asn Phe Ser Arg Cys Trp Val
225                 230                 235                 240

Ala Leu Thr Pro Thr Leu Ala Ala Arg Asn Ser Ser Ile Pro Thr Thr
                245                 250                 255

Thr Ile Arg Arg His Val Asp Leu Leu Val Gly Ala Ala Ala Leu Cys
            260                 265                 270

Ser Ala Met Tyr Val Gly Asp Leu Cys Gly Ser Val Phe Leu Val Ser
        275                 280                 285

Gln Leu Phe Thr Phe Ser Pro Arg Arg Tyr Glu Thr Val Gln Asp Cys
    290                 295                 300

Asn Cys Ser Ile Tyr Pro Gly His Val Ser Gly His Arg Met Ala Trp
305                 310                 315                 320

Asp Met Met Met Asn Trp Ser Pro Thr Thr Ala Leu Val Val Ser Gln
                325                 330                 335

Leu Leu Arg Ile Pro Gln Ala Val Val Asp Met Val Ala Gly Ala His
            340                 345                 350

Trp Gly Val Leu Ala Gly Leu Ala Tyr Tyr Ser Met Val Gly Asn Trp
        355                 360                 365

Ala Lys Val Leu Ile Val Met Leu Leu Phe Ala Gly Val Asp Gly His
    370                 375                 380

Thr His Val Thr Gly Gly Arg Val Ala Ser Ser Thr Gln Ser Leu Val
385                 390                 395                 400

Ser Trp Leu Ser Gln Gly Pro Ser Gln Lys Ile Gln Leu Val Asn Thr
                405                 410                 415

Asn Gly Ser Trp His Ile Asn Arg Thr Ala Leu Asn Cys Asn Asp Ser
            420                 425                 430
```

```
Leu Gln Thr Gly Phe Ile Ala Ala Leu Phe Tyr Ala His Arg Phe Asn
        435                 440                 445

Ala Ser Gly Cys Pro Glu Arg Met Ala Ser Cys Arg Pro Ile Asp Lys
    450                 455                 460

Phe Ala Gln Gly Trp Gly Pro Ile Thr His Val Val Pro Asn Ile Ser
465                 470                 475                 480

Asp Gln Arg Pro Tyr Cys Trp His Tyr Ala Pro Gln Pro Cys Gly Ile
                485                 490                 495

Val Pro Ala Ser Gln Val Cys Gly Pro Val Tyr Cys Phe Thr Pro Ser
            500                 505                 510

Pro Val Val Val Gly Thr Thr Asp Arg Ser Gly Val Pro Thr Tyr Ser
        515                 520                 525

Trp Gly Glu Asn Glu Thr Asp Val Leu Leu Leu Asn Asn Thr Arg Pro
    530                 535                 540

Pro Gln Gly Asn Trp Phe Gly Cys Thr Trp Met Asn Ser Thr Gly Phe
545                 550                 555                 560

Thr Lys Thr Cys Gly Gly Pro Pro Cys Asn Ile Gly Gly Val Gly Asn
                565                 570                 575

Asn Thr Leu Ile Cys Pro Thr Asp Cys Phe Arg Lys His Pro Glu Ala
            580                 585                 590

Thr Tyr Thr Lys Cys Gly Ser Gly Pro Trp Leu Thr Pro Arg Cys Leu
        595                 600                 605

Val Asp Tyr Pro Tyr Arg Leu Trp His Tyr Pro Cys Thr Ile Asn Phe
    610                 615                 620

Thr Ile Phe Lys Val Arg Met Tyr Val Gly Gly Val Glu His Arg Leu
625                 630                 635                 640

Asn Ala Ala Cys Asn Trp Thr Arg Gly Glu Arg Cys Asp Leu Glu Asp
                645                 650                 655

Arg Asp Arg Ser Glu Leu Ser Pro Leu Leu Leu Ser Thr Thr Glu Trp
            660                 665                 670

Gln Val Leu Pro Cys Ser Phe Thr Thr Leu Pro Ala Leu Ser Thr Gly
        675                 680                 685

Leu Ile His Leu His Gln Asn Ile Val Asp Val Gln Tyr Leu Tyr Gly
    690                 695                 700

Val Gly Ser Val Val Val Ser Val Val Ile Lys Trp Glu Tyr Val Leu
705                 710                 715                 720

Leu Leu Phe Leu Leu Leu Ala Asp Ala Arg Val Cys Ala Cys Leu Trp
                725                 730                 735

Met Met Leu Leu Ile Ala Gln Ala Glu Ala
            740                 745
```

<210> 9
<211> 29

<212> DNA
<213> Artificial sequence

<220>
<223> amorce oIV166

<400> 9
gggggggcta tggcgcctat cacggccta 29

<210> 10
<211> 32
<212> DNA
<213> Artificial sequence

<220>
<223> amorce oIV171

<400> 10
ggggggacgc gtttagcatg gcgtggagca gt 32

<210> 11
<211> 30
<212> DNA
<213> Artificial sequence

<220>
<223> amorce oIV232

<400> 11
ggggggagat ctccagcagg cagaagtatg 30

<210> 12
<211> 33
<212> DNA
<213> Artificial sequence

<220>
<223> amorceoIV233

<400> 12
gggggggtcg accgaaaatg gatatacaag ctc 33

<210> 13
<211> 35
<212> DNA
<213> Artificial sequence

<220>
<223> amorce oIV212

<400> 13
ggggggtcta gaatgtcaat gtcctacaca tggac 35

<210> 14
<211> 32
<212> DNA
<213> Artificial sequence

<220>
<223> amorce oIV218

<400> 14
ggggggtcta gattaccggt tggggagcag gt 32

<210> 15
<211> 32
<212> DNA
<213> Artificial sequence

<220>
<223> amorce oIV225

<400> 15
ggggggctgc agatggcgcc tatcacggcc ta 32

<210> 16
<211> 32
<212> DNA
<213> Artificial sequence

<220>
<223> amorce oIV226

<400> 16
ggggggtcta gattagcatg gcgtggagca gt 32

<210> 17
<211> 35
<212> DNA
<213> Artificial sequence

<220>
<223> amorce oIV227

<400> 17
gggggggtcg acatgtcaat gtcctacaca tggac 35

<210> 18
<211> 32
<212> DNA
<213> Artificial sequence

<220>
<223> amorce oIV228

<400> 18
gggggggcat gcttaccggt tggggagcag gt 32

<210> 19
<211> 33
<212> DNA
<213> Artificial sequence

<220>
<223> amorce oIV229

<400> 19
ggggggtcta gaccggtagt tcgcatatac ata 33

<210> 20
<211> 33
<212> DNA
<213> Artificial sequence

<220>
<223> amorce oIV172

<400> 20
ggggggggta ccatgtccgg ctcgtggcta agg 33

<210> 21
<211> 33
<212> DNA
<213> Artificial sequence

<220>
<223> amorce oIV173

<400> 21
ggggggtcta gattagcagc agacgatgtc gtc 33

<210> 22
<211> 33
<212> DNA
<213> Artificial sequence

<220>
<223> amorce oIV62

<400> 22
gggggggcta gcatgagcac aaatcctaaa cct 33

<210> 23
<211> 33
<212> DNA
<213> Artificial sequence

<220>
<223> amorce oIV68

<400> 23
ggggggtcta gatcaggcct cagcctgggc tat 33

<210> 24
<211> 10
<212> PRT
<213> Artificial sequence

<220>
<223> épitope GLL

<400> 24

Gly Leu Leu Gly Cys Ile Ile Thr Ser Leu
1 5 10

<210> 25
<211> 9
<212> PRT
<213> Artificial sequence

<220>
<223> épitope ALY

<400> 25

Ala Leu Tyr Asp Val Val Ser Thr Leu
1 5

<210> 26
<211> 9
<212> PRT
<213> Artificial sequence

<220>
<223> épitope KLQ

<400> 26

Lys Leu Gln Asp Cys Thr Met Leu Val
1 5

<210> 27
<211> 9
<212> PRT
<213> Artificial sequence

<220>
<223> épitope DLM

<400> 27

Asp Leu Met Gly Tyr Ile Pro Leu Val
1 5